# EUROPEAN PATENT APPLICATION

(11) **EP 1 449 834 A2**
(43) Date of publication of application: **25.08.2004**
(21) Application number: 03026051.7
(22) Date of filing: 22.12.1998
(51) Int. Cl.: C07D 213/40, C07D 213/70, C07D 213/36, C07D 277/68, C07D 213/68, C07D 333/20, C07C 275/36, C07C 275/40, C07C 275/28, C07C 275/30, C07C 275/42, A61K 31/17, A61K 31/38, A61K 31/44

(54) **Inhibition of raf kinase using symmetrical and unsymmetrical substituted diphenyl ureas**

(30) Priority: 22.12.1997 US 996344
(62) Divisional of application: 98963809.3
(71) Applicant: Bayer Corporation, Pittsburgh, PA 15205-9741 (US)
(72) Inventor: The designation of the inventor has not yet been filed
(74) Representative: Weiss, Wolfgang, Dipl.-Chem. Dr.

(57) **Abstract**

This invention relates to the use of a group of aryl ureas in treating raf mediated diseases, and pharmaceutical compositions for use in such therapy.

## Description

### Field of the Invention

This invention relates to the use of a group of aryl ureas in treating raf mediated diseases, and pharmaceutical compositions for use in such therapy.

### Background of the Invention

The p21^{ras} oncogene is a major contributor to the development and progression of human solid cancers and is mutated in 30% of all human cancers (Bolton et al. *Ann. Rep. Med. Chem.* **1994**, *29*, 165-74; Bos. *Cancer Res.* **1989**, *49*, 4682-9). In its normal, unmutated form, the ras protein is a key element of the signal transduction cascade directed by growth factor receptors in almost all tissues (Avruch et al. *Trends Biochem. Sci.* **1994**, *19*, 279-83). Biochemically, ras is a guanine nucleotide binding protein, and cycling between a GTP-bound activated and a GDP-bound resting form is strictly controlled by ras' endogenous GTPase activity and other regulatory proteins. In the ras mutants in cancer cells, the endogenous GTPase activity is alleviated and, therefore, the protein delivers constitutive growth signals to downstream effectors such as the enzyme raf kinase. This leads to the cancerous growth of the cells which carry these mutants (Magnuson et al. *Semin. Cancer Biol.* **1994**, *5*, 247-53). It has been shown that inhibiting the effect of active ras by inhibiting the raf kinase signaling pathway by administration of deactivating antibodies to raf kinase or by co-expression of dominant negative raf kinase or dominant negative MEK, the substrate of raf kinase, leads to the reversion of transformed cells to the normal growth phenotype (see: Daum et al. *Trends Biochem. Sci.* **1994**, *19*, 474-80; Fridman et al. *J. Biol. Chem.* **1994**, *269*, 30105-8. Kolch et al. (*Nature* **1991**, *349*, 426-28) have further indicated that inhibition of raf expression by antisense RNA blocks cell proliferation in membrane-associated oncogenes. Similarly, inhibition of raf kinase (by antisense oligodeoxynucleotides) has been correlated in vitro and in vivo with inhibition of the growth of a variety of human tumor types (Monia et al., *Nat. Med*. **1996**, *2*, 668-75).

### Summary of the Invention

The present invention provides compounds which are inhibitors of the enzyme raf kinase. Since the enzyme is a downstream effector of p21^{ras}, the instant inhibitors are useful in pharmaceutical compositions for human or veterinary use where inhibition of the raf kinase pathway is indicated, e.g., in the treatment of tumors and/or cancerous cell growth mediated by raf kinase. In particular, the compounds are useful in the treatment of human or animal cancers, e.g., murine, solid cancers, since the progression of these cancers is dependent upon the ras protein signal transduction cascade and therefore susceptible to treatment by interruption of the cascade, i.e., by inhibiting raf kinase. Accordingly, the compounds of the invention are useful in treating solid cancers, such as, for example, carcinomas (e.g., of the lungs, pancreas, thyroid, bladder or colon), myeloid disorders (e.g., myeloid leukemia) or adenomas (e.g., villous colon adenoma).

The present invention, therefore, provides compounds generally described as aryl ureas, including both aryl and heteroaryl analogues, which inhibit the raf pathway. The invention also provides a method for treating a raf mediated disease state in humans or mammals. Thus, the invention is directed to compounds and methods for the treatment of cancerous cell growth mediated by raf kinase, comprising administering a compound of Formula I
wherein 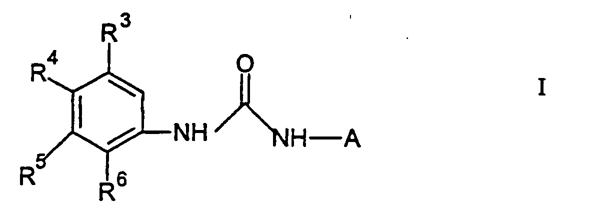 wherein
- A: is 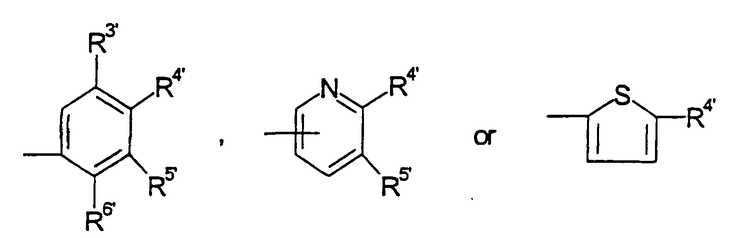
- R³, R⁴, R⁵ and R⁶: are each, independently, H, halogen, NO₂, C₁₋₁₀- alkyl, optionally substituted by halogen up to perhaloalkyl, C₁₋₁₀-alkoxy, optionally substituted by halogen up to perhaloalkoxy, C₆₋₁₂ aryl, optionally substituted by C₁₋₁₀ alkyl or C₁₋₁₀ alkoxy, or C₅₋₁₂ hetaryl, optionally substituted by C₁₋₁₀ alkyl or C₁₋₁₀ alkoxy,
and one of R³-R⁶ can be -X-Y;
or two adjacent R³-R⁶ can together be an aryl or hetaryl ring with 5-12 atoms, optionally substituted by C₁₋₁₀-alkyl, C₁₋₁₀-alkoxy, C₃₋₁₀-cycloalkyl, C₂₋₁₀-alkenyl, C₁₋₁₀-alkanoyl, C₆₋₁₂-aryl, C₅₋₁₂-hetaryl; C₆₋₁₂-aralkyl, C₆₋₁₂-alkaryl, halogen; NR¹R¹; -NO₂; -CF₃; -COOR¹; -NHCOR¹; -CN; -CONR¹R¹; -SO₂R²; -SOR²; -SR²; in which R¹ is H or C₁₋₁₀-alkyl and R² is C₁₋₁₀-alkyl, optionally substituted by halogen, up to perhalo with -S(O₂)- optionally incorporated in the aryl or hetaryl ring;
- R^{4'}, R^{5'} and R^{6'}: are independently H, halogen, C₁ - C₁₀ alkyl, optionally substituted by halogen up to perhaloalkyl, or by 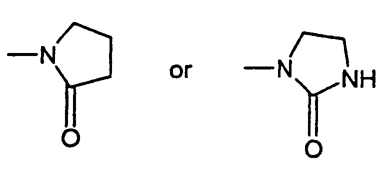 C₁ -C₁₀ alkoxy optionally substituted by halogen up to perhaloalkoxy or -X-Y, and either one of R^{4'}, R^{5'} or R^{6'} is -X-Y or two adjacent of R^{4'}, R^{5'} and R^{6'} together are a hetaryl ring with 5-12 atoms optionally substituted by C₁₋₁₀alkyl, C₁₋₁₀ alkoxy, C₃₋₁₀ cycloalkyl, C₂₋₁₀ alkenyl, C₁₋₁₀ alkanoyl, C₆₋₁₂ aryl, C₅₋₁₂ hetaryl or C₆₋₁₂ aralkyl;
- R^{6'}: is additionally -NHCOR¹, - NR¹COR¹ or NO₂;
- R¹: is C₁₋₁₀ alkyl optionally substituted by halogen up to perhalo;
- R^{3'}: is H, halogen, C₁-C₁₀ alkyl optionally substituted by halogen up to perhaloalkyl, C₁-C₁₀ alkoxy, optionally substituted by halogen up to perhaloalkoxy;
- X: is -CH₂-, -S- -N(CH₃)-, -NHC(O)- -CH₂-S-, -S-CH₂-, -C(O)-, or -O-; and
- X: is additionally a single bond where Y is pyridyl; and
- Y: is phenyl, pyridyl, naphthyl, pyridone, pyrazine, pyrimidine, benzodioxane, benzopyridine or benzothiazole, each optionally substituted by C₁₋₁₀-alkyl, C₁₋₁₀-alkoxy, halogen, OH, -SCH₃, NO₂ or, where Y is phenyl, by 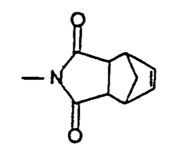 or a pharmaceutically acceptable salt thereof, with the proviso that if X is -O- or -S- , R^{3'} and R^{6'} are H, and Y is phenyl unsubstituted by OH, then R⁶ is alkoxy.

Preferably, R³ is halogen or C₁₋₁₀- alkyl, optionally substituted by halogen, up to perhaloalkyl; R⁴ is H, halogen or NO₂; R⁵ is H, halogen or C₁₋₁₀- alkyl; and R⁶ is H or C₁₋₁₀- alkoxy. More preferably, R³ is C₄₋₁₀-alkyl, Cl, F or CF₃; R⁴ is H, Cl, F or NO₂; R⁵ is H, Cl, F or C₄₋₁₀-alkyl; and R⁶ is H or OCH₃. Still more preferably, R³ or R⁴ is t-butyl. X is preferably -CH₂- or -S- and Y is phenyl or pyridyl, or X is -O- and Y is preferably phenyl, pyridyl or benzthiazole.

The invention is also directed to a compound of the formula

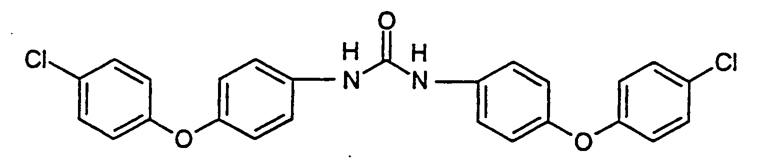

The invention is further directed to a method for the treatment of a cancerous cell growth mediated by raf kinase, comprising administering a compound of Formula II: 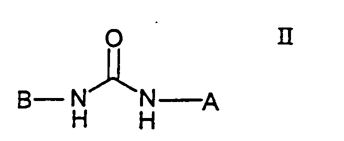 wherein
A is 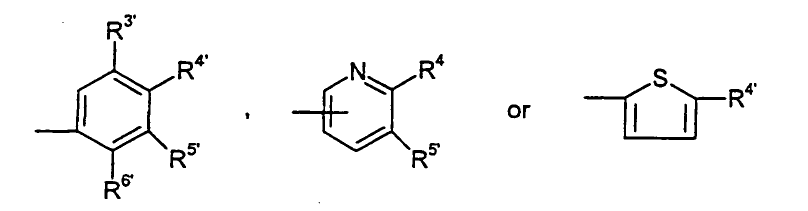
B is a substituted or unsubstituted, up to tricyclic aryl or heteroaryl moiety of up to 30 carbon atoms with at least one 6-member aromatic structure containing 0-4 members of the group consisting of nitrogen, oxygen and sulfur, wherein if B is substituted it is substituted by one or more substituents selected from the group consisting of halogen, up to per-halo, and Wₙ, wherein n is 0-3 and each W is independently selected from the group consisting of -CN, -CO₂R⁷, -C(O)NR⁷R⁷, -C(O)-R⁷, -NO₂, -OR⁷, - SR⁷, - NR⁷R⁷, -NR⁷C(O)OR⁷, -NR⁷C(O)R⁷, C₁-C₁₀ alkyl, C₂-C₁₀ alkenyl, C₁-C₁₀ alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₃-C₁₀ cycloalkyl, substituted C₂-C₁₀ alkenyl, substituted C₁-C₁₀ alkoxy, substituted C₄-C₂₃ alkheteroaryl and Q-Ar;
   wherein if W is a substituted group, it is substituted by one or more substituents independently selected from the group consisting of -CN, -CO₂R⁷, -C(O)R⁷, -C(O)NR⁷R⁷, -OR⁷, -SR⁷, -NR⁷R⁷, NO₂, -NR⁷C(O)R⁷, -NR⁷C(O)OR⁷ and halogen up to per-halo;
   wherein each R⁷ is independently selected from H, C₂-C₁₀ alkenyl, C₁-C₁₀ alkyl, C₃-C₁₀ cycloalkyl. C₆-C₁₄ aryl, C₃-C₁₃ hetaryl, C₇-C₂₄ alkaryl, C₄-C₂₃ alkheteroaryl, up to per-halosubstituted C₁-C₁₀ alkyl, up to per-halosubstituted C₂-C₁₀ alkenyl, up to per-halosubstituted C₃-C₁₀ cycloalkyl, up to per-halosubstituted C₆-C₁₄ aryl and up to per-halosubstituted C₃-C₁₃ hetaryl,
   wherein Q is -O-, -S-, -N(R⁷)-, -(CH₂)-ₘ, -C(O)-, -CH(OH)-, -(CH₂)ₘO-, -NR⁷C(O)NR⁷R⁷-, -NR⁷C(O)-, -C(O)NR⁷-, -(CH₂)ₘS-, -(CH₂)ₘN(R⁷)-, -O(CH₂)ₘ-, -CHX^{a}, -CX^{a}₂-, -S-(CH₂)ₘ- and -N(R⁷)(CH₂)ₘ-,
m = 1-3, and X^{a} is halogen; and
Ar is a 5-10 member aromatic structure containing 0-2 members of the group consisting of nitrogen, oxygen and sulfur, which is unsubstituted or substituted by halogen up to per-halo and optionally substituted by Zₙ₁, wherein ₙ₁ is 0 to 3 and each Z is independently selected from the group consisting of -CN, -CO₂R⁷, -C(O)NR⁷R⁷, -C(O)- NR⁷, -NO₂, -OR⁷, - SR⁷, - NR⁷R⁷, -NR⁷C(O)OR⁷, -C(O)R⁷, -NR⁷C(O)R⁷, C₁-C₁₀ alkyl, C₃-C₁₀ cycloalkyl, C₆-C₁₄ aryl, C₃-C₁₃ hetaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein the one or more substituents of Z is selected from the group consisting of -CN, -CO₂R⁷, -C(O)NR⁷R⁷, -OR⁷, -SR⁷, -NO₂, -NR⁷R⁷, -NR⁷C(O)R⁷ and -NR⁷C(O)OR⁷,
R^{4'}, R^{5'} and R^{6'} are each independently H, halogen, C₁₋₁₀- alkyl, optionally substituted by halogen up to perhaloalkyl, 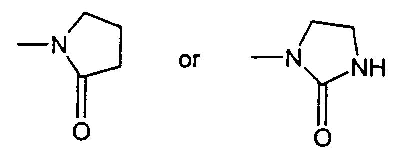 C₁-C₁₀ alkoxy, optionally substituted by halogen up to perhaloalkoxy or -X-Y, and
   either one of R^{4'}, R^{5'} or R^{6'} is -X-Y or two adjacent of R^{4'}, R^{5'} and R^{6'} together are a hetaryl ring with 5-12 atoms optionally substituted by C₁₋₁₀ alkyl, C₁₋₁₀ alkoxy, C₃₋₁₀ cycloalkyl, C₂₋₁₀ alkenyl, C₁₋₁₀ alkanoyl, C₆₋₁₂ aryl, C₅₋₁₂ hetaryl or C₆₋₁₂ aralkyl;

- R^{6'}: is additionally -NHCOR¹, - NR¹COR¹ or NO₂;
- R¹: is C₁₋₁₀ alkyl optionally substituted by halogen up to perhalo;
- R^{3'}: is independently H, halogen, C₁₋₁₀ alkyl, optionally substituted by halogen up to perhaloalkyl, C₁₋₁₀ alkoxy, optionally substituted by halogen up to perhaloalkoxy;
- X: is -CH₂-, -S-, -N(CH₃)-, -NHC(O)-, -CH₂-S-, -C(O)-, or -O-;
- X: is additionally a single bond where Y is pyridyl; and
- Y: is phenyl, pyridyl, naphthyl, pyridone, pyrazine, pyrimidine, benzodioxane, benzopyridine or benzothiazole, each optionally substituted by C₁₋₁₀-alkyl, C₁₋₁₀-alkoxy, halogen, OH, -SCH_{3,} or NO₂ or, where Y is phenyl, by 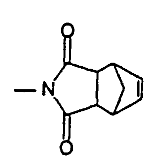 or a pharmaceutically acceptable salt thereof.

Preferably, compounds of formula II are of formula IIa: 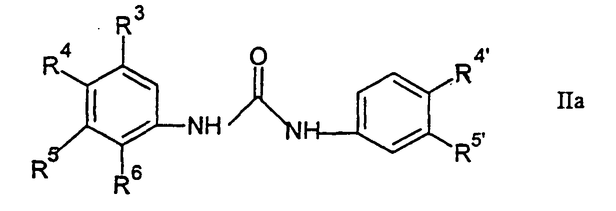 wherein
R³, R⁴, R⁵ and R⁶ are each independently H, halogen, NO₂, C₁₋₁₀- alkyl, optionally substituted by halogen, up to perhaloalkyl, or C₁₋₁₀-alkoxy, optionally substituted by halogen, up to perhalo; and one of R³-R⁶ can be -X-Y; or two adjacent R³-R⁶ can together be an aryl or hetaryl ring with 5-12 atoms, optionally substituted by C₁₋₁₀-alkyl, C₁₋₁₀-alkoxy, C₃₋₁₀-cycloalkyl, C₂₋₁₀-alkenyl, C₁₋₁₀-alkanoyl; C₆₋₁₂-aryl, C₅₋₁₂-hetaryl, C₆₋₁₂-alkaryl, halogen; -NR¹; -NO₂; -CF₃; -COOR¹; -NHCOR¹; -CN; -CONR¹R¹; -SO₂R²; -SOR²; -SR²; in which R¹ is H or C₁₋₁₀-alkyl, optionally substituted by halogen, up to perhalo, and R² is C₁₋₁₀-alkyl, optionally substituted by halogen, up to perhalo.

In formula I, suitable hetaryl groups B include, but are not limited to, 5-12 carbon-atom aromatic rings or ring systems containing 1-3 rings, at least one of which is aromatic, in which one or more, e.g., 1-4 carbon atoms in one or more of the rings can be replaced by oxygen, nitrogen or sulfur atoms. Each ring typically has 3-7 atoms. For example, B can be 2- or 3-furyl, 2- or 3-thienyl, 2- or 4-triazinyl, 1-, 2- or 3-pyrrolyl, 1-, 2-, 4- or 5-imidazolyl, 1-, 3-, 4- or 5-pyrazolyl, 2-, 4- or 5-oxazolyl, 3-, 4-or 5-isoxazolyl, 2-, 4- or 5-thiazolyl, 3-, 4- or 5-isothiazolyl, 2-, 3- or 4-pyridyl, 2-, 4-, 5- or 6-pyrimidinyl, 1,2,3-triazol-1-, -4- or -5-yl, 1,2,4-triazol-1-, -3- or -5-yl, 1- or 5-tetrazolyl, 1,2,3-oxadiazol-4- or -5-yl, 1,2,4-oxadiazol-3- or -5-yl, 1,3,4-thiadiazol-2-or -5-yl, 1,2,4-oxadiazol-3- or -5-yl, 1,3,4-thiadiazol-2- or -5-yl, 1,3,4-thiadiazol-3-or -5-yl, 1,2,3-thiadiazol-4- or -5-yl, 2-, 3-, 4-, 5- or 6-2H-thiopyranyl, 2-, 3- or 4-4H-thiopyranyl, 3- or 4-pyridazinyl, pyrazinyl, 2-, 3-, 4-, 5-, 6- or 7-benzofuryl, 2-, 3-, 4-, 5-, 6- or 7-benzothienyl, 1-, 2-, 3-, 4-, 5-, 6- or 7-indolyl, 1-, 2-, 4- or 5-benzimidazolyl, 1-, 3-, 4-, 5-, 6- or 7-benzopyrazolyl, 2-, 4-, 5-, 6- or 7-benzoxazolyl, 3-, 4-, 5- 6- or 7-benzisoxazolyl, 1-, 3-, 4-, 5-, 6- or 7-benzothiazolyl, 2-, 4-, 5-, 6- or 7-benzisothiazolyl, 2-, 4-, 5-, 6- or 7-benz-1,3-oxadiazolyl, 2-, 3-, 4-, 5-, 6-, 7- or 8-quinolinyl, 1-, 3-, 4-, 5-, 6-, 7-, 8- isoquinolinyl, 1-, 2-, 3-, 4- or 9-carbazolyl, 1-, 2-, 3-, 4-, 5-, 6-, 7-, 8- or 9-acridinyl, or 2-, 4-, 5-, 6-, 7- or 8-quinazolinyl, or additionally optionally substituted phenyl, 2- or 3-thienyl, 1,3,4-thiadiazolyl, 3-pyrryl, 3-pyrazolyl, 2-thiazolyl or 5-thiazolyl, etc. For example, B can be 4-methyl-phenyl, 5-methyl-2-thienyl, 4-methyl-2-thienyl, 1-methyl-3-pyrryl, 1-methyl-3-pyrazolyl, 5-methyl-2-thiazolyl or 5-methyl-1,2,4-thiadiazol-2-yl.

Suitable alkyl groups and alkyl portions of groups, e.g., alkoxy, etc. throughout include methyl, ethyl, propyl, butyl, etc., including all straight-chain and branched isomers such as isopropyl, isobutyl, *sec*-butyl, *tert*-butyl, etc.

Suitable aryl groups include, for example, phenyl and 1- and 2-naphthyl.

Suitable cycloalkyl groups include cyclopropyl, cyclobutyl, cyclohexyl, etc. The term "cycloalkyl", as used herein, refers to cyclic structures with or without alkyl substitutents such that, for example, "C₄ cycloalkyl" includes methyl substituted cyclopropyl groups as well as cyclobutyl groups. The term "cycloalkyl" also includes saturated heterocyclic groups.

Suitable halogen groups include F, Cl, Br, and/or I, from one to per-substitution (i.e., all H atoms on a group replaced by a halogen atom) being possible where an alkyl group is substituted by halogen, mixed substitution of halogen atom types also being possible on a given moiety.

The present invention is also directed to pharmaceutically acceptable salts of Formula I. Suitable pharmaceutically acceptable salts are well known to those skilled in the art and include basic salts of inorganic and organic acids, such as hydrochloric acid, hydrobromic acid, phosphoric acid, methanesulphonic acid, trifluoromethanesulfonic acid, sulphonic acid, acetic acid, trifluoroacetic acid, malic acid tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include acid salts of inorganic bases, such as salts containing alkaline cations (e.g., Li⁺ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations such as those arising from protonation or peralkylation of triethylamine, *N,N*-diethylamine, N,N-dicyclohexylamine, pyridine, *N,N*-dimethylaminopyridine (DMAP), 1,4-diazabicyclo[2.2.2]octane (DABCO), 1,5-diazabicyclo[4.3.0]non-5-ene (DBN) and 1,8-diazabicyclo[5.4.0]undec-7-ene (DBU).

A number of the compounds of Formula I possess asymmetric carbons and can therefore exist in racemic and optically active forms. Methods of separation of enantiomeric and diastereomeric mixtures are well known to one skilled in the art. The present invention encompasses any isolated racemic or optically active form of compounds described in Formula I which possess Raf kinase inhibitory activity.

The compounds of Formula I may be prepared by use of known chemical reactions and procedures. Nevertheless, the following general preparative methods are presented to aid one of skill in the art in synthesizing the inhibitors, with more detailed examples being presented in the experimental section describing the working examples.

### General Preparative Methods

The compounds of Formula I may be prepared by the use of known chemical reactions and procedures, some from starting materials which are commercially available. Nevertheless, general preparative methods are provided below to aid one skilled in the art in synthesizing these compounds, with more detailed examples being provided in the experimental section which follows.

Substituted anilines may be generated using standard methods (March. *Advanced Organic Chemistry*, 3^{rd} Ed.; John Wiley: New York (1985). Larock. *Comprehensive Organic Transformations*; VCH Publishers: New York (1989)). As shown in Scheme I, aryl amines are commonly synthesized by reduction of nitroaryls using a metal catalyst, such as Ni, Pd, or Pt, and H₂ or a hydride transfer agent, such as formate, cyclohexadiene, or a borohydride (Rylander. *Hydrogenation Methods;* Academic Press: London, UK (1985)). Nitroaryls may also be directly reduced using a strong hydride source, such as LiAlH₄ (Seyden-Penne. *Reductions by the Alumino- and Borohydrides in Organic Synthesis*; VCH Publishers: New York (1991)), or using a zero valent metal, such as Fe, Sn or Ca, often in acidic media. Many methods exist for the synthesis of nitroaryls (March. *Advanced Organic Chemistry*, 3^{rd} Ed.; John Wiley: New York (1985). Larock. *Comprehensive Organic Transformations*; VCH Publishers: New York (1989)).

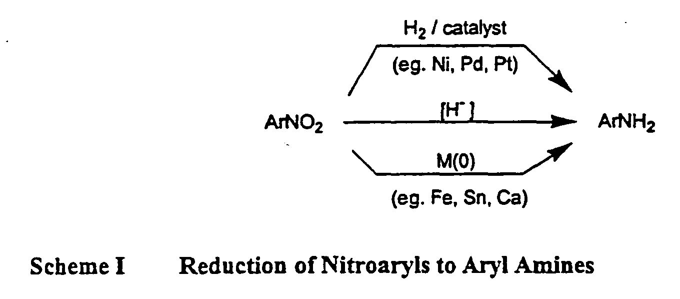

Nitroaryls are commonly formed by electrophilic aromatic nitration using HNO₃, or an alternative NO₂⁺ source. Nitroaryls may be further elaborated prior to reduction. Thus, nitroaryls substituted with 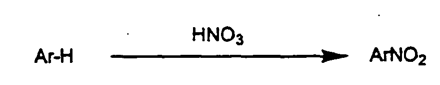 potential leaving groups (eg. F, Cl, Br, etc.) may undergo substitution reactions on treatment with nucleophiles, such as thiolate (exemplified in Scheme II) or phenoxide. Nitroaryls may also undergo Ullman-type coupling reactions (Scheme II).

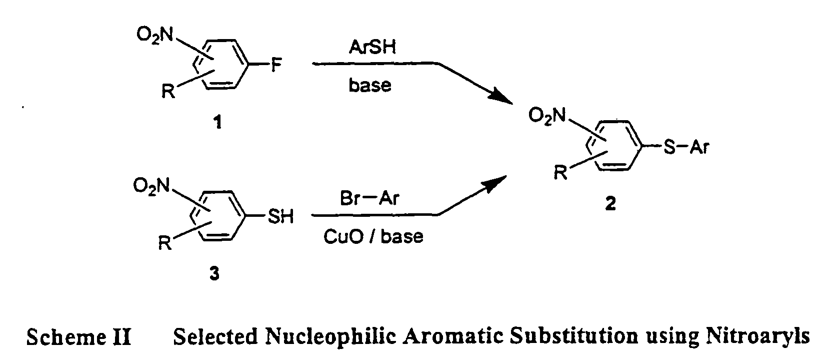

Nitroaryls may also undergo transition metal mediated cross coupling reactions. For example, nitroaryl electrophiles, such as nitroaryl bromides, iodides or triflates, undergo palladium mediated cross coupling reactions with aryl nucleophiles, such as arylboronic acids (Suzuki reactions, exemplified below), aryltins (Stille reactions) or arylzincs (Negishi reaction) to afford the biaryl (**5**).

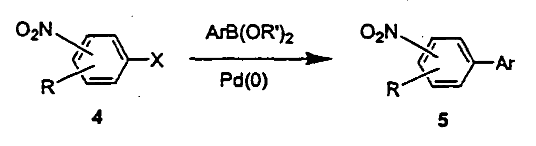

Either nitroaryls or anilines may be converted into the corresponding arenesulfonyl chloride (**7**) on treatment with chlorosulfonic acid. Reaction of the sulfonyl chloride with a fluoride source, such as KF then affords sulfonyl fluoride (**8**). Reaction of sulfonyl fluoride 8 with trimethylsilyl trifluoromethane in the presence of a fluoride source, such as tris(dimethylamino)sulfonium difluorotrimethylsiliconate (TASF) leads to the corresponding trifluoromethylsulfone (**9**). Alternatively, sulfonyl chloride **7** may be reduced to the arenethiol (**10**), for example with zinc amalgum. Reaction of thiol **10** with CHClF₂ in the presence of base gives the difluoromethyl mercaptam (**11**), which may be oxidized to the sulfone (**12**) with any of a variety of oxidants, including CrO₃-acetic anhydride (Sedova et al. *Zh. Org. Khim*. **1970**, *6*, (568). 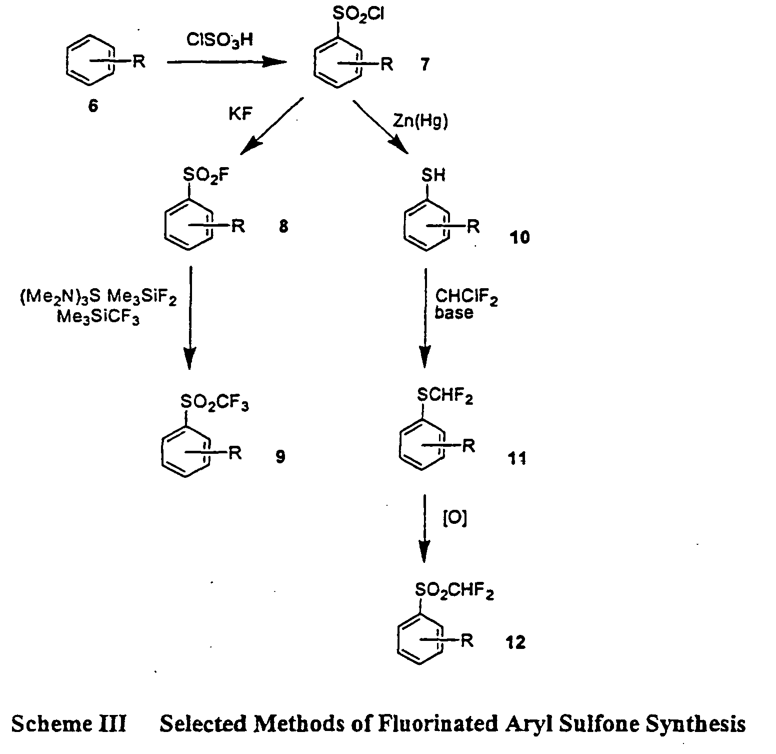 As shown in Scheme IV, non-symmetrical urea formation may involve reaction of an aryl isocyanate (**14**) with an aryl amine (**13**). The heteroaryl isocyanate may be synthesized from a heteroaryl amine by treatment with phosgene or a phosgene equivalent, such as trichloromethyl chloroformate (diphosgene), bis(trichloromethyl) carbonate (triphosgene), or *N,N'*-carbonyldiimidazole (CDI). The isocyanate may also be derived from a heterocyclic carboxylic acid derivative, such as an ester, an acid halide or an anhydride by a Curtius-type rearrangement. Thus, reaction of acid derivative 16 with an azide source, followed by rearrangement affords the isocyanate. The corresponding carboxylic acid (**17**) may also be subjected to Curtius-type rearrangements using diphenylphosphoryl azide (DPPA) or a similar reagent.

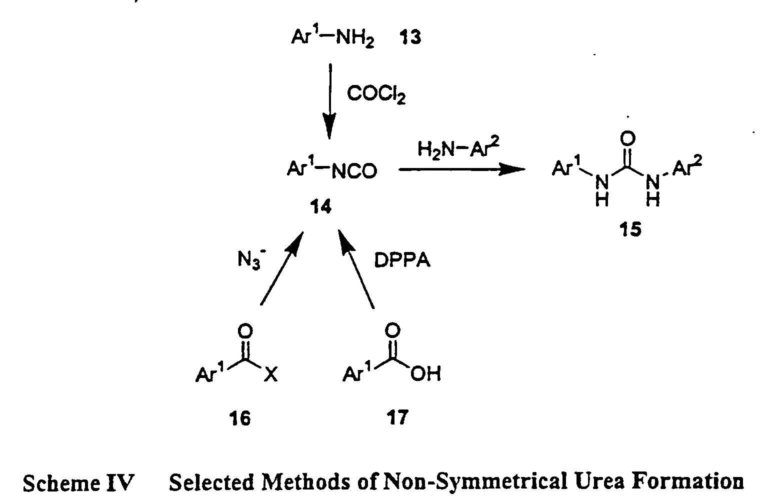

Finally, ureas may be further manipulated using methods familiar to those skilled in the art.

The invention also includes pharmaceutical compositions including a compound of Formula I, and a physiologically acceptable carrier.

The compounds may be administered orally, dermally, parenterally, by injection, by inhalation or spray, or sublingually rectally or vaginally in dosage unit formulations. The term 'administration by injection' includes intravenous, intraarticular, intramuscular, subcutaneous and parenteral injections, as well as use of infusion techniques. Dermal administration may include topical application or transdermal administration. One or more compounds may be present in association with one or more non-toxic pharmaceutically acceptable carriers and if desired other active ingredients.

Compositions intended for oral use may be prepared according to any suitable method known to the art for the manufacture of pharmaceutical compositions. Such compositions may contain one or more agents selected from the group consisting of diluents, sweetening agents, flavoring agents, coloring agents and preserving agents in order to provide palatable preparations. Tablets contain the active ingredient in admixture with non-toxic pharmaceutically acceptable excipients which are suitable for the manufacture of tablets. These excipients may be, for example, inert diluents, such as calcium carbonate, sodium carbonate, lactose, calcium phosphate or sodium phosphate; granulating and disintegrating agents, for example, corn starch, or alginic acid; and binding agents, for example magnesium stearate, stearic acid or talc. The tablets may be uncoated or they may be coated by known techniques to delay disintegration and adsorption in the gastrointestinal tract and thereby provide a sustained action over a longer period. For example, a time delay material such as glyceryl monostearate or glyceryl distearate may be employed. These compounds may also be prepared in solid, rapidly released form.

Formulations for oral use may also be presented as hard gelatin capsules wherein the active ingredient is mixed with an inert solid diluent, for example, calcium carbonate, calcium phosphate or kaolin, or as soft gelatin capsules wherein the active ingredient is mixed with water or an oil medium, for example peanut oil, liquid paraffin or olive oil.

Aqueous suspensions containing the active materials in admixture with excipients suitable for the manufacture of aqueous suspensions may also be used. Such excipients are suspending agents, for example sodium carboxymethylcellulose, methylcellulose, hydroxypropyl-methylcellulose, sodium alginate, polyvinylpyrrolidone, gum tragacanth and gum acacia; dispersing or wetting agents may be a naturally-occurring phosphatide, for example, lecithin, or condensation products of an alkylene oxide with fatty acids, for example polyoxyethylene stearate, or condensation products of ethylene oxide with long chain aliphatic alcohols, for example heptadecaethylene oxycetanol, or condensation products of ethylene oxide with partial esters derived from fatty acids and hexitol such as polyoxyethylene sorbitol monooleate, or condensation products of ethylene oxide with partial esters derived from fatty acids and hexitol anhydrides, for example polyethylene sorbitan monooleate. The aqueous suspensions may also contain one or more preservatives, for example ethyl, or *n*-propyl *p*-hydroxybenzoate, one or more coloring agents, one or more flavoring agents, and one or more sweetening agents, such as sucrose or saccharin.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example, sweetening, flavoring and coloring agents, may also be present.

The compounds may also be in the form of non-aqueous liquid formulations, e.g., oily suspensions which may be formulated by suspending the active ingredients in a vegetable oil, for example arachis oil, olive oil, sesame oil or peanut oil, or in a mineral oil such as liquid paraffin. The oily suspensions may contain a thickening agent, for example beeswax, hard paraffin or cetyl alcohol. Sweetening agents such as those set forth above, and flavoring agents may be added to provide palatable oral preparations. These compositions may be preserved by the addition of an anti-oxidant such as ascorbic acid.

Pharmaceutical compositions of the invention may also be in the form of oil-in-water emulsions. The oily phase may be a vegetable oil, for example olive oil or arachis oil, or a mineral oil, for example liquid paraffin or mixtures of these. Suitable emulsifying agents may be naturally-occurring gums, for example gum acacia or gum tragacanth, naturally-occurring phosphatides, for example soy bean, lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions may also contain sweetening and flavoring agents.

Syrups and elixirs may be formulated with sweetening agents, for example glycerol, propylene glycol, sorbitol or sucrose. Such formulations may also contain a demulcent, a preservative and flavoring and coloring agents.

The compounds may also be administered in the form of suppositories for rectal or vaginal administration of the drug. These compositions can be prepared by mixing the drug with a suitable non-irritating excipient which is solid at ordinary temperatures but liquid at the rectal temperature or vaginal temperature and will therefore melt in the rectum or vagina to release the drug. Such materials include cocoa butter and polyethylene glycols.

Compounds of the invention may also be administrated transdermally using methods known to those skilled in the art (see, for example: Chien; "Transdermal Controlled Systemic Medications"; Marcel Dekker, Inc.; 1987. Lipp et al. WO94/04157 3Mar94). For example, a solution or suspension of a compound of Formula I in a suitable volatile solvent optionally containing penetration enhancing agents can be combined with additional additives known to those skilled in the art, such as matrix materials and bacteriocides. After sterilization, the resulting mixture can be formulated following known procedures into dosage forms. In addition, on treatment with emulsifying agents and water, a solution or suspension of a compound of Formula I may be formulated into a lotion or salve.

Suitable solvents for processing transdermal delivery systems are known to those skilled in the art, and include lower alcohols such as ethanol or isopropyl alcohol, lower ketones such as acetone, lower carboxylic acid esters such as ethyl acetate, polar ethers such as tetrahydrofuran, lower hydrocarbons such as hexane, cyclohexane or benzene, or halogenated hydrocarbons such as dichloromethane, chloroform, trichlorotrifluoroethane, or trichlorofluoroethane. Suitable solvents may also include mixtures of one or more materials selected from lower alcohols, lower ketones, lower carboxylic acid esters, polar ethers, lower hydrocarbons, halogenated hydrocarbons.

Suitable penetration enhancing materials for transdermal delivery system are known to those skilled in the art, and include, for example, monohydroxy or polyhydroxy alcohols such as ethanol, propylene glycol or benzyl alcohol, saturated or unsaturated C₈-C₁₈ fatty alcohols such as lauryl alcohol or cetyl alcohol, saturated or unsaturated C₈-C₁₈ fatty acids such as stearic acid, saturated or unsaturated fatty esters with up to 24 carbons such as methyl, ethyl, propyl, isopropyl, n-butyl, sec-butyl, isobutyl, tertbutyl or monoglycerin esters of acetic acid, capronic acid, lauric acid, myristinic acid, stearic acid, or palmitic acid, or diesters of saturated or unsaturated dicarboxylic acids with a total of up to 24 carbons such as diisopropyl adipate, diisobutyl adipate, diisopropyl sebacate, diisopropyl maleate, or diisopropyl fumarate. Additional penetration enhancing materials include phosphatidyl derivatives such as lecithin or cephalin, terpenes, amides, ketones, ureas and their derivatives, and ethers such as dimethyl isosorbid and diethyleneglycol monoethyl ether. Suitable penetration enhancing formulations may also include mixtures of one or more materials selected from monohydroxy or polyhydroxy alcohols, saturated or unsaturated C₈-C₁₈ fatty alcohols, saturated or unsaturated C₈-C₁₈ fatty acids, saturated or unsaturated fatty esters with up to 24 carbons, diesters of saturated or unsaturated discarboxylic acids with a total of up to 24 carbons, phosphatidyl derivatives, terpenes, amides, ketones, ureas and their derivatives, and ethers.

Suitable binding materials for transdermal delivery systems are known to those skilled in the art and include polyacrylates, silicones, polyurethanes, block polymers, styrenebutadiene copolymers, and natural and synthetic rubbers. Cellulose ethers, derivatized polyethylenes, and silicates may also be used as matrix components. Additional additives, such as viscous resins or oils may be added to increase the viscosity of the matrix.

For all regimens of use disclosed herein for compounds of Formula I, the daily oral dosage regimen will preferably be from 0.01 to 200 mg/Kg of total body weight. The daily dosage for administration by injection, including intravenous, intramuscular, subcutaneous and parenteral injections, and use of infusion techniques will preferably be from 0.01 to 200 mg/Kg of total body weight. The daily vaginal dosage regime will preferably be from 0.01 to 200 mg/Kg of total body weight. The daily topical dosage regimen will preferably be from 0.1 to 200 mg administered between one to four times daily. The transdermal concentration will preferably be that required to maintain a daily does of from 0.01 to 200 mg/Kg. The daily inhalation dosage regimen will preferably be from 0.01 to 10 mg/Kg of total body weight.

It will be appreciated by those skilled in the art that the particular method of administration will depend on a variety of factors, all of which are considered routinely when administering therapeutics. It will also be understood, however, that the specific dose level for any given patient will depend upon a variety of factors, including, the activity of the specific compound employed, the age of the patient, the body weight of the patient, the general health of the patient, the gender of the patient, the diet of the patient, time of administration, route of administration, rate of excretion, drug combinations, and the severity of the condition undergoing therapy. It will be further appreciated by one skilled in the art that the optimal course of treatment, i.e., the mode of treatment and the daily number of doses of a compound of Formula I or a pharmaceutically acceptable salt thereof given for a defined number of days, can be ascertained by those skilled in the art using conventional treatment tests.

The compounds of Figure I are producible from known compounds (or from starting materials which, in turn, are producible from known compounds), e.g., through the general preparative methods shown above. The activity of a given compound to inhibit raf kinase can be routinely assayed, e.g., according to procedures disclosed below. The following examples are for illustrative purposes only and are not intended, nor should they be construed to limit the invention in any way.

The entire disclosure of all applications, patents and publications cited above and below, are hereby incorporated by reference, including provisional application (Attorney Docket Number Bayer 6 V1), filed on December 22, 1997, as SN 08/996,344 and converted on December 22,1998.

### EXAMPLES

All reactions were performed in flame-dried or oven-dried glassware under a positive pressure of dry argon or dry nitrogen, and were stirred magnetically unless otherwise indicated. Sensitive liquids and solutions were transferred via syringe or cannula, and introduced into reaction vessels through rubber septa. Unless otherwise stated, the term 'concentration under reduced pressure' refers to use of a Buchi rotary evaporator at approximately 15 mmHg.

All temperatures are reported uncorrected in degrees Celsius (°C). Unless otherwise indicated, all parts and percentages are by weight.

Commercial grade reagents and solvents were used without further purification. Thin-layer chromatography (TLC) was performed using Whatman® pre-coated glass-backed silica gel 60A F-254 250 µm plates. Visualization of plates was effected by one or more of the following techniques: (a) ultraviolet illumination, (b) exposure to iodine vapor, (c) immersion of the plate in a 10% solution of phosphomolybdic acid in ethanol followed by heating, (d) immersion of the plate in a cerium sulfate solution followed by heating, and/or (e) immersion of the plate in an acidic ethanol solution of 2,4-dinitrophenylhydrazine followed by heating. Column chromatography (flash chromatography) was performed using 230-400 mesh EM Science® silica gel.

Melting points (mp) were determined using a Thomas-Hoover melting point apparatus or a Mettler FP66 automated melting point apparatus and are uncorrected. Fourier transform infrared sprectra were obtained using a Mattson 4020 Galaxy Series spectrophotometer. Proton (¹H) nuclear magnetic resonance (NMR) spectra were measured with a General Electric GN-Omega 300 (300 MHz) spectrometer with either Me₄Si (d 0.00) or residual protonated solvent (CHCl₃ δ 7.26; MeOH δ 3.30; DMSO δ 2.49) as standard. Carbon (¹³C) NMR spectra were measured with a General Electric GN-Omega 300 (75 MHz) spectrometer with solvent (CDCl₃ δ 77.0; MeOD-d₃; δ 49.0; DMSO-d₆ δ 39.5) as standard. Low resolution mass spectra (MS) and high resolution mass spectra (HRMS) were either obtained as electron impact (EI) mass spectra or as fast atom bombardment (FAB) mass spectra. Electron impact mass spectra (EI-MS) were obtained with a Hewlett Packard 5989A mass spectrometer equipped with a Vacumetrics Desorption Chemical Ionization Probe for sample introduction. The ion source was maintained at 250 °C. Electron impact ionization was performed with electron energy of 70 eV and a trap current of 300 µA. Liquid-cesium secondary ion mass spectra (FAB-MS), an updated version of fast atom bombardment were obtained using a Kratos Concept 1-H spectrometer. Chemical ionization mass spectra (CI-MS) were obtained using a Hewlett Packard MS-Engine (5989A) with methane or ammonia as the reagent gas (1x10⁻⁴ torr to 2.5x10⁻⁴ torr). The direct insertion desorption chemical ionization (DCI) probe (Vaccumetrics, Inc.) was ramped from 0-1.5 amps in 10 sec and held at 10 amps until all traces of the sample disappeared ( ∼1-2 min). Spectra were scanned from 50-800 amu at 2 sec per scan. HPLC - electrospray mass spectra (HPLC ES-MS) were obtained using a Hewlett-Packard 1100 HPLC equipped with a quaternary pump, a variable wavelength detector, a C-18 column, and a Finnigan LCQ ion trap mass spectrometer with electrospray ionization. Spectra were scanned from 120-800 amu using a variable ion time according to the number of ions in the source. Gas chromatography - ion selective mass spectra (GC-MS) were obtained with a Hewlett Packard 5890 gas chromatograph equipped with an HP-1 methyl silicone column (0.33 mM coating; 25 m x 0.2 mm) and a Hewlett Packard 5971 Mass Selective Detector (ionization energy 70 eV). Elemental analyses are conducted by Robertson Microlit Labs, Madison NJ.

All compounds displayed NMR spectra, LRMS and either elemental analysis or HRMS consistant with assigned structures.

### List of Abbreviations and Acronyms:

- AcOH: acetic acid
- anh: anhydrous
- BOC: *tert*-butoxycarbonyl
- conc: concentrated
- dec: decomposition
- DMPU: 1,3-dimethyl-3,4,5,6-tetrahydro-2(1H)-pyrimidinone
- DMF: *N,N*-dimethylformamide
- DMSO: dimethylsulfoxide
- DPPA: diphenylphosphoryl azide
- EtOAc: ethyl acetate
- EtOH: ethanol (100%)
- Et₂O: diethyl ether
- Et₃N: triethylamine
- *m*-CPBA: 3-chloroperoxybenzoic acid
- MeOH: methanol
- pet. ether: petroleum ether (boiling range 30-60 °C)
- THF: tetrahydrofuran
- TFA: trifluoroacetic acid
- Tf: trifluoromethanesulfonyl

### A. General Methods for Synthesis of Substituted Anilines

### A1. Synthesis of 2,5-Dioxopyrrolidinylanilines

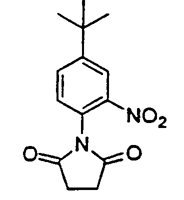

**Step 1. 4-*tert*-Butyl-1-(2,5-dioxo-1-pyrrolidinyl)-2-nitrobenzene:** To a solution of 4-*tert*-butyl-2-nitroaniline (1.04 g, 5.35 mmol) in xylene (25 mL) was added succinic anhydride (0.0535 g, 5.35 mmol) and triethylamine (0.75 mL, 5.35 mmol). The reaction mixture was heated at the reflux temp. for 24 h, cooled to room temp. and diluted with Et₂O (25 mL). The resulting mixture was sequentially washed with a 10% HCl solution (50 mL), a saturated NH₄Cl solution (50 mL) and a saturated NaCl solution (50 mL), dried (MgSO₄), and concentrated under reduced pressure. The residue was purified by flash cromatography (60% EtOAc/40% hexane) to yield the succinimide as a yellow solid (1.2 g, 86%): mp 135-138 °C; ¹H NMR (CHCl₃) δ 1.38 (s, 9H), 2.94-2.96 (m, 4H), 7.29-7.31 (m, 1H), 7.74-7.78 (m, 1H), 8.18-8.19 (m, 1H).

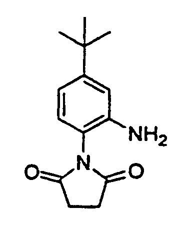

**Step 2. 5-*tert*-Butyl-2-(2,5-dioxo-1-pyrrolidinyl)aniline:** To a solution of 4-*tert*-butyl-1-(2,5-dioxo-1-pyrrolidinyl)-2-nitrobenzene (1.1 g, 4.2 mmol) in EtOAc (25 mL) was added a 10% Pd/C (0.1 g). The resulting slurry was placed under a H₂ atmosphere using 3 cycles of an evacuate-quench protocol and was allowed to stir under a H₂ atmosphere for 8 h. The reaction mixture was filtered through a pad of Celite® and the residue was washed with CHCl₃. The combined filtrate was concentrated under reduced pressure to yield the desired aniline as an off-white solid (0.75 g, 78%): mp 208-211 °C; ¹H-NMR (DMSO-d₆) δ 1.23 (s, 9H), 2.62-2.76 (m, 4H), 5.10 (br s, 2H), 6.52-6,56 (m, 1H), 6.67-6.70 (m, 2H).

### A2. General Method for the Synthesis of Tetrahydrofuranyloxyanilines

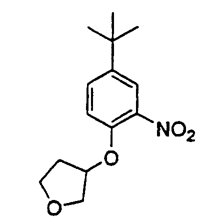

**Step 1.4-*tert*-Butyl-1-(3-tetrahydrofuranyloxy)-2-nitrobenzene:** To a solution of 4-*tert*-butyl-2-nitrophenol (1.05 g, 5.4 mmol) in anh THF (25 mL) was added 3-hydroxytetrahydrofuran (0.47 g, 5.4 mmol) and triphenylphosphine (1.55 g, 5.9 mmol) followed by diethyl azodicarboxylate (0.93 ml, 5.9 mmol) and the mixture was allowed to stir at room temp. for 4 h. The resulting mixture was diluted with Et₂O (50 mL) and washed with a saturated NH₄Cl solution (50 mL) and a saturated NaCl solution (50 mL), dried (MgSO₄), and concentrated under reduced pressure. The residue was purified by flash cromatography (30% EtOAc/70% hexane) to yield the desired ether as a yellow solid (1.3 g, 91%): ¹H-NMR (CHCl₃) δ 1.30 (s, 9H), 2.18-2.24 (m, 2H), 3 .91-4.09 (m, 4H), 5.00-5.02 (m, 1H), 6.93 (d, *J*=8.8 Hz, 1H), 7.52 (dd, *J*=2.6, 8.8 Hz, 1H), 7.81 (d, *J*=2.6 Hz, 1H).

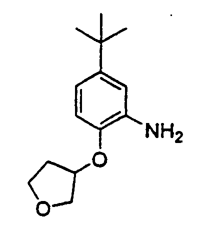

**Step 2.5-*tert*-Butyl-2-(3-tetrahydrofuranyloxy)aniline:** To a solution of 4-*tert*-butyl-1-(3-tetrahydrofuranyloxy)-2-nitrobenzene (1.17 g, 4.4 mmol) in EtOAc (25 mL) was added 10% Pd/C (0.1). The resulting slurry was placed under a H₂ atmosphere using 3 cycles of an evacuate-quench protocol and was allowed to stir under a H₂ atmosphere for 8 h. The reaction mixture was filtered through a pad of Celite® and washed with CHCl₃. The combined filtrate was concentrated under reduced pressure to yield of the desired aniline as a yellow solid (0.89 g, 86%): mp 79-82 °C; ¹H-NMR (CHCl₃) δ 1.30 (s, 9H), 2.16-2.20 (m, 2H), 3.78 (br s, 2H), 3.85-4.10 (m, 4H),4.90 (m, 1H), 6.65-6.82 (m, 3H).

### A3. General Method for the Synthesis of Trifluoromethanesulfonylanilines

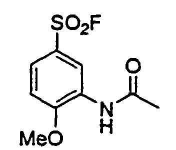

**Step 1. 2-Methoxy-5-(fluorosulfonyl)acetanilide:** Acetic anhydride (0.90 mL, 9.6 mmol) was added to a solution of 4-methoxymetanilyl fluoride (1.0 g, 4.8 mmol) in pyridine (15 mL). After being stirred at room temp. for 4 h, the reaction mixture was concentrated under reduced pressure. The resulting residue was dissolved in CH₂Cl₂ (25 mL), washed with a saturated NaHCO₃ solution (25 mL), dried (Na₂SO₄), and concentrated under reduced pressure to give a foam which was triturated with a Et₂O/hexane solution to provide the title compound (0.85 g): ¹H-NMR (CDCl₃) δ 2.13 (s, 3H), 3.98 (s, 3H), 7.36 (d, *J*=8.5 Hz, 1H), 7.82 (dd, *J*=2.6, 8.8 Hz, 1H), 8.79 (d, J=2.2 Hz, 1H), 9.62 (br s, 1H).

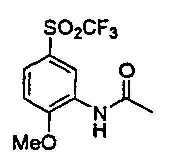

**Step 2.2-Methoxy-5-(trifluoromethanesulfonyl)acetanilide:** To an ice-cooled suspension of tris(dimethylamino)sulfonium difluorotrimethylsiliconate (0.094 g, 0.34 mmol) in THF (4 mL) was added a solution of (trifluoromethyl)trimethylsilane (1.0 mL, 6.88 mmol) in THF (3 mL) followed by a solution of 2-methoxy-5-(fluorosulfonyl)acetanilide (0.85 g, 3.44 mmol) in THF (3 mL). The reaction mixture was stirred for 2 h on an ice bath, then was allowed to warm to room temp. and was then concentrated under reduced pressure. The resulting residue was dissolved in CH₂Cl₂ (25 mL), washed with water (25 mL), dried (Na₂SO₄), and concentrated under reduced pressure. The resulting material was purified by flash chromatography (3% MeOH/97% CH₂Cl₂) to provide the title compound as a white solid (0.62 g): ¹H-NMR (CDCl₃) δ 2.13 (s, 3H) 4.00 (s, 3H), 7.42 (d, *J*=8.8 Hz, 1H), 7.81 (dd, *J*=2.6, 8.8 Hz, 1H), 8.80 (d, *J*=2.2 Hz, 1H), 9.64 (br s, 1H); FAB-MS *m*/*z* 298 ((M+1)⁺).

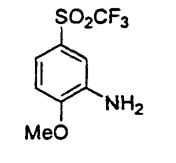

**Step 3.2-Methoxy-5-(trifluoromethanesulfonyl)aniline:** A solution of 2-methoxy-5-(trifluoromethanesulfonyl)acetanilide (0.517 g, 1.74 mmol) in EtOH (5 mL) and a 1 N HCl solution (5 mL) was heated at the reflux temp. for 4 h and the resulting mixture was concentrated under reduced pressure. The residue was dissolved in CH₂Cl₂ (30 mL), washed with water (30 mL), dried (Na₂SO₄), and concentrated under reduced pressure to afford the title compound as a gum (0.33 g): ¹H-NMR (CDCl₃) δ 3.90 (s, 3H) 5.57 (br s, 2H), 7.11-7.27 (m, 3H); FAB-MS *m*/*z* 256 ((M+1)⁺). This material was used in urea formation without further purification.

### A4. General Method for Aryl Amine Formation via Phenol Nitration Followed by Ether Formation and Reduction

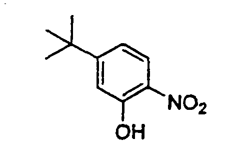

**Step 1.2-Nitro-5-*tert*-butylphenol** : A mixture of fuming nitric acid (3.24 g, 77.1 mmol) in glacial HOAc (10 mL) was added dropwise to a solution of *m-tert*-butylphenol (11.58 g, 77.1 mmol) in glacial HOAc (15 mL) at 0 °C. The mixture was allowed to stir at 0 °C for 15 min then warmed to room temp. After 1 h the mixture was poured into ice water (100 mL) and extracted with Et₂O (2 x 50 mL). The organic layer was washed with a saturated NaCl solution (100 mL), dried (MgSO₄) and concentrated *in vacuo.* The residue was purified by flash chromatography (30% EtOAc/70% hexane) to give the desired phenol (4.60 g, 31 %): ¹H-NMR (DMSO-d₆) δ 1.23 (s, 9H), 7.00 (dd, *J*=1.84, 8.83 Hz, 1H), 7.07 (d, *J*=1.84 Hz, 1H), 7.82 (d, *J*=8.83 Hz, 1H), 10.74 (s, 1H).

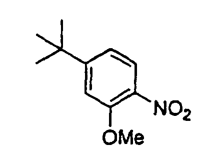

**Step 2. 2-Nitro-5-*tert*-butylanisole:** A slurry of 2-nitro-5-*tert*-butylphenol (3.68 g, 18.9 mmol) and K₂CO₃ (3.26 g, 23.6 mmol) in anh DMF (100 mL) was stirred at room temp with stirring for 15 min then treated with iodomethane (2.80 g, 19.8 mmol) via syringe. The reaction was allowed to stir at room temp for 18 h., then was treated with water (100 mL) and extracted with EtOAc (2 x 100 mL). The combined organic layers were washed with a saturated NaCl solution (50 mL), dried (MgSO₄) and concentrated *in vacuo* to give the desired ether (3.95 g, 100%): ¹H-NMR (DMSO-d₆) δ 1.29 (s, 9H), 3.92 (s, 3H), 7.10 (dd, *J*=1.84, 8.46 Hz, 1H), 7.22 (d, *J*=1.84 Hz, 1H), 7.79 (d, *J*=8.46 Hz, 1H). This material was used in the next step without further purification.

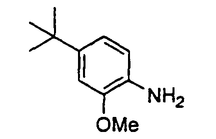

**Step 3. 4-*tert*-Butyl-2-methoxyaniline:** A solution of 2-nitro-5-*tert*-butylanisole (3.95 g, 18.9 mmol) in MeOH (65 mL) and added to a flask containing 10% Pd/C in MeOH (0.400 g), then placed under a H₂ atmosphere (balloon). The reaction was allowed to stir for 18 h at room temp, then filtered through a pad of Celite® and concentrated *in vacuo* to afford the desired product as a dark sitcky solid (3.40 g, 99%): ¹H-NMR (DMSO-d₆) δ 1.20 (s, 9H), 3.72 (s, 3H), 4.43 (br s, 2H), 6.51 (d, *J*=8.09 Hz, 1H), 6.64 (dd, *J*=2.21, 8.09 Hz, 1H), 6.76 (d, *J*=2.21 Hz, 1H).

### A5. General Method for Aryl Amine Formation via Carboxylic Acid Esterification Followed by Reduction

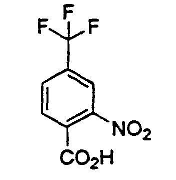

**Step 1. Methyl 2-Nitro-4-(trifluoromethyl)benzoate:** To a solution of 2-nitro-4-(trifluoromethyl)benzoic acid (4.0 g, 17.0 mmol) in MeOH (150 mL) at room temp was added conc H₂SO₄ (2.5 mL). The mixture was heated at the reflux temp for 24 h., cooled to room temp and concentrated in vacuo. The residue was diluted with water (100 mL) and extracted with EtOAc (2 x 100 mL). The combined organic layers were washed with a saturated NaCl solution, dried (MgSO₄), concentrated *in vacuo*. The residue was purified by flash chromatography (14% EtOAc/86% hexane) to give the desired ester as a pale yellow oil (4.17 g, 98%): ¹H-NMR (DMSO-d₆) δ 3.87 (s, 3H), 8.09 (d, *J*=7.72 Hz, 1H), 8.25 (dd, *J*=1.11, 8.09 Hz, 1H), 8.48 (d, *J*=1.11 Hz, 1H).

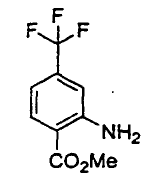

**Step 2. Methyl 2-Amino-4-(trifluoromethyl)benzoate:** A solution of methyl 2-nitro-4-(trifluoromethyl)benzoate (3.90 g, 15.7 mmol) in EtOAc (100 mL) and added to a flask containing 10% Pd/C (0.400 mg) in EtOAc (10 mL), then placed under a H₂ atmosphere (balloon). The reaction was allowed to stir for 18 h at room temp, then was filtered through Celite® and concentrated *in vacuo* to afford the desired product as a white crystalline solid (3.20 g, 93%): ¹H-NMR (DMSO-d₆) δ 3.79 (s, 3H), 6.75 (dd, *J*=1.84, 8.46 Hz, 1H), 6.96 (br s, 2H), 7.11 (d, *J*=0.73 Hz, 1H), 7.83 (d, *J=*8.09 Hz, 1H).

### A6. General Method for Aryl Amine Formation via Ether Formation Followed Ester Saponification, Curtius Rearrangement, and Carbamate Deprotection

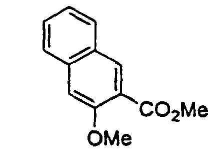

**Step 1. Methyl 3-Methoxy-2-naphthoate:** A slurry of methyl 3-hydroxy-2-naphthoate (10.1 g, 50.1 mmol) and K₂CO₃ (7.96 g, 57.6 mmol) in DMF (200 mL) was stirred at room temp for 15 min, then treated with iodomethane (3.43 mL, 55.1 mmol). The mixture was allowed to stir at room temp overnight, then was treated with water (200 mL). The resulting mixture was extracted with EtOAc (2 x 200 mL). The combined organic layers were washed with a saturated NaCl solution (100 mL), dried (MgSO₄), concentrated *in vacuo* (approximately 0.4 mmHg overnight) to give the desired ether as an amber oil (10.30 g): ¹H-NMR (DMSO-d₆) δ 2.70 (s, 3H), 2.85 (s, 3H), 7.38 (app t, *J*=8.09 Hz, 1H), 7.44 (s, 1H), 7.53 (app t, *J*=8.09 Hz, 1H), 7.84 (d, *J*=8.09 Hz, 1H), 7.90 (s, 1H), 8.21 (s, 1H).

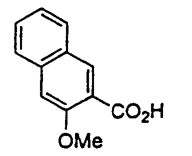

**Step 2. 3-Methoxy-2-naphthoic Acid**: A solution of methyl 3-methoxy-2-naphthoate (6.28 g, 29.10 mmol) and water (10 mL) in MeOH (100 mL) at room temp was treated with a 1 N NaOH solution (33.4 mL, 33.4 mmol). The mixture was heated at the reflux temp for 3 h, cooling to room temp, and made acidic with a 10% citric acid solution. The resulting solution was extracted with EtOAc (2 x 100 mL). The combined organic layers were washed with a saturated NaCl solution, dried (MgSO₄) and concentrated *in vacuo*. The residue was triturated with hexanes and washed several times with hexanes to give the desired carboxylic acid as a white crystalline solid (5.40 g, 92%): ¹H-NMR (DMSO-d₆) δ 3.88 (s, 3H), 7.34-7.41 (m, 2H), 7.49-7.54 (m, 1H), 7.83 (d, *J*=8.09 Hz, 1H), 7.91 (d, *J*=8.09 Hz, 1H), 8.19 (s, 1H), 12.83 (br s, 1H).

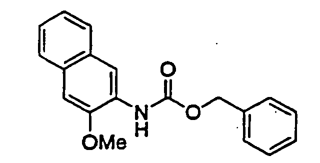

**Step 3. 2-(*N*-(Carbobenryloxy)amino-3-methoxynaphthalene**: A solution of 3-methoxy-2-naphthoic acid (3.36 g, 16.6 mmol) and Et₃N (2.59 mL, 18.6 mmol) in anh toluene (70 mL) was stirred at room temp. for 15 min., then treated with a solution of diphenylphosphoryl azide (5.12 g, 18.6 mmol) in toluene (10 mL) via pipette. The resulting mixture was heated at 80 °C for 2 h. After cooling the mixture to room temp. benzyl alcohol (2.06 mL, 20 mmol) was added via syringe. The mixture was then warmed to 80 °C overnight. The resulting mixture was cooled to room temp., quenched with a 10% citric acid solution, and extracted with EtOAc (2 x 100 mL). The combined organic layers were washed with a saturated NaCl solution, dried (MgSO₄), and concentrated *in vacuo*. The residue was purified by flash chromatography (14% EtOAc/86% hexane) to give the benzyl carbamate as a pale yellow oil (5.1 g, 100%): ¹H-NMR (DMSO-d₆) δ 3.89 (s, 3H), 5.17 (s, 2H), 7.27-7.44 (m, 8H), 7.72-7.75 (m, 2H), 8.20 (s, 1H), 8.76 (s, 1H).

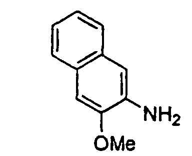

**Step 4.2-Amino-3-methoxynaphthalene**: A slurry of 2-(*N*-(carbobenzyloxy)amino-3-methoxynaphthalene (5.0 g, 16.3 mmol) and 10% Pd/C (0.5 g) in EtOAc (70mL) was maintained under a H₂ atmospheric (balloon) at room temp. overnight. The resulting mixture was filtered through Celite® and concentrated *in vacuo* to give the desired amine as a pale pink powder (2.40 g, 85%): ¹H-NMR (DMSO-d₆) δ 3.86 (s, 3H), 6.86 (s, 2H), 7.04-7.16 (m, 2H), 7.43 (d, *J*=8.0 Hz, 1H), 7.56 (d, *J*=8.0 Hz, 1H); EI-MS *m*/*z* 173 (M⁺).

### A7. General Method for the Synthesis of Aryl Amines via Metal-Mediated Cross Coupling Followed by Reduction

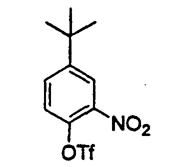

**Step 1.5-*tert*-Butyl-2-(trifluoromethanesulfonyl)oxy-1-nitrobenzene:** To an ice cold solution of 4-*tert*-butyl-2-nitrophenol (6.14 g, 31.5 mmol) and pyridine (10 mL, 125 mmol) in CH₂Cl₂ (50 mL) was slowly added trifluoromethanesulfonic anhydride (10 g, 35.5 mmol) via syringe. The reaction mixture was stirred for 15 min, then allowed to warm up to room temp. and diluted with CH₂Cl₂ (100 mL). The resulting mixture was sequentially washed with a 1M NaOH solution (3 x 100 mL), and a 1M HCl solution (3 x 100 mL), dried (MgSO₄), and concentrated under reduced pressure to afford the title compound (8.68 g, 84%): ¹H-NMR (CDCl₃) δ 1.39 (s, 9H), 7.30-8.20 (m, 3H).

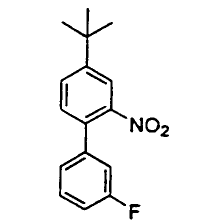

**Step 2.5-*tert*-Butyl-2-(3-fluorophenyl)-1-nitrobenzene:** A mixture of 3-fluorobenzeneboronic acid (3.80 g, 27.5 mmol), KBr (2.43 g, 20.4 mmol), K₃PO₄ (6.1 g, 28.8 mmol), and Pd(PPh₃)₄ (1.0 g, 0.9 mmol) was added to a solution of 5-*tert*-butyl-2-(trifluoromethanesulfonyl)oxy-1-nitrobenzene (6.0 g, 18.4 mmol) in dioxane (100 mL). The reaction mixture was heated at 80 °C for 24 h, at which time TLC indicated complete reaction. The reaction mixture was treated with a saturated NH₄Cl solution (50 mL) and extracted EtOAc (3 x 100 mL). The combined organic layers were dried (MgSO₄) and concentrated under reduced pressure. The residue was purified by flash chromatography (3% EtOAc/97% hexane) to give the title compound (4.07 g, 81 %): ¹H-NMR (CDCl₃) δ 1.40 (s, 9H), 6.90-7.90 (m, 7H).

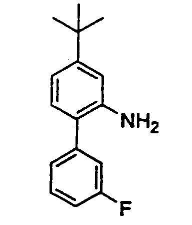

**Step 3.5-*tert*-Butyl-2-(3-fluorophenyl)aniline:** To a solution of 5-*tert*-butyl-2-(3-fluorophenyl)-1-nitrobenzene (3.5 g, 12.8 mmol) and EtOH (24 mL) in EtOAc (96 mL) was added 5% Pd/C (0.350 g) and the resulting slurry was stirred under a H₂ atmosphere for 24 h, at which time TLC indicated complete consumption of starting material. The reaction mixture was filtered through a pad of Celite® to give the desired product (2.2 g, 72%): ¹H-NMR (CDCl₃) δ 1.35 (s, 9H), 3.80 (br s, 2H), 6.90-7.50 (m, 7H).

### A8. General Method for the Synthesis of Nitroanilines

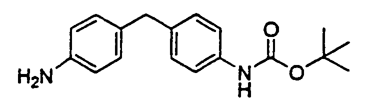

**Step 1.4-(4-(2-Propoxycarbonylamino)phenyl)methylaniline:** A solution of di-*tert*-butyl dicarbonate (2.0 g, 9.2 mmol) and 4,4'-methylenedianiline (1.8g, 9.2 mmol) in DMF (100 mL) was heated at the reflux temp. for 2 h, then cooled to room temp. This mixture was diluted with EtOAc (200 mL) sequentially washed with a saturated NH₄Cl (200 mL) and a saturated NaCl solution (100 mL), and dried (MgSO₄). The residue was purified by flash chromatography (30% EtOAc/70% hexane) to give the desired carbamate (1.3 g, 48%): ¹H-NMR (CDCl₃) δ 1.51 (s, 9H), 3.82 (s, 2H), 6.60-7.20 (m, 8H).

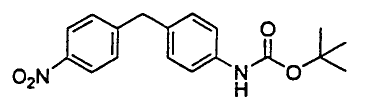

**Step 2.4-(4-(2-Propoxycarbonylamino)phenyl)methyl-1-nitrobenzene**: To an ice cold solution of 4-(4-(2-propoxycarbonylamino)phenyl)methylaniline (1.05 g, 3.5 mmol) in CH₂Cl₂ (15 mL) was added *m*-CPBA (1.2 g, 7.0 mmol). The reaction mixture was slowly allowed to warm to room temp. and was stirred for 45 min, at which time TLC indicated disappearance of starting material. The resulting mixture was diluted with EtOAc (50 mL), sequentially washed with a 1M NaOH solution (50 mL) and a saturated NaCl solution (50 mL), and dried (MgSO₄). The residue was purified by flash chromatography (20% EtOAc/80% hexane) to give the desired nitrobenzene (0.920 g): FAB-MS *m*/*z* 328 (M⁺).

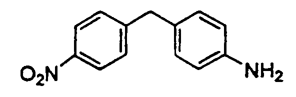

**Step 3.4-(4-Nitrophenyl)methylaniline**: To a solution of 4-(4-(2-propoxycarbonylamino)phenyl)methyl-1-nitrobenzene (0.920 g, 2.8 mmol) in dioxane (10 mL) was added a cone. HCl solution (4.0 mL) and the resulting mixture was heated at 80 °C for 1 h at which time TLC indicated disappearance of starting material. The reaction mixture was cooled to room temp. The resulting mixture was diluted with EtOAc (50 mL), then washed with a 1M NaOH solution (3 x 50 mL), and dried (MgSO₄) to give the desired aniline (0.570 mg, 89%): ¹H-NMR (CDCl₃) δ 3.70 (br s, 2H), 3.97 (s, 2H), 6.65 (d, *J*=8.5 Hz, 2H), 6.95 (d, *J*=8.5 Hz, 2H), 7.32 (d, *J*=8.8 Hz, 2H), 8.10 (d, *J*=8.8 Hz, 2H).

### A9. General Method for Synthesis of Aryl Anilines via Alkylation of a Nitrophenol Followed by Reduction

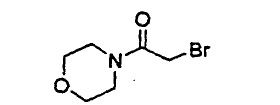

**Step 1.4-(α-Bromoacetyl)morpholine:** To an ice cold solution of morpholine (2.17 g, 24.9 mmol) and diisopropylethylamine (3.21 g, 24.9 mmol) in CH₂Cl₂ (70 mL) was added a solution of bromoacetyl bromide (5.05 g, 25 mmole) in CH₂Cl₂ (8 mL) via syringe. The resulting solution was kept at 0 °C for 45 min, then was allowed to warm to room temp. The reaction mixture was diluted with EtOAc (500 mL), sequentially washed with a 1M HCl solution (250 mL) and a saturated NaCl solution (250 mL), and dried (MgSO₄) to give the desired product (3.2 g, 62%): ¹H-NMR (DMSO-d₆) δ 3.40-3.50 (m, 4H), 3.50-3.60 (m, 4H), 4.11 (s, 2H).

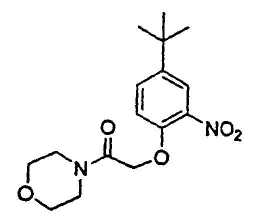

**Step 2.2-(*N*-Morpholinylcarbonyl)methoxy-5-*tert*-butyl-1-nitrobenzene**: A slurry of 4-*tert*-butyl-2-nitrophenol (3.9 g, 20 mmol) and K₂CO₃ (3.31 g, 24 mmol) in DMF (75 mL) was stirred at room temp. for 15 minutes, then a solution of 4-(α-bromoacetyl)morpholine (4.16 g, 20 mmol) in DMF (10 mL) was added. The reaction was allowed to stir at room temp. overnight, then was diluted with EtOAc (500 mL) and sequentially washed with a saturated NaCl solution (4 x 200 mL) and a 1M NaOH solution (400 mL). The residue was purified by flash chromatography (75% EtOAc/25% hexane) to give the nitrobenzene (2.13 g, 33%): ¹H-NMR (DMSO-d₆) δ 1.25 (s, 9H), 3.35-3.45 (m, 4H), 3.50-3.58 (m, 4H), 5.00 (s, 2H), 7.12 (d, *J*=8.8 Hz, 1H), 7.50-7.80 (m, 2H).

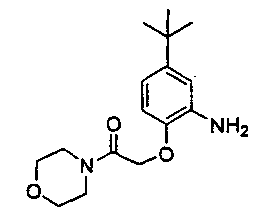

**Step 3.2-(*N*-Morpholinylcarbonyl)methoxy-5-*tert*-butylaniline**: To a solution of 2-(*N*-morpholinylcarbonyl)methoxy-5-*tert*-butyl-1-nitrobenzene(2.13 g, 6.6 mmol) and EtOH (10 mL) in EtOAc (40 mL) was added 5% Pd/C (0.215 g). The resulting slurry was stirred under a H₂ atmosphere for 6 h, at which time TLC indicated complete consumption of starting material. The reaction mixture was filtered through a pad of Celite® to give the desired product (1.9 g, 98%): ¹H-NMR (DMSO-d₆) δ 1.18 (s, 9H), 3.40-3.50 (m, 4H), 3.50-3.60 (m, 4H), 4.67 (brs, 2H), 4.69 (s, 2H), 6.40-6.70 (m, 3H).

### A10. General Method for Aryl Amine Formation via Nitrophenol Alkylation Followed by Reduction

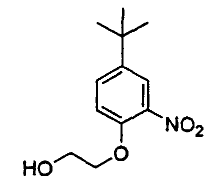

**Step 1.5-*tert*-Butyl-2-(2-hydroxyethoxy)-1-nitrobenzene:** A solution of 4-*tert*-butyl-2-nitrophenol (30 g, 0.15 mol) and tetra-n-butylammonium fluoride (0.771 g, 3.0 mmol) in ethylene carbonate (10.24 mL. 0.15 mol) was heated at 150 °C for 18 h, then cooled to room temp. and separated between water (50 mL) and CH₂Cl₂ (50 mL). The organic layer was dried (MgSO₄) and concentrated under reduced pressure. The residue was purified by column chromatography (20% EtOAc/80% hexane) to afford the desired product as a brown oil (35.1 g, 90%): ¹H-NMR (DMSO-d₆) δ 1.25 (s, 9H), 3.66-3.69 (m, 2H), 4.10-4.14 (t, *J*=5.0 Hz, 2H), 4.85 (t, *J*=5.0 Hz, 1H), 7.27 (d, *J*=8.8 Hz, 1H), 7.60-7.64 (m, 1H), 7.75 (d, *J*=2.6 Hz, 1H).

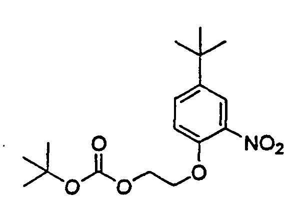

**Step 2.5-*tert*-Butyl-2-(2-*tert*-butoxycarbonyloxy)ethoxy)-1-nitrobenzene:** A solution of 5-*tert*-butyl-2-(2-hydroxyethoxy)-1-nitrobenzene (0.401 g, 1.68 mmol), di-*tert*-butyl dicarbonate (0.46 mL, 2.0 mmol) and dimethylaminopyridine (0.006 g, 0.05 mmol) in CH₂Cl₂ (15 mL) was stirred at room temp. for 30 min, at which time TLC indicated consumption of starting material. The resulting mixture was washed with water (20 mL), dried (MgSO₄) and concentrated under reduced pressure. The residue was purified by column chromatography (3% MeOH/97% CH₂Cl₂) to give the desired product as a yellow oil (0.291 g, 51%): ¹H-NMR (DMSO-d₆) δ 1.25 (s, 9H), 1.38 (s, 9H), 4.31 (br s, 4H), 7.27 (d, *J*=9.2 Hz, 1H) 7.64 (dd, *J*=2.6, 8.8 Hz, 1H) 7.77 (d, *J*=2.6 Hz, 1H).

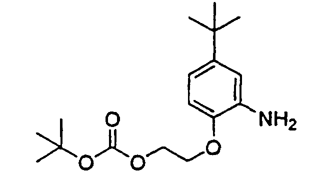

**Step 3.5-*tert*-Butyl-2-(2-*tert-*butoxycarbonyloxy)ethoxy)aniline:** To a mixture of 5-*tert*-butyl-2-(2-*tert*-butoxycarbonyloxy)ethoxy)-1-nitrobenzene (0.290 g, 0.86 mmol) and 5% Pd/C (0.058 g) in MeOH (2 mL) was ammonium formate (0.216 g, 3.42 mmol), and the resulting mixture was stirred at room temp. for 12 h, then was filtered through a pad of Celite® with the aid of EtOH. The filtrate was concentrated under reduced pressure and the residue was purified by column chromatography (2% MeOH/98% CH₂Cl₂) tp give the desired product as a pale yellow oil (0.232 g, 87%): TLC (20% EtOAc/80% hexane) R_{*f*} 0.63; ¹H-NMR (DMSO-d₆) δ 1. 17 (s, 9H), 1.39 (s, 9H), 4.03-4.06 (m, 2H), 4.30-4.31 (m, 2H), 4.54 (br s, 2H), 6.47 (dd, *J*=2.2, 8.1 Hz, 1H) 6.64-6.67 (m, 2H).

### A11. General Method for Substituted Aniline Formation via Hydrogenation of a Nitroarene

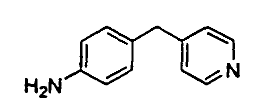

**4-(4-Pyridinylmethyl)aniline**: To a solution of 4-(4-nitrobenzyl)pyridine (7.0 g, 32.68 mmol) in EtOH (200 mL) was added 10% Pd/C (0.7 g) and the resulting slurry was shaken under a H₂ atmosphere (50 psi) using a Parr shaker. After 1 h, TLC and ¹H-NMR of an aliquot indicated complete reaction. The mixture was filtered through a short pad of Celite®. The filtrate was concentrated *in vacuo* to afford a white solid (5.4 g, 90%): ¹H-NMR (DMSO-d₆) δ 3.74 (s, 2H), 4.91 (br s, 2H), 6.48 (d, *J*=8.46 Hz, 2H), 6.86 (d, *J*=8.09 Hz, 2H), 7.16 (d, *J*=5.88 Hz, 2H), 8.40 (d, *J*=5.88 Hz, 2H); EI-MS *m*/*z* 184 (M⁺). This material was used in urea formation reactions without further purification.

### A12. General Method for Substituted Aniline Formation via Dissolving Metal Reduction of a Nitroarene

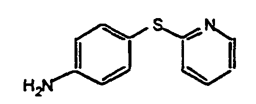

**4-(2-Pyridinylthio)aniline:** To a solution of 4-(2-pyridinylthio)-1-nitrobenzene (Menai ST 3355A; 0.220 g, 0.95 mmol) and H₂O (0.5 mL) in AcOH ( 5 mL) was added iron powder (0.317 g, 5.68 mmol) and the resulting slurry stirred for 16 h at room temp. The reaction mixture was diluted with EtOAc (75 mL) and H₂O (50 mL), basified to pH 10 by adding solid K₂CO₃ in portions (***Caution***: foaming). The organic layer was washed with a saturated NaCl solution, dried (MgSO₄), concentrated *in vacuo*. The residual solid was purified by MPLC (30% EtOAc/70% hexane) to give the desired product as a thick oil (0.135 g, 70%): TLC (30% EtOAc/70% hexanes) R_{*f*} 0.20.

### 2A13a. General Method for Substituted Aniline Formation via Nitroarene Formation Through Nucleophilic Aromatic Substitution, Followed by Reduction

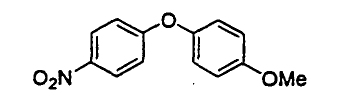

**Step 1. 1-Methoxy-4-(4-nitrophenoxy)benzene:** To a suspension of NaH (95%, 1.50 g, 59 mmol) in DMF (100 mL) at room temp. was added dropwise a solution of 4-methoxyphenol (7.39 g, 59 mmol) in DMF (50 mL). The reaction was stirred 1 h, then a solution of 1-fluoro-4-nitrobenzene (7.0 g, 49 mmol) in DMF (50 mL) was added dropwise to form a dark green solution. The reaction was heated at 95 °C overnight, then cooled to room temp., quenched with H₂O, and concentrated *in vacuo*. The residue was partitioned between EtOAc (200 mL) and H₂O (200 mL). The organic layer was sequentially washed with H₂O (2 x 200 mL), a saturated NaHCO₃ solution (200 mL), and a saturated NaCl solution (200 mL), dried (Na₂SO₄), and concentrated *in vacuo*. The residue was triturated (Et₂O/hexane) to afford 1-methoxy-4-(4-nitrophenoxy)benzene (12.2 g, 100%): ¹H-NMR (CDCl₃) δ 3.83 (s, 3H), 6.93-7.04 (m, 6H), 8.18 (d, *J*=9.2 Hz, 2H); EI-MS *m*/*z* 245 (M⁺).

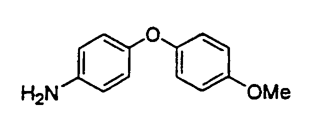

**Step 2. 4-(4-Methoxyphenoxy)aniline:** To a solution of 1-methoxy-4-(4-nitrophenoxy)benzene (12.0 g, 49 mmol) in EtOAc (250 mL) was added 5% Pt/C (1.5 g) and the resulting slurry was shaken under a H₂ atmosphere (50 psi) for 18 h. The reaction mixture was filtered through a pad of Celite® with the aid of EtOAc and concentrated *in vacuo* to give an oil which slowly solidified (10.6 g, 100%): ¹H-NMR (CDCl₃) δ 3.54 (br s, 2H), 3.78 (s, 3H), 6.65 (d, *J*=8.8 Hz, 2H), 6.79-6.92 (m, 6H); EI-MS *m*/*z* 215 (M⁺).

### A13b. General Method for Substituted Aniline Formation via Nitroarene Formation Through Nucleophilic Aromatic Substitution, Followed by Reduction

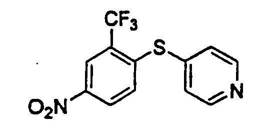

**Step 1. 3-(Trifluoromethyl)-4-(4-pyridinylthio)nitrobenzene:** A solution of 4-mercaptopyridine (2.8 g, 24 mmoles), 2-fluoro-5-nitrobenzotrifluoride (5 g, 23.5 mmoles), and potassium carbonate (6.1 g, 44.3 mmoles) in anhydrous DMF (80 mL) was stirred at room temperature and under argon overnight. TLC showed complete reaction. The mixture was diluted with Et₂O (100 mL) and water (100 mL) and the aqueous layer was back-extracted with Et₂O (2 x 100 mL). The organic layers were washed with a saturated NaCl solution (100 mL), dried (MgSO₄), and concentrated under reduced pressure. The solid residue was triturated with Et₂O to afford the desired product as a tan solid (3.8 g, 54%): TLC (30% EtOAc/70% hexane) R_{*f*} 0.06; ¹H-NMR (DMSO-d₆) δ 7.33 (dd, *J*=1.2, 4.2 Hz, 2H), 7.78 (d, *J*=8.7 Hz, 1H), 8.46 (dd, *J*=2.4, 8.7Hz, 1H), 8.54-8.56 (m, 3H).

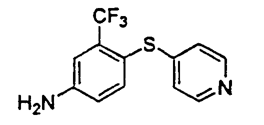

**Step 2. 3-(Trifluoromethyl)-4-(4-pyridinylthio)aniline:** A slurry of 3-trifluoromethyl-4-(4-pyridinylthio)nitrobenzene (3.8 g, 12.7 mmol), iron powder (4.0 g, 71.6 mmol), acetic acid (100 mL), and water (1 mL) were stirred at room temp. for 4 h. The mixture was diluted with Et₂O (100 mL) and water (100 mL). The aqueous phase was adjusted to pH 4 with a 4 N NaOH solution. The combined organic layers were washed with a saturated NaCl solution (100 mL), dried (MgSO₄), and concentrated under reduced pressure. The residue was filtered through a pad of silica (gradient from 50% EtOAc/50% hexane to 60% EtOAc/40% hexane) to afford the desired product (3.3 g): TLC (50% EtOAc/50% hexane) R_{*f*} 0.10; ¹H-NMR (DMSO-d₆) δ 6.21 (s, 2H), 6.84-6.87 (m, 3H), 7.10 (d, *J*=2.4 Hz, 1H), 7.39 (d, *J*=8.4 Hz, 1H), 8.29 (d, *J*=6.3 Hz, 2H).

### A13c. General Method for Substituted Aniline Formation via Nitroarene Formation Through Nucleophilic Aromatic Substitution, Followed by Reduction

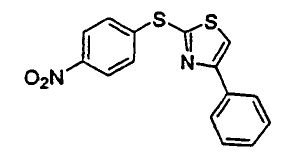

**Step 1. 4-(2-(4-Phenyl)thiazolyl)thio-1-nitrobenzene:** A solution of 2-mercapto-4-phenylthiazole (4.0 g, 20.7 mmoles) in DMF (40 mL) was treated with 1-fluoro-4-nitrobenzene (2.3 mL, 21.7 mmoles) followed by K₂CO₃ (3.18 g, 23 mmol), and the mixture was heated at approximately 65 °C overnight. The reaction mixture was then diluted with EtOAc (100 mL), sequentially washed with water (100 mL) and a saturated NaCl solution (100 mL), dried (MgSO₄) and concentrated under reduced pressure. The solid residue was triturated with a Et₂O/hexane solution to afford the desired product (6.1 g): TLC (25% EtOAc/75% hexane) R_{*f*} 0.49; ¹H-NMR (CDCl₃) δ 7.35-7.47 (m, 3H), 7.58-7.63 (m, 3H), 7.90 (d, *J*=6.9 Hz, 2H), 8.19 (d, *J*=9.0 Hz, 2H).

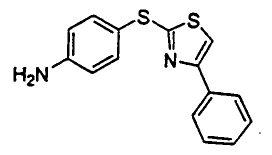

**Step 2. 4-(2-(4-Phenyl)thiazolyl)thioaniline:** 4-(2-(4-Phenyl)thiazolyl)thio-1-nitrobenzene was reduced in a manner analagous to that used in the preparation of 3-(trifluoromethyl)-4-(4-pyridinylthio)aniline: TLC (25% EtOAc/75% hexane) R_{*f*} 0.18; ¹H-NMR (CDCl₃) δ 3.89 (br s, 2H), 6.72-6.77 (m, 2H), 7.26-7.53 (m, 6H), 7.85-7.89 (m, 2H).

### A13d. General Method for Substituted Aniline Formation via Nitroarene Formation Through Nucleophilic Aromatic Substitution, Followed by Reduction

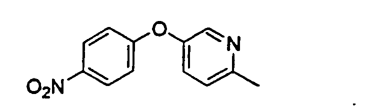

**Step 1. 4-(6-Methyl-3-pyridinyloxy)-1-nitrobenzene:** To a solution of 5-hydroxy-2-methylpyridine (5.0 g, 45.8 mmol) and 1-fluoro-4-nitrobenzene (6.5 g, 45.8 mmol) in anh DMF (50 mL) was added K₂CO₃ (13.0 g, 91.6 mmol) in one portion. The mixture was heated at the reflux temp. with stirring for 18 h and then allowed to cool to room temp. The resulting mixture was poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* to afford the desired product (8.7 g, 83%). The this material was carried to the next step without further purification.

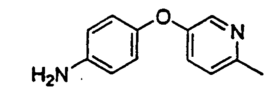

**Step 2. 4-(6-Methyl-3-pyridinyloxy)aniline:** A solution of 4-(6-methyl-3-pyridinyloxy)-1-nitrobenzene (4.0 g, 17.3 mmol) in EtOAc (150 mL) was added to 10% Pd/C (0.500 g, 0.47 mmol) and the resulting mixture was placed under a H₂ atmosphere (balloon) and was allowed to stir for 18 h at room temp. The mixture was then filtered through a pad of Celite® and concentrated *in vacuo* to afford the desired product as a tan solid (3.2 g, 92%): EI-MS *m*/*z* 200 (M⁺).

### A13e. General Method for Substituted Aniline Formation via Nitroarene Formation Through Nucleophilic Aromatic Substitution, Followed by Reduction

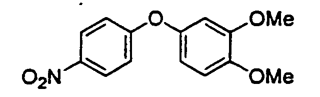

**Step 1. 4-(3,4-Dimethoxyphenoxy)-1-nitrobenzene:** To a solution of 3,4-dimethoxyphenol (1.0 g, 6.4 mmol) and 1-fluoro-4-nitrobenzene (700 µL, 6.4 mmol) in anh DMF (20 mL) was added K₂CO₃ (1.8 g, 12.9 mmol) in one portion. The mixture was heated at the reflux temp with stirring for 18 h and then allowed to cool to room temp. The mixture was then poured into water (100 mL) and extracted with EtOAc (3 x 100 mL). The combined organics were sequentially washed with water (3 x 50 mL) and a saturated NaCl solution (2 x 50 mL), dried (Na₂SO₄), and concentrated *in vacuo* to afford the desired product (0.8 g, 54%). The crude product was carried to the next step without further purification.

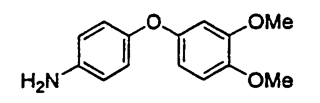

**Step 2. 4-(3,4-Dimethoxyphenoxy)aniline:** A solution of 4-(3,4-dimethoxyphenoxy)-1-nitrobenzene (0.8 g, 3.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite® and concentrated *in vacuo* to afford the desired product as a white solid (0.6 g, 75%): EI-MS *m*/*z* 245 (M⁺).

### A13f. General Method for Substituted Aniline Formation via Nitroarene Formation Through Nucleophilic Aromatic Substitution, Followed by Reduction

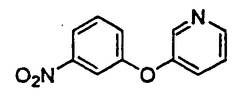

**Step 1. 3-(3-Pyridinyloxy)-1-nitrobenzene:** To a solution of 3-hydroxypyridine (2.8 g, 29.0 mmol), 1-bromo-3-nitrobenzene (5.9 g, 29.0 mmol) and copper(I) bromide (5.0 g, 34.8 mmol) in anh DMF (50 mL) was added K₂CO₃ (8.0 g, 58.1 mmol) in one portion. The resulting mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo*. The resulting oil was purified by flash chromatography (30% EtOAc/70% hexane) to afford the desired product (2.0 g, 32 %). This material was used in the next step without further purification.

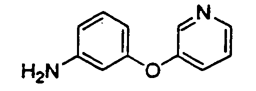

**Step 2. 3-(3-Pyridinyloxy)aniline:** A solution of 3-(3-pyridinyloxy)-1-nitrobenzene (2.0 g, 9.2 mmol) in EtOAc (100 mL) was added to 10% Pd/C (0.200 g) and the resulting mixture was placed under a H₂ atmosphere (balloon) and was allowed to stir for 18 h at room temp. The mixture was then filtered through a pad of Celite® and concentrated *in vacuo* to afford the desired product as a red oil (1.6 g, 94%): EI-MS *m*/*z* 186 (M⁺).

### A13g. General Method for Substituted Aniline Formation via Nitroarene Formation Through Nucleophilic Aromatic Substitution, Followed by Reduction

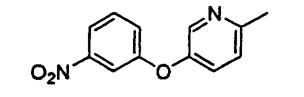

**Step 1. 3-(5-Methyl-3-pyridinyloxy)-1-nitrobenzene:** To a solution of 3-hydroxy-5-methylpyridine (5.0 g, 45.8 mmol), 1-bromo-3-nitrobenzene (12.0 g, 59.6 mmol) and copper(I) iodide (10.0 g, 73.3 mmol) in anh DMF (50 mL) was added K₂CO₃ (13.0 g, 91.6 mmol) in one portion. The mixture was heated at the reflux temp. with stirring for 18 h and then allowed to cool to room temp. The mixture was then poured into water (200 mL) and extracted with EtOAc (3 x 150 mL). The combined organics were sequentially washed with water (3 x 100 mL) and a saturated NaCl solution (2 x 100 mL), dried (Na₂SO₄), and concentrated *in vacuo* . The resulting oil was purified by flash chromatography (30% EtOAc/70% hexane) to afford the desired product (1.2 g, 13%).

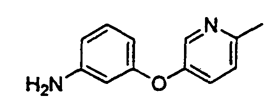

**Step 2. 3-(5-Methyl-3-pyridinyloxy)-1-nitrobenzene:** A solution of 3-(5-methyl-3-pyridinyloxy)-1-nitrobenzene (1.2 g, 5.2 mmol) in EtOAc (50 mL) was added to 10% Pd/C (0.100 g) and the resulting mixture was placed under a H₂ atmosphere (balloon) and was allowed to stir for 18 h at room temp. The mixture was then filtered through a pad of Celite® and concentrated *in vacuo* to afford the desired product as a red oil (0.9 g, 86%): CI-MS *m*/*z* 201 ((M+H)⁺).

### A13h. General Method for Substituted Aniline Formation via Nitroarene Formation Through Nucleophilic Aromatic Substitution, Followed by Reduction

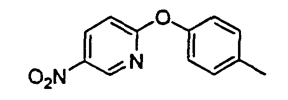

**Step 1. 5-Nitro-2-(4-methylphenoxy)pyridine:** To a solution of 2-chloro-5-nitropyridine (6.34 g, 40 mmol) in DMF (200 mL) were added of 4-methylphenol (5.4 g, 50 mmol, 1.25 equiv) and K₂CO₃ (8.28 g, 60 mmol, 1.5 equiv). The mixture was stirred overnight at room temp. The resulting mixture was treated with water (600 mL) to generate a precipitate. This mixture was stirred for 1 h, and the solids were separated and sequentially washed with a 1 N NaOH solution (25 mL), water (25 mL) and pet ether (25 mL) to give the desired product (7.05 g, 76%): mp 80-82 °C; TLC (30% EtOAc/70% pet ether) R_{*f*} 0.79; ¹H-NMR (DMSO-d₆) δ 2.31 (s, 3H), 7.08 (d, *J*=8.46 Hz, 2H), 7.19 (d, *J*=9.20 Hz, 1H), 7.24 (d, *J*=8.09 Hz, 2H), 8.58 (dd, *J*=2.94, 8.82 Hz, 1H), 8.99 (d, *J*=2.95 Hz, 1H); FAB-MS *m*/*z* (rel abundance) 231 ((M+H)⁺), 100%).

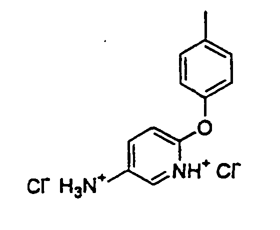

**Step 2. 5-Amino-2-(4-methylphenoxy)pyridine Dihydrochloride**: A solution 5-nitro-2-(4-methylphenoxy)pyridine (6.94 g, 30 mmol, 1 eq) and EtOH (10 mL) in EtOAc (190 mL) was purged with argon then treated with 10% Pd/C (0.60 g). The reaction mixture was then placed under a H₂ atmosphere and was vigorously stirred for 2.5 h. The reaction mixture was filtered through a pad of Celite®. A solution of HCl in Et₂O was added to the filtrate was added dropwise. The resulting precipitate was separated and washed with EtOAc to give the desired product (7.56 g, 92%): mp 208-210 °C (dec); TLC (50% EtOAc/50% pet ether) R_{*f*} 0.42; ¹H-NMR (DMSO-d₆) δ 2.25 (s, 3H), 6.98 (d, *J*=8.45 Hz, 2H), 7.04 (d, *J*=8.82 Hz, 1H), 7.19 (d, *J*=8.09 Hz, 2H), 8.46 (dd, *J*=2.57, 8.46 Hz, 1H), 8.63 (d, *J*=2.57 Hz, 1H); EI-MS *m*/*z* (rel abundance) (M⁺, 100%).

### A13i. General Method for Substituted Aniline Formation via Nitroarene Formation Through Nucleophilic Aromatic Substitution, Followed by Reduction

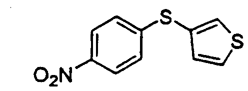

**Step 1. 4-(3-Thienylthio)-1-nitrobenzene:** To a solution of 4-nitrothiophenol (80%pure; 1.2 g, 6.1 mmol), 3-bromothiophene (1.0 g, 6.1 mmol) and copper(II) oxide (0.5 g, 3.7 mmol) in anhydrous DMF (20 mL) was added KOH (0.3 g, 6.1 mmol), and the resulting mixture was heated at 130 °C with stirring for 42 h and then allowed to cool to room temp. The reaction mixture was then poured into a mixture of ice and a 6N HCl solution (200 mL) and the resulting aqueous mixture was extracted with EtOAc (3 x 100 mL). The combined organic layers were sequentially washed with a 1M NaOH solution (2 x 100 mL) and a saturated NaCl solution (2 x 100 mL), dried (MgSO₄), and concentrated *in vacuo*. The residual oil was purified by MPLC (silica gel; gradient from 10% EtOAc/90% hexane to 5% EtOAc/95% hexane) to afford of the desired product (0.5 g, 34%). GC-MS *m*/*z* 237 (M⁺).

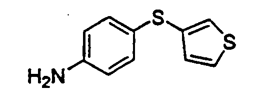

**Step 2. 4-(3-Thienylthio)aniline:** 4-(3-Thienylthio)-1-nitrobenzene was reduced to the aniline in a manner analogous to that described in Method B1.

### A13j. General Method for Substituted Aniline Formation via Nitroarene Formation Through Nucleophilic Aromatic Substitution, Followed by Reduction

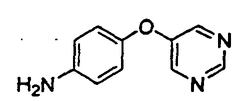

**4-(5-Pyrimininyloxy)aniline:** 4-Aminophenol (1.0 g, 9.2 mmol) was dissolved in DMF (20 mL) then 5-bromopyrimidine (1.46 g, 9.2 mmol) and K₂CO₃ (1.9 g, 13.7 mmol) were added. The mixture was heated to 100 °C for 18 h and at 130 °C for 48 h at which GC-MS analysis indicated some remaining starting material. The reaction mixture was cooled to room temp. and diluted with water (50 mL). The resulting solution was extracted with EtOAc (100 mL). The organic layer was washed with a saturated NaCl solution (2 x 50 mL), dried (MgSO₄), and concentrated *in vacuo*. The residular solids were purified by MPLC (50% EtOAc/50% hexanes) to give the desired amine (0.650 g, 38%).

### 1A13k. General Method for Substituted Aniline Formation via Nitroarene Formation Through Nucleophilic Aromatic Substitution, Followed by Reduction

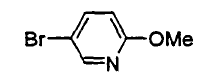

**Step 1. 5-Bromo-2-methoxypyridine:** A mixture of 2,5-dibromopyridine (5.5 g, 23.2 mmol) and NaOMe (3.76g, 69.6 mmol) in MeOH (60 mL) was heated at 70 °C in a sealed reaction vessel for 42 h, then allowed to cool to room temp. The reaction mixture was treated with water (50 mL) and extracted with EtOAc (2 x 100 mL). The combined organic layers were dried (Na₂SO₄) and concentrated under reduced pressure to give a pale yellow, volatile oil (4.1g, 95% yield): TLC (10% EtOAc / 90% hexane) R_{*f*} 0.57.

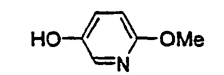

**Step 2. 5-Hydroxy-2-methoxypyridine:** To a stirred solution of 5-bromo-2-methoxypyridine (8.9 g, 47.9 mmol) in THF (175 mL) at -78 °C was added an n-butyllithium solution (2.5 M in hexane; 28.7 mL, 71.8 mmol) dropwise and the resulting mixture was allowed to stir at -78 °C for 45 min. Trimethyl borate (7.06 mL, 62.2 mmol) was added via syringe and the resulting mixture was stirred for an additional 2 h. The bright orange reaction mixture was warmed to 0 °C and was treated with a mixture of a 3 N NaOH solution (25 mL, 71.77 mmol) and a hydrogen peroxide solution (30%; approx. 50 mL). The resulting yellow and slightly turbid reaction mixture was warmed to room temp. for 30 min and then heated to the reflux temp. for 1 h. The reaction mixture was then allowed to cool to room temp. The aqueous layer was neutralized with a 1N HCl solution then extracted with Et₂O (2 x 100 mL). The combined organic layers were dried (Na₂SO₄) and concentrated under reduced pressure to give a viscous yellow oil (3.5g, 60%).

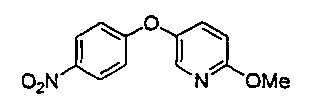

**Step 3. 4-(5-(2-Methoxy)pyridyl)oxy-1-nitrobenzene:** To a stirred slurry of NaH (97%, 1.0 g, 42 mmol) in anh DMF (100 mL) was added a solution of 5-hydroxy-2-methoxypyridine (3.5g, 28 mmol) in DMF (100 mL). The resulting mixture was allowed to stir at room temp. for 1 h, 4-fluoronitrobenzene (3 mL, 28 mmol) was added via syringe. The reaction mnixture was heated to 95 °C overnight, then treated with water (25 mL) and extracted with EtOAc (2 x 75 mL). The organic layer was dried (MgSO₄) and concentrated under reduced pressure. The residual brown oil was crystalized EtOAc/hexane) to afford yellow crystals (5.23 g, 75%).

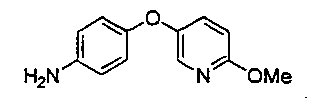

**Step 4. 4-(5-(2-Metboxy)pyridyl)oxyaniline:** 4-(5-(2-Methoxy)pyridyl)oxy-1-nitrobenzene was reduced to the aniline in a manner analogous to that described in Method B3d, Step2.

### A14a. General Method for Substituted Aniline Synthesis via Nucleophilic Aromatic Substitution using a Halopyridine

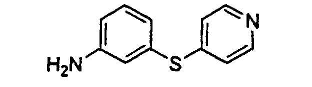

**3-(4-Pyridinylthio)aniline:** To a solution of 3-aminothiophenol (3.8 mL, 34 mmoles) in anh DMF (90mL) was added 4-chloropyridine hydrochloride (5.4 g, 35.6 mmoles) followed by K₂CO₃ (16.7 g, 121 mmoles). The reaction mixture was stirred at room temp. for 1.5 h, then diluted with EtOAc (100 mL) and water (100mL). The aqueous layer was back-extracted with EtOAc (2 x 100 mL). The combined organic layers were washed with a saturated NaCl solution (100 mL), dried (MgSO₄), and concentrated under reduced pressure. The residue was filtered through a pad of silica (gradient from 50% EtOAc/50% hexane to 70% EtOAc/30% hexane) and the resulting material was triturated with a Et₂O/hexane solution to afford the desired product (4.6 g, 66%): TLC (100 % ethyl acetate) R_{*f*} 0.29; ¹H-NMR (DMSO-d₆) δ 5.41 (s, 2H), 6.64-6.74 (m, 3H), 7.01 (d, J=4.8, 2H), 7.14 (t, J=7.8 Hz, 1H), 8.32 (d, J=4.8, 2H).

### 1A14b. General Method for Substituted Aniline Synthesis via Nucleophilic Aromatic Substitution using a Halopyridine

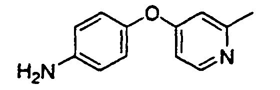

**4-(2-Methyl-4-pyridinyloxy)aniline:** To a solution of 4-aminophenol (3.6 g, 32.8 mmol) and 4-chloropicoline (5.0 g, 39.3 mmol) in anh DMPU (50 mL) was added potassium *tert*-butoxide (7.4 g, 65.6 mmol) in one portion. The reaction mixture was heated at 100 °C with stirring for 18 h, then was allowed to cool to room temp. The resulting mixture was poured into water (200 mL) and extracted with EtOAc (3 x 150 mL). The combined extracts were sequentially washed with water (3 x 100 mL) and a saturated NaCl solution (2 x 100 mL), dried (Na₂SO₄), and concentrated *in vacuo*. The resulting oil was purified by flash chromatography (50 % EtOAc/50% hexane) to afford the desired product as a yellow oil (0.7 g, 9%): CI-MS *m*/*z* 201 ((M+H)⁺).

### A14c. General Method for Substituted Aniline Synthesis via Nucleophilic Aromatic Substitution using a Halopyridine

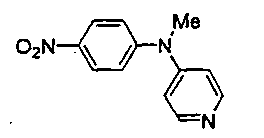

**Step 1. Methyl(4-nitrophenyl)-4-pyridylamine:** To a suspension of *N*-methyl-4-nitroaniline (2.0 g, 13.2 mmol) and K₂CO₃ (7.2 g, 52.2 mmol) in DMPU (30mL) was added 4-chloropyridine hydrochloride (2.36 g, 15.77 mmol). The reaction mixture was heated at 90 °C for 20 h, then cooled to room temperature. The resulting mixture was diluted with water (100 mL) and extracted with EtOAc (100 mL). The organic layer was washed with water (100 mL), dried (Na₂SO₄) and concentrated under reduced pressure. The residue was purified by column chromatography (silica gel, gradient from 80% EtOAc /20% hexanes to 100% EtOAc) to afford methyl(4-nitrophenyl)-4-pyridylamine (0.42 g)

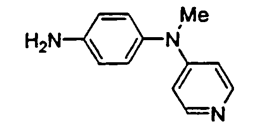

**Step 2. Methyl(4-aminophenyl)-4-pyridylamine:** Methyl(4-nitrophenyl)-4-pyridylamine was reduced in a manner analogous to that described in Method B1.

### A15. General Method of Substituted Aniline Synthesis via Phenol Alkylation Followed by Reduction of a Nitroarene

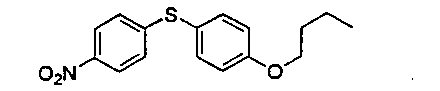

**Step 1. 4-(4-Butoxyphenyl)thio-1-nitrobenzene:** To a solution of 4-(4-nitrophenylthio)phenol (1.50 g, 6.07 mmol) in anh DMF (75 ml) at 0 °C was added NaH (60% in mineral oil, 0.267 g, 6.67 mmol). The brown suspension was stirred at 0 °C until gas evolution stopped (15 min), then a solution of iodobutane (1.12 g, .690 ml, 6.07 mmol) in anh DMF (20 mL) was added dropwise over 15 min at 0 °C. The reaction was stirred at room temp. for 18 h at which time TLC indicated the presence of unreacted phenol, and additional iodobutane (56 mg, 0.035 mL, 0.303 mmol, 0.05 equiv) and NaH (13 mg, 0.334 mmol) were added. The reaction was stirred an additional 6 h room temp., then was quenched by the addition of water (400 mL). The resulting mixture was extracted with Et₂O (2 x 500 mL). The combibed organics were washed with water (2 x 400 mL), dried (MgSO₄), and concentrated under reduced pressure to give a clear yellow oil, which was purified by silica gel chromatography (gradient from 20% EtOAc/80% hexane to 50% EtOAc/50% hexane) to give the product as a yellow solid (1.24 g, 67%): TLC (20% EtOAc/80% hexane) R_{*f*} 0.75; ¹H-NMR (DMSO-d₆) δ 0.92 (t, *J*= 7.5 Hz, 3H), 1.42 (app hex, *J*=7.5 Hz, 2H), 1.70 (m, 2H), 4.01 (t, *J=* 6.6 Hz, 2H), 7.08 (d, *J*=8.7 Hz, 2H), 7.17 (d, *J*=9 Hz, 2H), 7.51 (d, *J=* 8.7 Hz, 2H), 8.09 (d, *J=* 9 Hz, 2H).

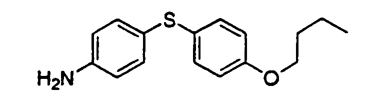

**Step 2. 4-(4-Butoxyphenyl)thioaniline:** 4-(4-Butoxyphenyl)thio-1-nitrobenzene was reduced to the aniline in a manner analagous to that used in the preparation of 3-(trifluoromethyl)-4-(4-pyridinylthio)aniline (Method B3b, Step 2): TLC (33% EtOAc/77% hexane) R_{*f*} 0.38.

### A16. General Method for Synthesis of Substituted Anilines by the Acylation of Diaminoarenes

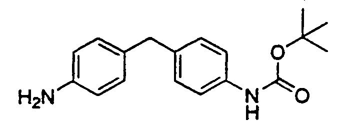

**4-(4-*tert*-Butoxycarbamoylbenzyl)aniline**: To a solution of 4,4'-methylenedianiline (3.00 g, 15.1 mmol) in anh THF (50 mL) at room temp was added a solution of di-*tert*-butyl dicarbonate (3.30 g, 15.1 mmol) in anh THF (10 mL). The reaction mixture was heated at the reflux temp. for 3 h, at which time TLC indicated the presence of unreacted methylenedianiline. Additional di-*tert*-butyl dicarbonate (0.664 g, 3.03 mmol, 0.02 equiv) was added and the reaction stirred at the reflux temp. for 16 h. The resulting mixture was diluted with Et₂O (200 mL), sequentially washed with a saturated NaHCO₃ solution (100 ml), water (100 mL) and a saturated NaCl solution (50 mL), dried (MgSO₄), and concentrated under reduced pressure. The resulting white solid was purified by silica gel chromatography (gradient from 33% EtOAc/67% hexane to 50% EtOAc/50% hexane) to afford the desired product as a white solid ( 2.09 g, 46%): TLC (50% EtOAc/50% hexane) R_{*f*} 0.45; ¹H-NMR (DMSO-d₆) δ 1.43 (s, 9H), 3.63 (s, 2H), 4.85 (br s, 2H), 6.44 (d, *J*=8.4 Hz, 2H), 6.80 (d, *J*=8.1 Hz, 2H), 7.00 (d, *J*=8.4 Hz, 2H), 7.28 (d, *J*=8.1 Hz, 2H), 9.18 (br s, 1H); FAB-MS *m*/*z* 298 (M⁺).

### A17. General Method for the Synthesis of Aryl Amines via Electrophilic Nitration Followed by Reduction

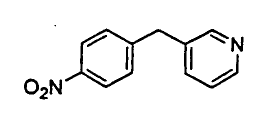

**Step 1. 3-(4-Nitrobenzyl)pyridine**: A solution of 3-benzylpyridine (4.0 g, 23.6 mmol) and 70% nitric acid (30 mL) was heated overnight at 50 °C. The resulting mixture was allowed to cool to room temp. then poured into ice water (350 mL). The aqueous mixture then made basic with a 1N NaOH solution, then extracted with Et₂O (4 x 100 mL). The combined extracts were sequentially washed with water (3 x 100 mL) and a saturated NaCl solution (2 x 100 mL), dried (Na₂SO₄), and concentrated *in vacuo*. The residual oil was purified by MPLC (silica gel; 50 % EtOAc/50% hexane) then recrystallization (EtOAc/hexane) to afford the desired product (1.0 g, 22%): GC-MS *m*/*z* 214 (M⁺).

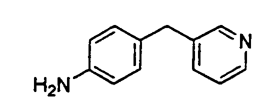

**Step 2. 3-(4-Pyridinyl)methylaniline**: 3-(4-Nitrobenzyl)pyridine was reduced to the aniline in a manner analogous to that described in Method B1.

### A18. General Method for Synthesis of Aryl Amines via Substitution with Nitrobenzyl Halides Followed by Reduction

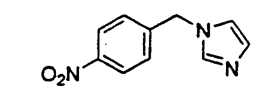

**Step 1. 4-(1-Imidazolylmethyl)-1-nitrobenzene:** To a solution of imidazole (0.5 g, 7.3 mmol) and 4-nitrobenzyl bromide (1.6 g, 7.3 mmol) in anh acetonitrile (30 mL) was added K₂CO₃ (1.0 g, 7.3 mmol). The resulting mixture was stirred at rooom temp. for 18 h and then poured into water (200 mL) and the resulting aqueous solution wasextracted with EtOAc (3 x 50 mL). The combined organic layers were sequentially washed with water (3 x 50 mL) and a saturated NaCl solution (2 x 50 mL), dried (MgSO₄), and concentrated *in vacuo*. The residual oil was purified by MPLC (silica gel; 25% EtOAc/75% hexane) to afford the desired product (1.0 g, 91 %): EI-MS *m*/*z* 203 (M⁺).

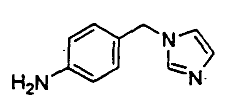

**Step 2. 4-(1-Imidazolylmethyl)aniline:** 4-(1-Imidazolylmethyl)-1-nitrobenzene was reduced to the aniline in a manner analogous to that described in Method B2.

### A19. Formation of Substituted Hydroxymethylanilines by Oxidation of Nitrobenzyl Compounds Followed by Reduction

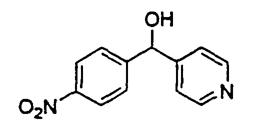

**Step 1. 4-(1-Hydroxy-1-(4-pyridyl)methyl-1-nitrobenzene:** To a stirred solution of 3-(4-nitrobenzyl)pyridine (6.0 g, 28 mmol) in CH₂Cl₂ (90 mL) was added *m*-CPBA (5.80 g, 33.6 mmol) at 10 °C, and the mixture was stirred at room temp. overnight. The reaction mixture was successively washed with a 10% NaHSO₃ solution (50 mL), a saturated K₂CO₃ solution (50 mL) and a saturated NaCl solution (50 mL), dried (MgSO₄) and concentrated under reduced pressure. The resulting yellow solid (2.68 g) was dissolved in anh acetic anhydride (30 mL) and heated at the reflux temperature overnight. The mixture was concentrated under reduced pressure. The residue was dissolved in MeOH (25 mL) and treated with a 20% aqueous NH₃ solution (30 mL). The mixture was stirred at room temp. for 1 h, then was concentrated under reduced pressure. The residue was poured into a mixture of water (50 mL) and CH₂Cl₂ (50 mL). The organic layer was dried (MgSO₄), concentrated under reduced pressure, and purified by column chromatography (80% EtOAc/ 20% hexane) to afford the desired product as a white solid. (0.53 g, 8%): mp 110-118 °C; TLC (80% EtOAc/20% hexane) R_{*f*} 0.12; FAB-MS *m*/*z* 367 ((M+H)⁺, 100%).

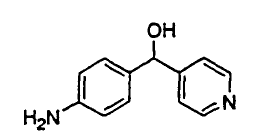

**Step 2. 4-(1-Hydroxy-1-(4-pyridyl)methylaniline:** 4-(1-Hydroxy-1-(4-pyridyl)-methyl-1-nitrobenzene was reduced to the aniline in a manner analogous to that described in Method B3d, Step2.

### A20. Formation of 2-(N-methylcarbamoyl)pyridines via the Menisci reaction

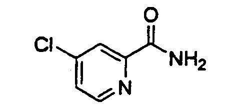

**Step 1. 2-(*N*-methylcarbamoyl)-4-chloropyridine. (Caution:** this is a highly hazardous, potentially explosive reaction.) To a solution of 4-chloropyridine (10.0 g) in *N*-methylformamide (250 mL) under argon at ambient temp was added conc. H₂SO₄ (3.55 mL) (exotherm). To this was added H₂O₂ (17 mL, 30% wt in H2O) followed by FeSO₄7H2O (0.55 g) to produce an exotherm. The reaction was stirred in the dark at ambient temp for 1h then was heated slowly over 4 h at 45 °C. When bubbling subsided,the reaction was heated at 60 °C for 16 h. The opaque brown solution was diluted with H2O (700 mL) fol.lowed by a 10% NaOH solution (250 mL). The aqueous mixture was extracted with EtOAc (3 x 500 mL) and the organic layers were washed separately with a saturated NaCl solution (3 x 150 mlL. The combined organics were dried (MgSO₄) and filtered through a pad of silica gel eluting with EtOAc. The solvent was removed in vacuo and the brown residue was purified by silica gel chromatography (gradient from 50% EtOAc / 50% hexane to 80% EtOAc /20% hexane). The resulting yellow oil crystallized at 0 °C over 72 h to give 2-(*N*-methylcarbamoyl)-4-chloropyridine in yield (0.61 g, 5.3%): TLC (50% EtOAc/50% hexane) R_{*f*} 0.50; MS; ¹H NMR (CDCl₃): d 8.44 (d, 1 H, J = 5.1 Hz, CHN), 8.21 (s, 1H, CHCCO), 7.96 (b s, 1H, NH), 7.43 (dd, 1H, J = 2.4, 5.4 Hz, ClCHCN), 3.04 (d, 3H, J = 5.1 Hz, methyl); CI-MS *m*/*z* 171 ((M+H)+).

### A21. Generalmethod for the Synthesis of ω-Sulfonylphenyl Anilines

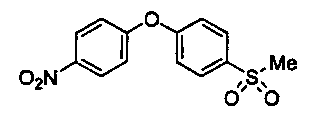

**Step 1. 4-(4-Methylsulfonylphenoxy)-1-nitrobenzene:** To a solution of 4-(4-methylthiophenoxy)-1-ntirobenzene (2 g, 7.66 mmol) in CH₂Cl₂ (75 mL) at 0 °C was slowly added *m*CPBA (57-86%, 4 g), and the reaction mixture was stirred at room temperature for 5 h. The reaction mixture was treated with a 1 N NaOH solution (25 mL). The organic layer was sequentially washed with a 1N NaOH solution (25 mL), water (25 mL) and a saturated NaCl solution (25 mL), dried (MgSO₄), and concentrated under reduced pressure to give 4-(4-methylsulfonylphenoxy)-1-nitrobenzene as a solid (2.1 g).

**Step 2. 4-(4-Methylsulfonylphenoxy)-1-aniline:** 4-(4-Methylsulfonylphenoxy)-1-nitrobenzene was reduced to the aniline in a manner anaologous to that described in Method B3d, step 2.

### A22. General Method for Synthesis of ω-Alkoxy-ω-carboxyphenyl Anilines

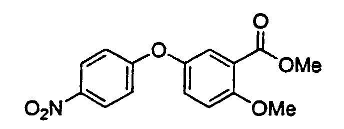

**Step 1. 4-(3-Methoxycarbonyl-4-methoxyphenoxy)-1-nitrobenzene:** To a solution of -(3-carboxy-4-hydroxyphenoxy)-1-nitrobenzene (prepared in a manner analogous to that described in Method B3a, step 1, 12 mmol) in acetone (50 mL) was added K₂CO₃ (5 g) and dimethyl sulfate (3.5 mL). The resulting mixture was heated aaaaaat the reflux tempoerature overnight, then cooled to room temperature and filtered through a pad of Celite®. The resulting solution was concentrrated under reduced pressure, absorbed onto silica gel, and purified by column chromatography (50% EtOAc / 50% hexane) to give 4-(3-methoxycarbonyl-4-methoxyphenoxy)-1-nitrobenzene as a yellow powder (3 g): mp 115 118 °C.

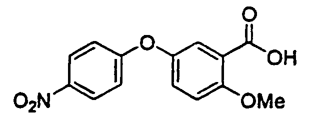

**Step 2. 4-(3-Carboxy-4-methoxyphenoxy)-1-nitrobenzene:** A mixture of 4-(3-methoxycarbonyl-4-methoxyphenoxy)-1-nitrobenzene (1.2 g), KOH (0.33 g),and water (5 mL) in MeOH (45 mL) was stirred at room temperature overnight and then heated at the reflux temperature for 4 h. The resulting mixture was cooled to room temperature and concentrated under reduced pressure. The residue was dissolved in water (50 mL), and the aqueous mixture was made acidic with a 1N HCl solution. The resulting mixture was extracted with EtOAc (50 mL). The organic layer was dried (MgSO₄) and concentrated under reduced pressure to give 4-(3-carboxy-4-methoxyphenoxy)-1-nitrobenzene (1.04 g).

### B. General Methods of Urea Formation

### B1a. General Method for the Reaction of an Aryl Amine with an Aryl Isocyanate

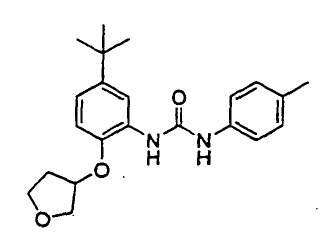

***N-*(5*-tert*-Butyl-2-(3-tetrahydrofuranyloxy)phenyl)-*N'*-(4-methylphenyl)urea:** To a solution of 5-*tert*-butyl-2-(3-tetrahydrofuranyloxy)aniline (0.078 g, 0.33 mmol) in toluene (2.0 mL) was added *p*-tolyl isocyanate (0.048 g, 0.36 mmol) and the resulting mixture was allowed to stir at room temp. for 8 h to produce a precipitate. The reaction mixture was filtered and the residue was sequentially washed with toluene and hexanes to give the desired urea as a white solid (0.091 g, 75%): mp 229-231 °C; ¹H-NMR (DMSO-d₆) δ 1.30 (s, 9H), 1.99-2.03 (m, 1H), 2.19-2.23 (m, 4H), 3.69-3.76 (m, 1H), 3.86-3.93 (m, 3H), 4.98-5.01 (m, 1H), 6.81-6.90 (m, 2H), 7.06 (d, *J*=8.09 Hz, 2H, 7.32 (d, *J*=8.09 Hz, 2H), 7.84 (s, 1H), 8.22 (d, *J*=2.21 Hz, 1H), 9.26 (s, 1H).

### B1b. General Method for the Reaction of an Aryl Amine with an Aryl Isocyanate

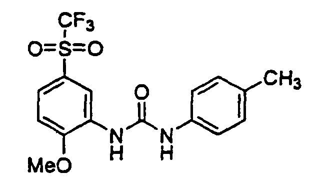

***N*-(2-Methoxy-5-(trifluoromethanesulfonyl)phenyl)-*N'*(4-methylphenyl)urea**: *p*-Tolyl isocyanate (0.19 mL, 1.55 mmol) was added to a solution of 2-methoxy-5-(trifluoromethanesulfonyl)aniline (0.330 g, 1.29 mmol) in EtOAc (5 mL), and the reaction mixture was stirred at room temp. for 18 h. The resulting precipitate was collected by filtration and washed with Et₂O to give a white solid (0.28 g). This material was then purified by HPLC (C-18 column, 50% CH₃CN/50% H₂O) and the resulting solids were triturated with Et₂O to provide the title compound (0.198 g): ¹H-NMR (CDCl₃) δ 7.08 (d, *J*=8.5 Hz, 2H), 7.33 (d, *J*=8.5 Hz, 2H), 7.40 (d, *J*=8.8 Hz, 1H), 7.71 (dd, *J*=2.6, 8.8 Hz, 1H), 8.66 (s, 1H), 8.90 (d, *J*=2.6 Hz, 1H), 9.36 (s, 1H); FAB-MS *m*/*z* 389 ((M+1)⁺).

### B1c. General Method for the Reaction of an Aryl Amine with an Aryl Isocyanate

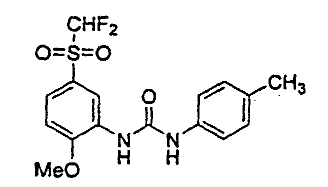

***N*-(2-Metboxy-5-(difluoromethanesulfonyl)phenyl)-*N*'-(4-methylphenyl)urea:** *p*-Tolyl isocyanate (0.058 mL, 0.46 mmol) was added to a solution of 2-methoxy-5-(difluoromethanesulfonyl)aniline (0.100 g, 0.42 mmol) in EtOAc (0.5 mL) and the resulting mixture was stirred at room temp. for 3 d. The resulting precipitate was filtered and washed with Et₂O to provide the title compound as a white solid (0.092 g): ¹H-NMR (CDCl₃) δ 2.22 (s, 3H) 4.01 (s, 3H), 7.02-7.36 (m, 6H), 7.54 (dd, *J*=2.4, 8.6 Hz, 1H), 8.57 (s, 1H), 8.79 (d, *J*=2.6 Hz, 1H), 9.33 (s, 1H); EI-MS *m*/*z* 370 (M⁺).

### B1d. General Method for the Reaction of an Aryl Amine with an Aryl Isocyanate

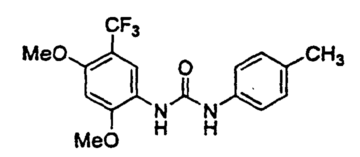

***N*-(2,4-Dimethoxy-5-(trifluoromethyl)phenyl)-*N'*-(4-methylphenyl)urea:** *p*-Tolyl isocyanate (0.16 mL, 1.24 mmol) was added to a solution of 2,4-dimethoxy-5-(trifluoromethyl)aniline (0.25 g, 1.13 mmol) in EtOAc (3 mL) and the resulting mixture was stirred at room temp. for 18 h. A resulting precipitate was washed with Et₂O to give the title compound as a white solid (0.36 g): ¹H-NMR (CDCl₃) δ 2.21 (s, 3H). 3.97 (s, 3H), 3.86 (s, 3H), 6.88 (s, 1H), 7.05 (d, *J*=8.5 Hz, 2H), 7.29 (d, *J*=8.5 Hz, 2H), 8.13 (s, 1H), 8.33 (s, 1H), 9.09 (s, 1H); FAB-MS *m*/*z* 355 ((M+1)⁺),

### B1e. General Method for the Reaction of an Aryl Amine with an Aryl Isocyanate

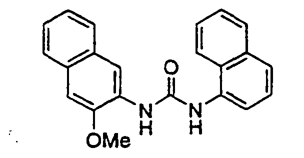

***N*-(3-Methoxy-2-naphthyl)-*N'*-(1-naphthyl)urea:** To a solution of 2-amino-3-methoxynaphthalene (0.253 g, 1.50 mmol) in CH₂Cl₂ (3 mL) at room temp. was added a solution of 1-naphthyl isocyanate (0.247 g, 1.50 mmol) in CH₂Cl₂ (2 mL) and the resulting mixture was allowed to stir overnight. The resulting precipitate was separated and washed with CH₂Cl₂ to give the desired urea as a white powder (0.450 g, 90%): mp 235-236 °C; ¹H-NMR (DMSO-d₆) δ 4.04 (s, 3H), 7.28-7.32 (m, 2H), 7.38 (s, 1H), 7.44-7.72 (m, 6H), 7.90-7.93 (m, 1H), 8.05-8.08 (m, 1H), 8.21-8.24 (m, 1H), 8.64 (s, 1H), 9.03 (s, 1H), 9.44 (s, 1H); FAB-MS *m*/*z* 343 ((M+H)⁺).

### B1f. General Method for the Reaction of an Aryl Amine with an Aryl Isocyanate

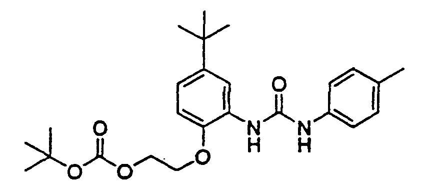

***N*-(5-*tert*-Butyl-2-(2-*tert*-butoxycarbonyloxy)ethoxy)phenyl)-*N'*-(4-methylphenyl)urea**: A mixture of 5-*tert*-butyl-2-(2-*tert*-butoxycarbonyloxy)ethoxy)aniline (Method A10, 0.232 g, 0.75 mmol) and *p*-tolyl isocyanate (0.099 mL, 0.79 mmol) in EtOAc (1 mL) was stirred at room temp. for 3 d to produce a solid, which was separated. The filtrate was purified by column chromatography (100% CH₂Cl₂) and the residue was triturated (Et₂O/hexane) to give the desired product (0.262 g, 79%): mp 155-156 °C; TLC (20% EtOAc/80% hexane) R_{*f*} 0.49; ¹H-NMR (DMSO-d₆) δ 1.22 (s, 9H), 1.37 (s, 9H), 2.21 (s, 3H), 4.22-4.23 (m, 2H), 4.33-4.35 (m, 2H), 6.89-7.00 (m, 4H), 7.06 (d, *J*=8.5 Hz, 2H), 7.32 (d, *J*=8.1 Hz, 2H), 7.96 (s, 1H); 8.22 (d, *J*=1.5 Hz, 1H), 9.22 (s, 1H); FAB-MS *m*/*z* (rel abundance) 443 ((M+H)⁺, 6%).

### B2a. General Method for Reaction of an Aryl Amine with Phosgene Followed by Addition of a Second Aryl Amine

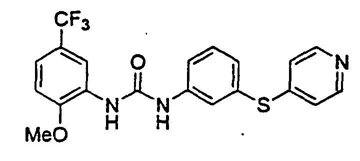

***N*-(2-Methoxy-5-(trifluoromethyl)phenyl)-*N'*-(3-(4-pyridinylthio)phenyl)urea:** To a solution of pyridine (0.61 mL, 7.5 mmol, 3.0 equiv) and phosgene (20% in toluene; 2.65 mL, 5.0 mmol, 2.0 equiv) in CH₂Cl₂ (20 mL) was added 2-methoxy-5-(trifluoromethyl)aniline (0.48 g, 2.5 mmol) at 0 °C. The resulting mixture was allowed warm to room temp. stirred for 3 h, then treated with anh. toluene (100 mL) and concentrated under reduced pressure. The residue was suspended in a mixture of CH₂Cl₂ (10 mL) and anh. pyridine (10 mL) and treated with 3-(4-pyridinylthio)aniline (0.61 g, 2.5 mmol, 1.0 equiv). The mixture was stirred overnight at room temp., then poured into water (50 mL) and extracted with CH₂Cl₂ (3 x 25 mL). The combined organic layers were dried (MgSO₄) and concentrated under reduced pressure. The residue was dissolved in a minimal amount of CH₂Cl₂ and treated with pet. ether to give the desired product as a white precipitate (0.74 g, 70%): mp 202 °C; TLC (5% acetone/95% CH₂Cl₂) R_{*f*} 0.09; ¹H-NMR (DMSO-d₆) δ 7.06 (d, *J*=5.5 Hz, 2H), 7.18 (dd, *J*=2.4, 4.6 Hz, 2H), 7.31 (dd, *J*= 2.2, 9.2 Hz, 1H), 7.44 (d, *J*=5.7 Hz, 1H), 7.45 (s, 1H), 7.79 (d, *J*=2.2 Hz, 1H), 8.37 (s, 2H), 8.50 (dd, *J*=2.2, 9.2 Hz, 2H), 9.63 (s, 1H), 9.84 (s, 1H); FAB-MS *m*/*z* 420 ((M+H)⁺, 70%).

### B2b. General Method for Reaction of an Aryl Amine with Phosgene Followed by Addition of a Second Aryl Amine

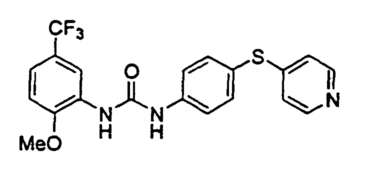

***N*-(2-Methoxy-5-(trifluoromethyl)phenyl)-*N*'-(4-(4-pyridinylthio)phenyl)urea:** To a solution of pyridine (0.61 mL, 7.5 mmol, 3.0 equiv) and phosgene (20% in toluene; 2.65 mL, 5.0 mmol, 2.0 equiv) in CH₂Cl₂ (20 mL) was added 4-(4-pyridinylthio)aniline (0.506 g, 2.5 mmol) at 0 °C. After stirring for 3 h at room temp., the mixture was treated with anh. toluene (100 mL) then concentrated under reduced pressure. The residue was suspended in a mixture of CH₂Cl₂ (10 mL) and anh. pyridine (10 mL) and treated with 2-methoxy-5-(trifluoromethyl)aniline (0.50 g, 2.5 mmol, 1.0 equiv). After stirring the mixture overnight at room temp., it was poured into a 1 N NaOH solution (50 mL) and extracted with CH₂Cl₂ (3 x 25 mL). The combined organic layers were dried (MgSO₄) and concentrated under reduced pressure to give the desired urea (0.74 g, 71%): mp 215 °C; TLC (5% acetone/95% CH₂Cl₂) R_{*f*} 0.08; ¹H-NMR (DMSO-d₆) δ 3.96 (s, 3H), 6.94 (dd, *J*=1.1, 4.8 Hz, 2H), 7.19 (d, *J*=8.4 Hz, 1H), 7.32 (dd, *J*=2.2, 9.3 Hz, 1H), 7.50 (d, *J*=8.8 Hz, 2H), 7.62 (d, *J*=8.8 Hz, 2H), 8.32 (d, *J*=5.1 Hz, 2H), 8.53 (d, *J*=0.7 Hz, 1H), 8.58 (s, 1H), 9.70 (s, 1 H); FAB-MS *m*/*z* 420 ((M+H)⁺).

### B3a. General Method for the Reaction of an Aryl Amine with Phosgene with Isolation of the Isocyanate, Followed by Reaction with a Second Aryl Amine

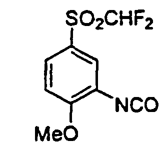

**Step 1. 5-(Difluoromethanesulfonyl)-2-methoxyphenyl isocyanate**: To a solution of phosgene (1.95 M in toluene; 3.0 mL, 5.9 mmol) in CH₂Cl₂ (40 mL) at 0 °C was added a solution of 5-(difluoromethanesulfonyl)-2-methoxyaniline (0.70 g, 2.95 mmol) and pyridine (0.44 mL, 8.85 mmol) in CH₂Cl₂ (10 mL) dropwise. After being stirred at 0 °C for 30 min and at room temp. for 3 h, the reaction mixture was concentrated under reduced pressure, then treated with toluene (50 mL). The resulting mixture was concentrated under reduced pressure, then was treated with Et₂O (50 mL) to produce a precipitate (pyridinium hydrochloride). The resulting filtrate was concentrated under reduced pressure to provide the title compound as a white solid (0.33 g). This material was used in the next step without further purification.

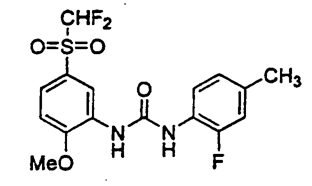

**Step 2. *N*-(2-Methoxy-5-(difluoromethanesulfonyl)phenyl)-*N*'-(2-fluoro-4-methylphenyl)urea**: 2-Fluoro-4-methylaniline (0.022 mL, 0.19 mmol) was added to a solution of 5-(difluoromethanesulfonyl)-2-methoxyphenyl isocyanate (0.046 g, 0.17 mmol) in EtOAc (1 mL). The reaction mixture was stirred at room temp. for 3 d. The resulting precipitate was washed with Et₂O to provide the title compound as a white solid (0.055 g): ¹H-NMR (CDCl₃) δ 2.24 (s, 3H), 4.01 (s, 3H), 6.93 (d, *J*=8.5 Hz, 1H), 7.01-7.36 (m, 3H), 7.56 (dd, *J*=2.4, 8.6 Hz, 1H), 7.98 (app t, *J*=8.6 Hz, 1H), 8.79 (d, *J*=2.2 Hz, 1H), 9.07 (s, 1H), 9.26 (s, 1H); FAB-MS *m*/*z* 389 ((M+1)⁺).

### 1B3b. General Method for the Reaction of an Aryl Amine with Phosgene with Isolation of the Isocyanate, Followed by Reaction with a Second Aryl Amine

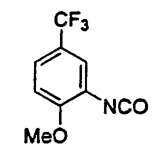

**Step 1. 2-Methoxy-5-trifluoromethylphenyl Isocyanate**: To a solution of phosgene (1.93 M in toluene; 16 mL, 31.4 mmol) in CH₂Cl₂ (120 mL) at 0 °C was added a solution of 2-methoxy-5-(trifluoromethyl)aniline (3.0 g, 15.7 mmol) and pyridine (2.3 mL, 47.1 mmol) in CH₂Cl₂ (30 mL) dropwise. The resulting mixture was stirred at 0 °C for 30 min and at room temp for 3 h, then concentrated under reduced pressure. The residue was diluted with toluene (30 mL), concentrated under reduced pressure, and treated with Et₂O. The resulting precipitate (pyridinium hydrochloride) was removed and the filtrate was concentrated under redeuced pressure to give the title compound as a yellow oil (3.0 g) which crystallized upon standing at room temp. for a few days.

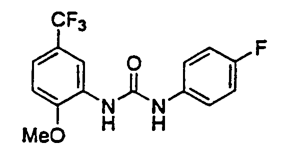

**Step 2. *N*-(2-Methoxy-5-(trifluoromethyl)phenyl)- *N'*-(4-fluorophenyl)urea**: 4-Fluoroaniline (0.24 mL, 2.53 mmol) was added to a solution of 2-methoxy-5-(trifluoromethyl)phenyl isocyanate (0.50 g, 2.30 mmol) in EtOAc (6 mL) and the reaction mixture was stirred at room temp. for 3 d. The resulting precipitate was washed with Et₂O to give the title compound as a white solid (0.60 g): NMR: 3.94 (s, 3H). 7.13-7.18 (m, 3H), 7.30 (dd, *J*=1.5, 8.4 Hz, 1H), 7.44 (m, 2H), 8.45 (s, 1H), 8.52 (d, *J*=2.2 Hz, 1H), 9.42 (s, 1H); FAB-MS *m*/*z* 329 ((M+1)⁺).

### General Method for Urea Formation via Curtius Rearrangement, Followed by Trapping with an Amine

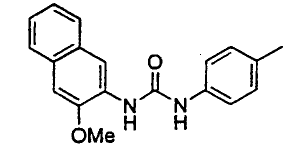

***N*-(3-Methoxy-2-naphthyl)-*N*'-(4-methylphenyl)urea:** To a solution of 3-methoxy-2-naphthoic acid (Method A6, Step 2; 0.762 g, 3.80 mmol) and Et₃N (0.588 mL, 4.2 mmol) in anh toluene (20 mL) at room temp. was added a solution of diphenylphosphoryl azide (1.16 g, 4.2 mmol) in toluene (5 mL). The resulting mixture was heated to 80 °C for 2 h, cooled to room temp., and *p*-toluidine (0.455 g, 4.1 mmol) was added. The mixture was heated at 80 °C overnight, cooled to room temp., quenched with a 10% citric acid solution, and extracted with EtOAc (2 x 25 mL). The combined organic layers were washed with a saturated NaCl solution (25 mL), dried (MgSO₄), and concentrated *in vacuo.* The residue was triturated with CH₂Cl₂ to give the desired urea as white powder (0.700 g, 61%): mp 171-172 °C; ¹H-NMR (DMSO-d₆) δ 2.22 (s, 3H), 3.99 (s, 3H), 7.07 (d, *J*=8.49 Hz, 2H), 7.27-7.36 (m, 5H), 7.67-7.72 (m, 2H), 8.43 (s, 1H), 8.57 (s, 1H), 9.33 (s, 1H); FAB-MS *m*/*z* 307 ((M+H)⁺).

### B5. General Method for the Reaction of Substituted Aniline with N,N'-Carbonyldiimidazole Followed by Reaction with a Second Amine

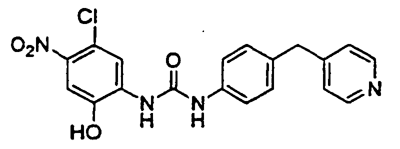

***N*-(5-Chloro-2-hydroxy-4-nitrophenyl)-*N'*-(4-(4-pyridinylmethyl)phenyl)urea:** A solution of 4-(4-pyridinylmethyl)aniline (0.300 g, 1.63 mmol) and *N,N'*-carbonyldiimidazole (0.268 g, 1.65 mmol) in CH₂Cl₂ (10 mL) was stirred at room temp. for 1 h at which time TLC analysis indicated no starting aniline. The reaction mixture was then treated with 2-amino-4-chloro-5-nitrophenol (0.318 g, 1.65 mmol) and stirred at 40-45 °C for 48 h. The resulting mixture was cooled to room temp. and diluted with EtOAc (25 mL). The resulting precipitate was separated to give the desired product (0.416 g, 64%): TLC (50% acetone/50% CH₂Cl₂) R_{*f*} 0.40; ¹H-NMR (DMSO-d₆) δ 3.90 (s, 2H), 7.18 (d, *J*=8.4 Hz, 2H), 7.21(d, *J*=6 Hz, 2H), 7.38 (d, *J*=8.4 Hz, 2H), 7.54 (s, 1H), 8.43-8.45 (m, 3H), 8.78 (s, 1H), 9.56 (s, 1H), 11.8 (br s, 1H); FAB-MS *m*/*z* (rel abundance) 399 ((M+H)⁺, 10%).

### B6. General Method for the Synthesis of Symmetrical Diphenyl Ureas as Side-Products of Urea Forming reactions

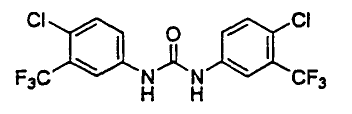

**Bis(4-chloro-3-(trifluoromethyl)phenyl)urea:** To a solution of 5-amino-3-*tert*-butylisoxazole (0.100 g) in anh toluene (5 mL) was added 4-chloro-3-(trifluoromethyl)phenyl isocyanate (0.395 g). The reaction vessel was sealed, heated at 85 °C for 24 h, and cooled to room temp. The reaction mixture was added to a slurry of Dowex® 50WX2-100 resin (0.5 g) in CH₂Cl₂ (40 mL), and the resulting mixture was stirred vigorously for 72 h. The mixture was filtered and the filtrate was concentrated under reduced pressure. The residue was purified by column chromatography (gradient form 100% CH₂Cl₂ to 5% MeOH/95% CH₂Cl₂) to give bis(4-chloro-3-(trifluoromethyl)phenyl)urea followed by *N*-(3-*tert*-butyl-5-isoxazolyl)-*N'*-(4-chloro-3-(trifluoromethyl)phenyl)urea. The residue from the symmetrical urea fractions was triturated (Et₂O/hexane) to give the urea as a white solid (0.110 g): TLC (3% MeOH/97% CH₂Cl₂) R_{*f*} 0.55; FAB-MS *m*/*z* 417 ((M+H)⁺).

### B. Combinatorial Method for the Synthesis of Diphenyl Ureas Using Triphosgene

One of the anilines to be coupled was dissolved in dichloroethane (0.10 M). This solution was added to an 8 mL vial (0.5 mL) containing dichloroethane (1 mL). To this was added a triphosgene solution (0.12 M in dichloroethane, 0.2 mL, 0.4 equiv.), followed by diisopropylethylamine (0.35 M in dichloroethane, 0.2 mL, 1.2 equiv.). The vial was capped and heated at 80°C for 5 h, then allowed to cool to room temp. for approximately 10 h. The second aniline was added (0.10 M in dichloroethane, 0.5 mL, 1.0 equiv.), followed by diisopropylethylamine (0.35 M in dichloroethane, 0.2 mL, 1.2 equiv.). The resulting mixture was heated at 80°C for 4 h, cooled to room temperature and treated with MeOH (0.5 mL). The resulting mixture was concentrated under reduced pressure and the products were purified by reverse phase HPLC.

### C. Urea Interconversions and Misc. Reactions

### C1. General Method for Alkylation of Hydroxyphenyl Ureas

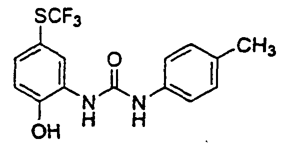

**Step 1.*N*-(2-Hydroxy-5-(trifluoromethylthio)phenyl)-*N*'-(4-methylphenyl)urea:** *p*-Tolyl isocyanate (0.066 mL, 0.52 mmol) was added to a solution of 2-hydroxy-5-(trifluoromethylthio)aniline (0.100 g, 0.48 mmol) in EtOAc (2 mL) and the reaction mixture was stirred at room temp. for 2 d. The resulting precipitate was washed with EtOAc to provide the title compound (0.13 g): ¹H-NMR (CDCl₃) δ 2.24 (s, 3H). 7.44-7.03 (m, 6H), 8.46 (s, 1H), 8.60 (d, *J*=1.8 Hz, 1H), 9.16 (s, 1H), 10.41 (s, 1H); FAB-MS *m*/*z* 343 ((M+1)⁺). This material was used in the next step without purification.

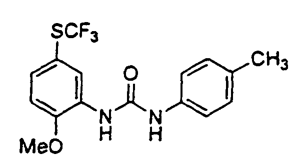

**Step 2.*N*-(2-Methoxy-5-(trifluoromethylthio)phenyl)-*N'*-(4-methylphenyl)urea:** A solution of *N*-(2-hydroxy-5-(trifluoromethylthio)phenyl)-*N'*-(4-methylphenyl)urea (0.125 g, 0.36 mmol), iodomethane (0.045 mL, 0.73 mmol), and K₂CO₃ (100 mg, 0.73 mmol) in acetone (2 mL) was heated at the reflux temp. for 6 h, then was cooled to room temp. and concentrated under reduced pressure. The residue was dissolved in a minimal amount of MeOH, absorbed onto silica gel, and then purified by flash chromatograpy (3% Et₂O/97% CH₂Cl₂) to provide the title compound as a white solid (68 mg): ¹H-NMR (CDCl₃) δ 2.22 (s, 3H), 3.92 (s, 3H), 7.05-7.32 (m, 6H), 8.37 (s, 1H), 8.52 (d, *J*=2.2 Hz, 1H), 9.27 (s, 1H); FAB-MS *m*/*z* 357 ((M+1)⁺).

### C2. General Method for the Reduction of Nitro-Containing Ureas

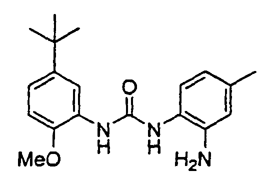

***N*-(5-*tert*-Butyl-2-metboxyphenyl)-*N'*-(2-amino-4-methylphenyl)urea**: A solution of *N*-(5-*tert*-butyl-2-methoxyphenyl)-*N'*-(2-nitro-4-methylphenyl)urea (prepared in a manner analogous to Method B1a; 4.0 g, 11.2 mmol) in EtOH (100 mL) was added to a slurry of 10% Pd/C (0.40 g) in EtOH (10 mL), and the resulting mixture was stirred under an atmosphere of H₂ (balloon) at room temp. for 18 h. The mixture was filtered through a pad of Celite® and concentrated *in vacuo* to afford the desired product (3.42 g, 94%) as a powder: mp 165-166 °C; ¹H-NMR (DMSO-d₆) δ 1.30 (s, 9H), 2.26 (s, 3H), 3.50 (br s, 2H), 3.71 (s, 3H), 6.39 (br s, 1H), 6.62 (s, 1H), 6.73 (d, *J*=8.46 Hz, 1H), 6.99 (dd, *J*=2.21, 8.46 Hz, 1H), 7.05 (d, *J*=8.46 Hz, 1H), 7.29 (s, 1H), 8.22 (d, *J*=2.57 Hz, 1H); FAB-MS *m*/*z* 328 ((M+H)⁺).

### C3. General Method of Thiourea Formation by Reaction with a Thioisocyanate

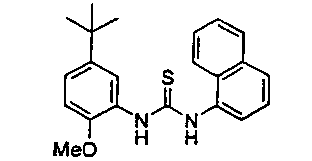

***N*-(5-*tert*-Butyl-2-methoxyphenyl)-*N*'-(1-naphthyl)thiourea:** To a solution of 5-*tert*-butyl-2-methoxyaniline (0.372 g, 2.07 mmol) in toluene (5 mL) was added 1-naphthyl thioisocyanate (0.384 g, 2.07 mmol) and the resulting mixture was allowed to stir at room temp. for 8 h to produce a precipitate. The solids were separated and sequentially washed with toluene and hexane to give the desired product as an off-white pwoder (0.364 g, 48%): mp 158-160 °C; ¹H-NMR (DMSO-d₆) δ 1.31 (s, 9H), 3.59 (s, 3H), 6.74 (d, *J*=8.46 Hz, 1H), 7.13 (dd, *J*=2.21, 8.46 Hz, 1H), 7.53-7.62 (m, 4H), 7.88-7.95 (m, 4H), 8.06-8.08 (m, 1H), 8.09 (br s, 1H); FAB-MS *m*/*z* 365 ((M+H)⁺).

### C4. General Method for Deprotection of tert-Butyl Carbonate-Containing Ureas

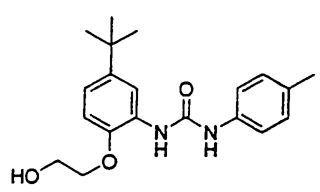

***N*-(5-*tert*-Butyl-2-(2-hydroxyethoxy)phenyl)-*N'*-(4-methylphenyl)urea**: A solution of *N*-(5-*tert*-butyl-2-(2-*tert*-butoxycarbonyloxy)ethoxy)phenyl)-*N*'-(4-methylphenyl)urea (Method B1f; 0.237 g, 0.54 mmol) and TFA (0.21 mL, 2.7 mmol) in CH₂Cl₂ (2 mL) was stirred at room temp for 18 h, then was washed with a saturated NaHCO₃ solution (2 mL). The organic layer was dried by passing through 1PS filter paper (Whatman®) and concentrated under reduced pressure. The resulting white foam was triturated (Et₂O/hexane), then recrystallized (Et₂O) to give the desired product (3.7 mg): TLC (50% EtOAc/50% hexane) R_{*f*} 0.62; ¹H-NMR (DMSO-d₆) δ 1.22 (s, 9H), 3.75-3.76 (m, 2H), 4.00-4.03 (m, 2H), 4.80 (t, *J*=5.0 Hz, 1H), 6.88-6.89 (m, 4H), 7.06 (d, *J*=8.5 Hz, 2H), 7.33 (d, *J*=8.1 Hz, 2H), 7.97 (s, 1H), 8.20 br s, 1H), 9.14 (s, 1H); FAB-MS *m*/*z* (rel abundance) 343 ((M+H)⁺, 100%).

The following compounds have been synthesized according to the General Methods listed above:

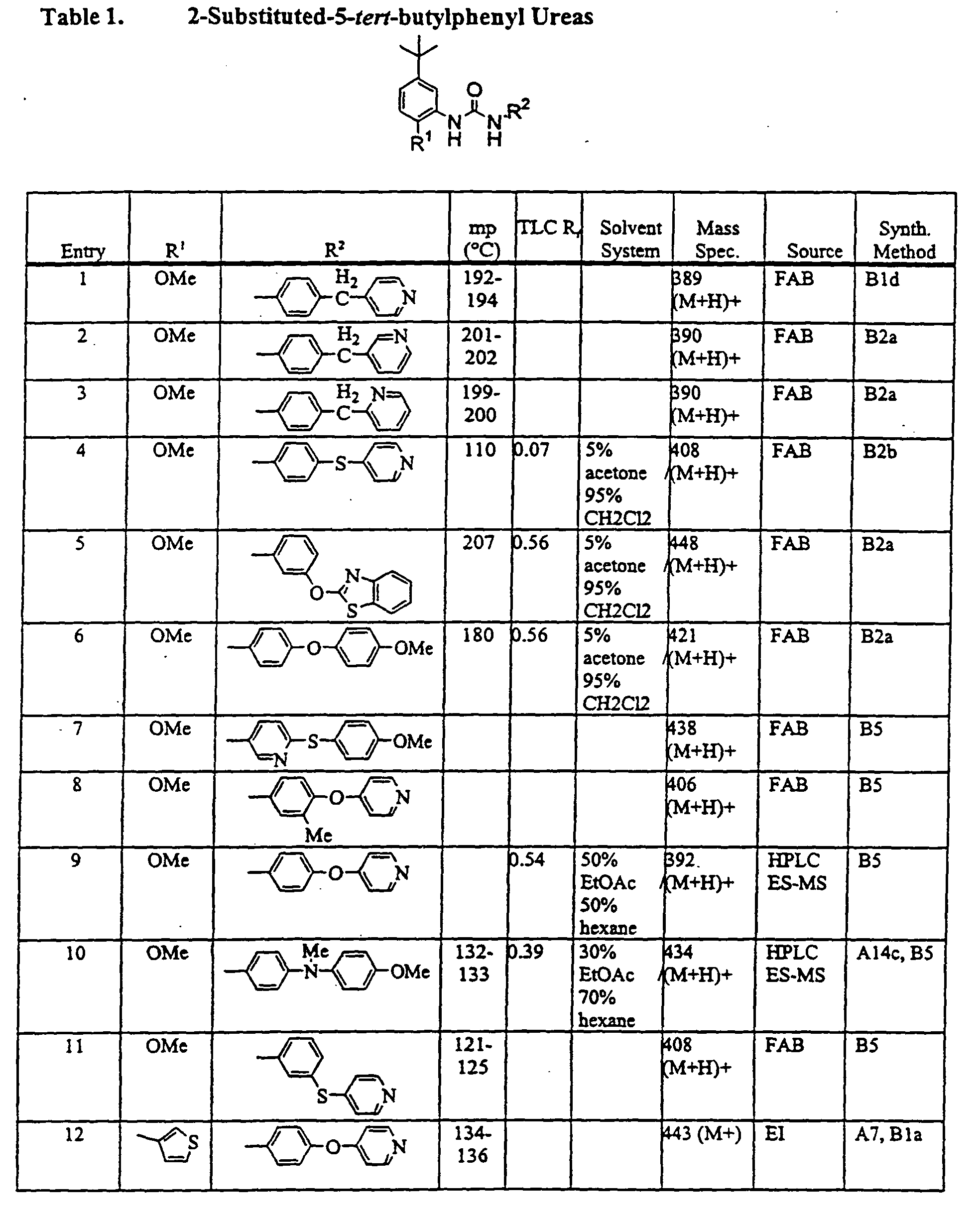

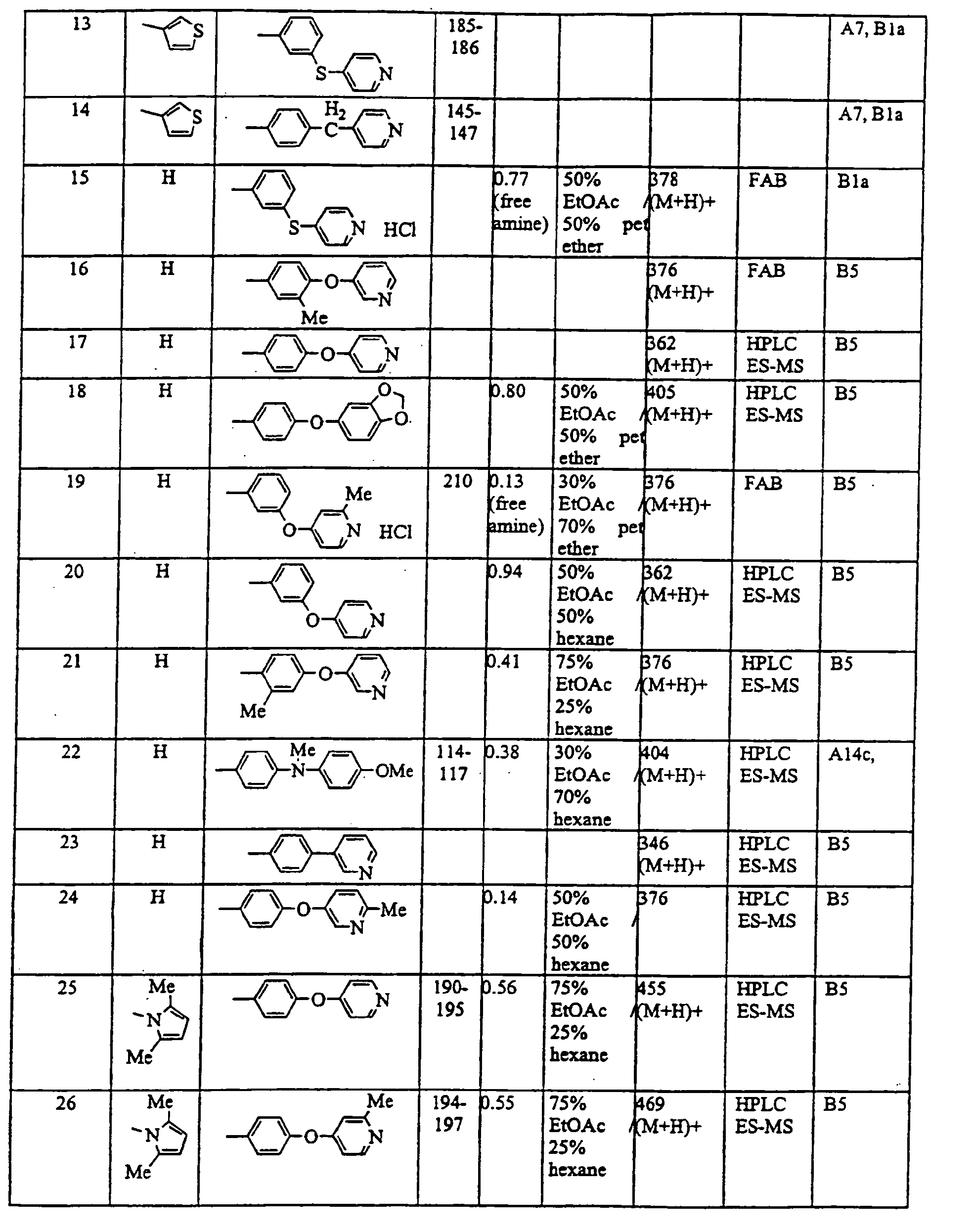

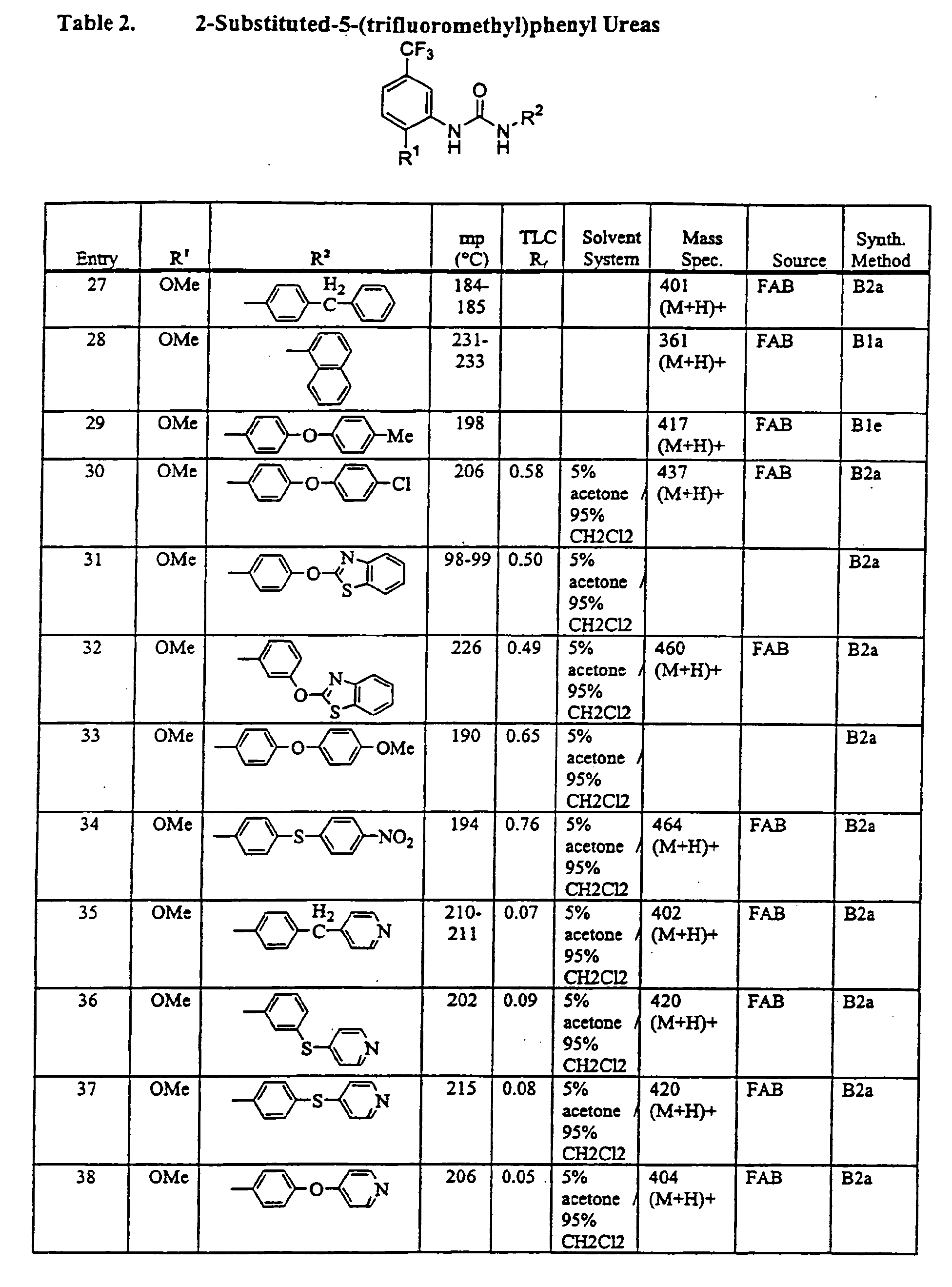

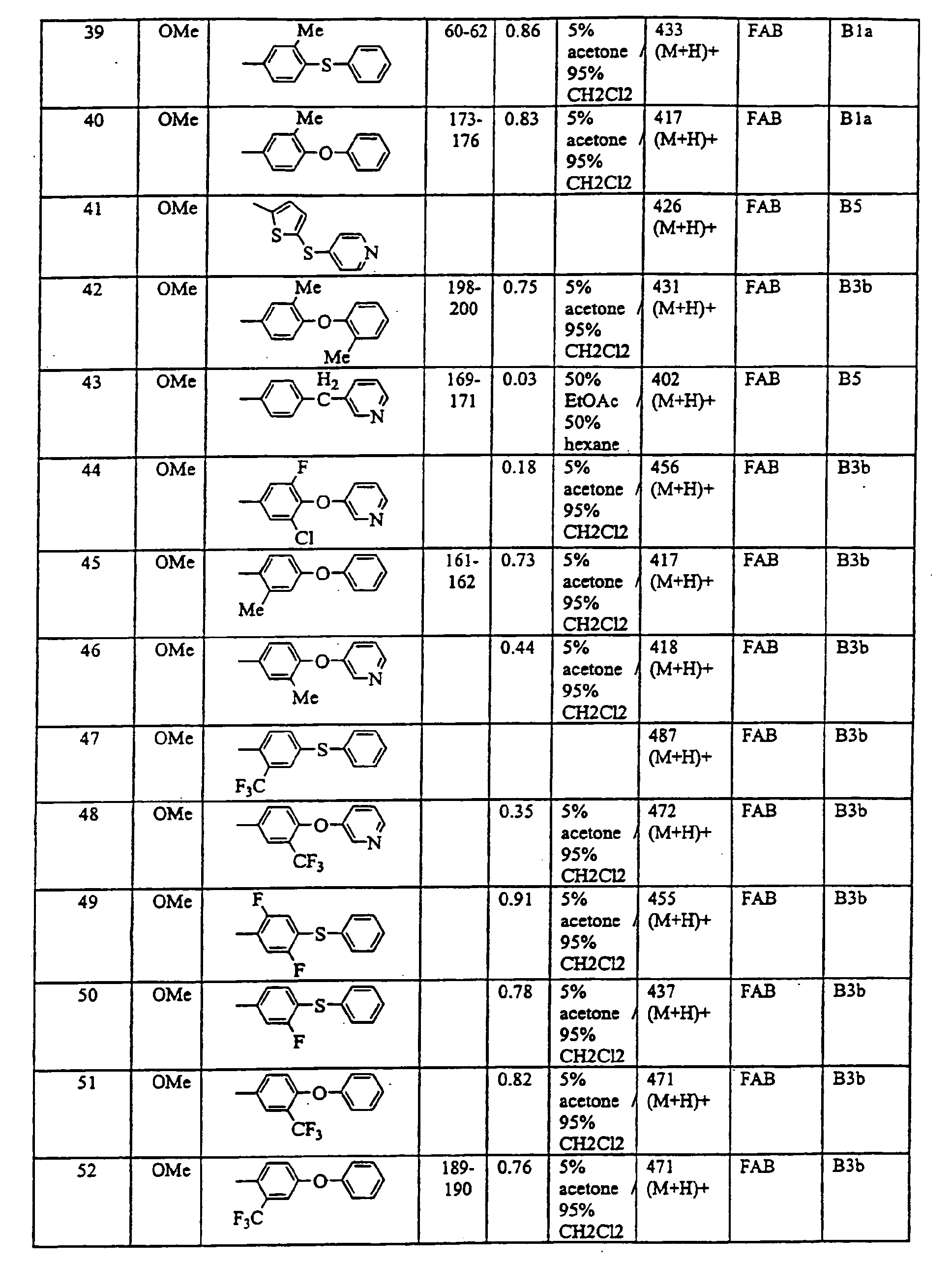

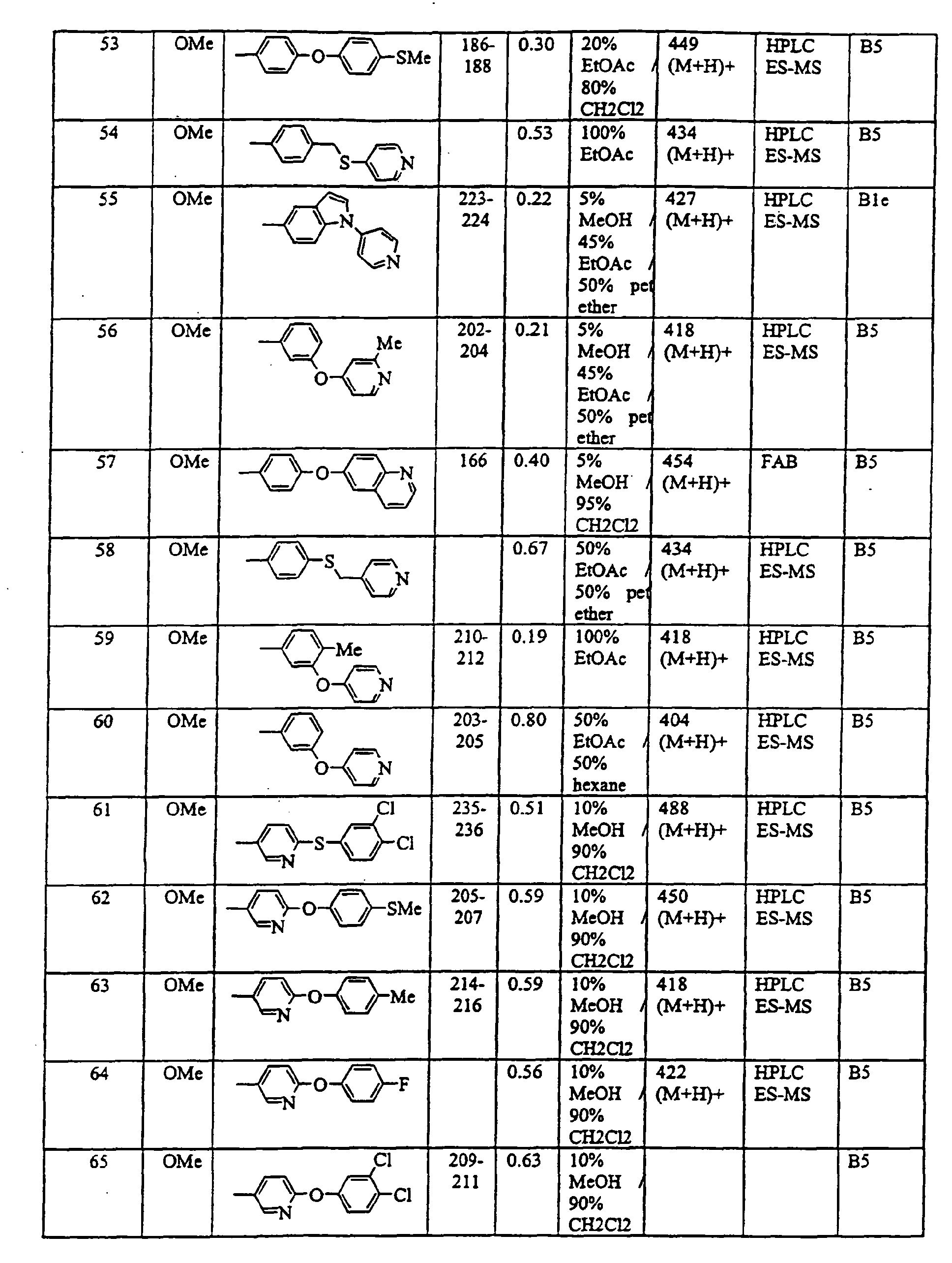

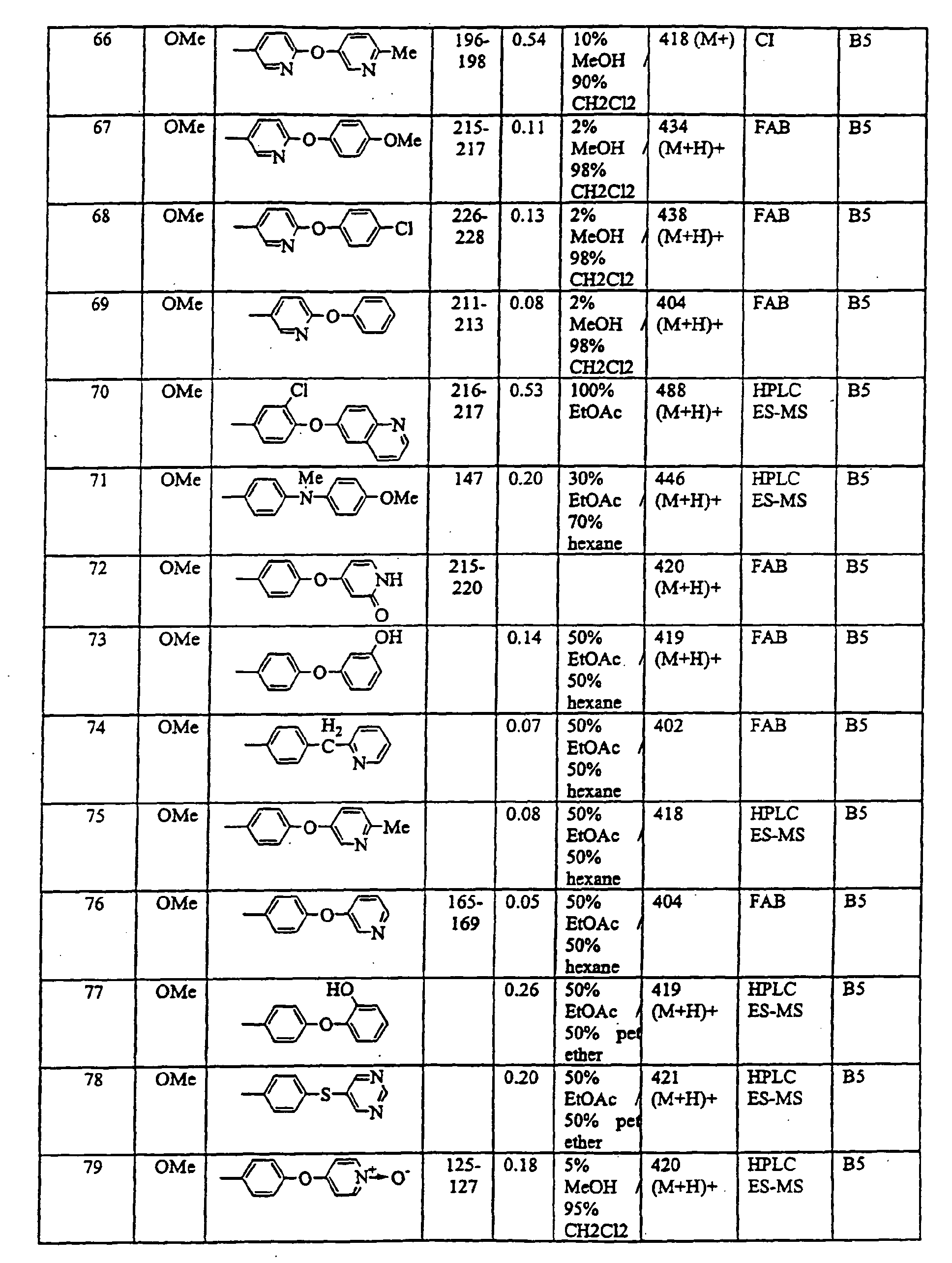

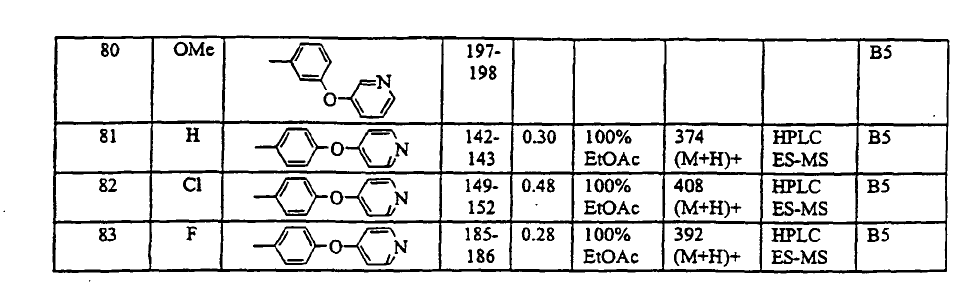

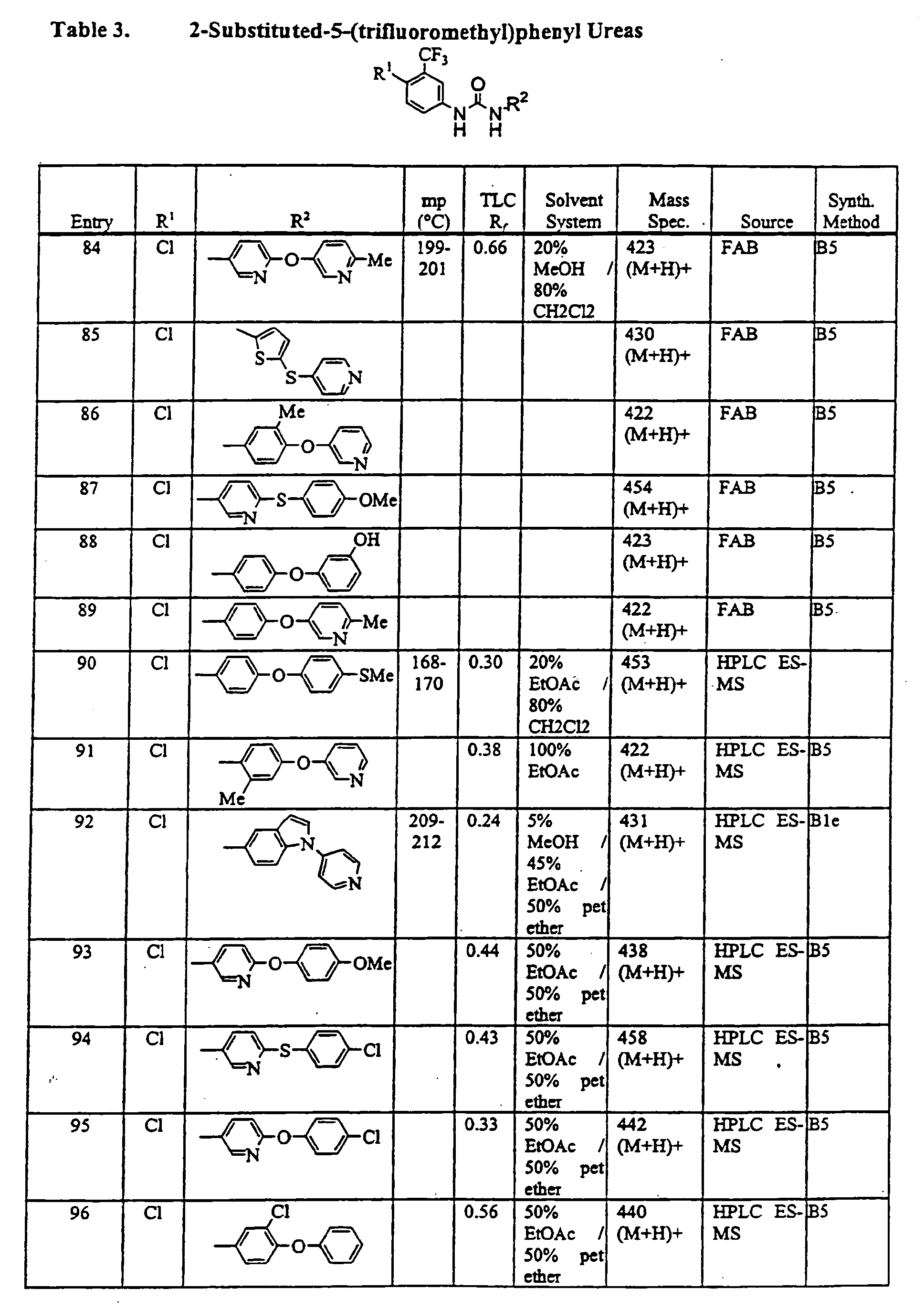

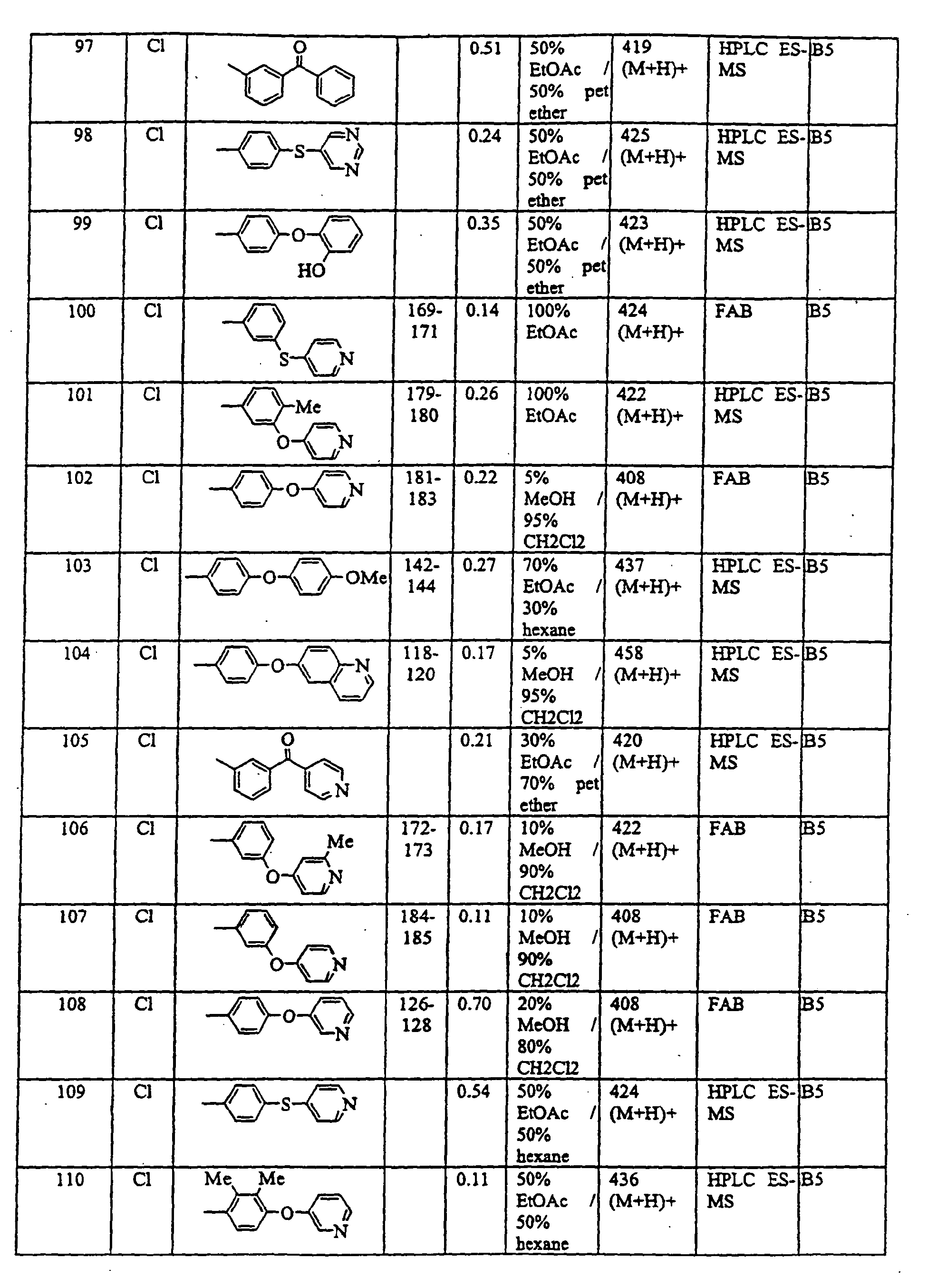

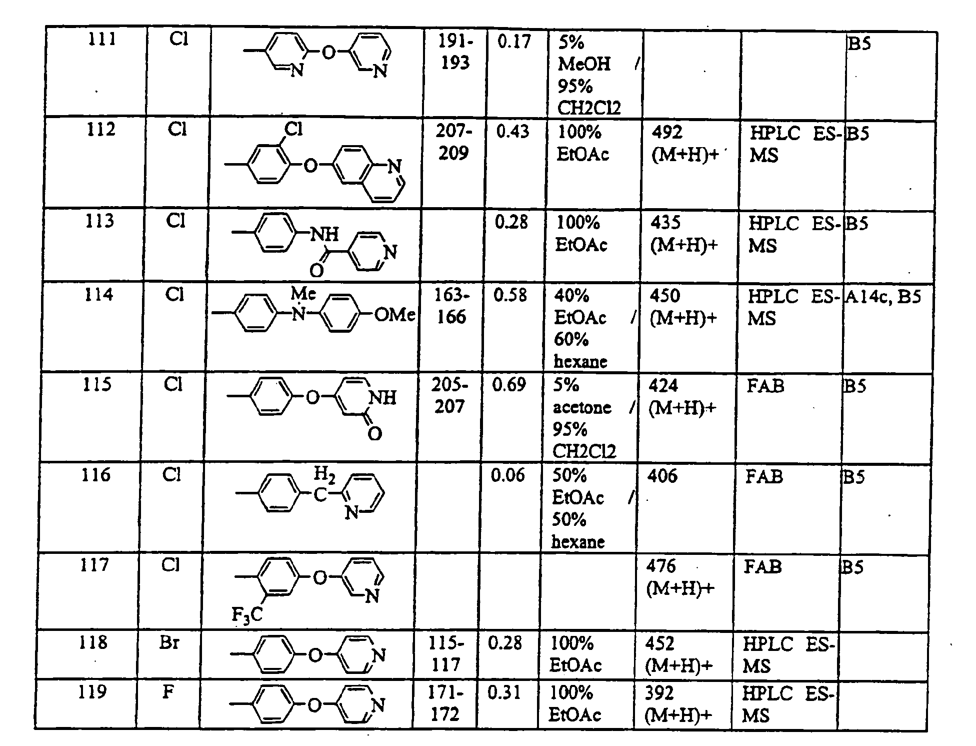

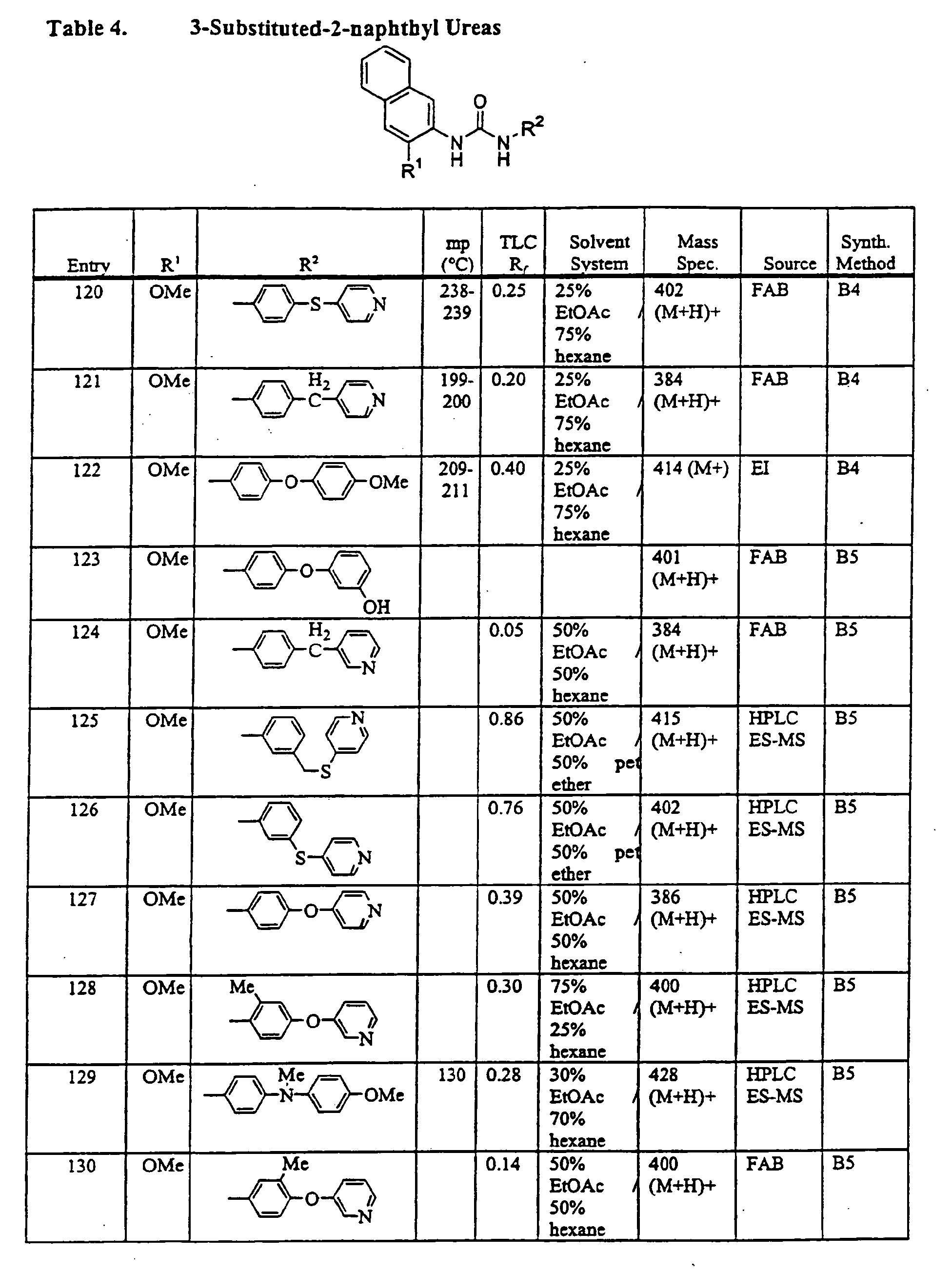

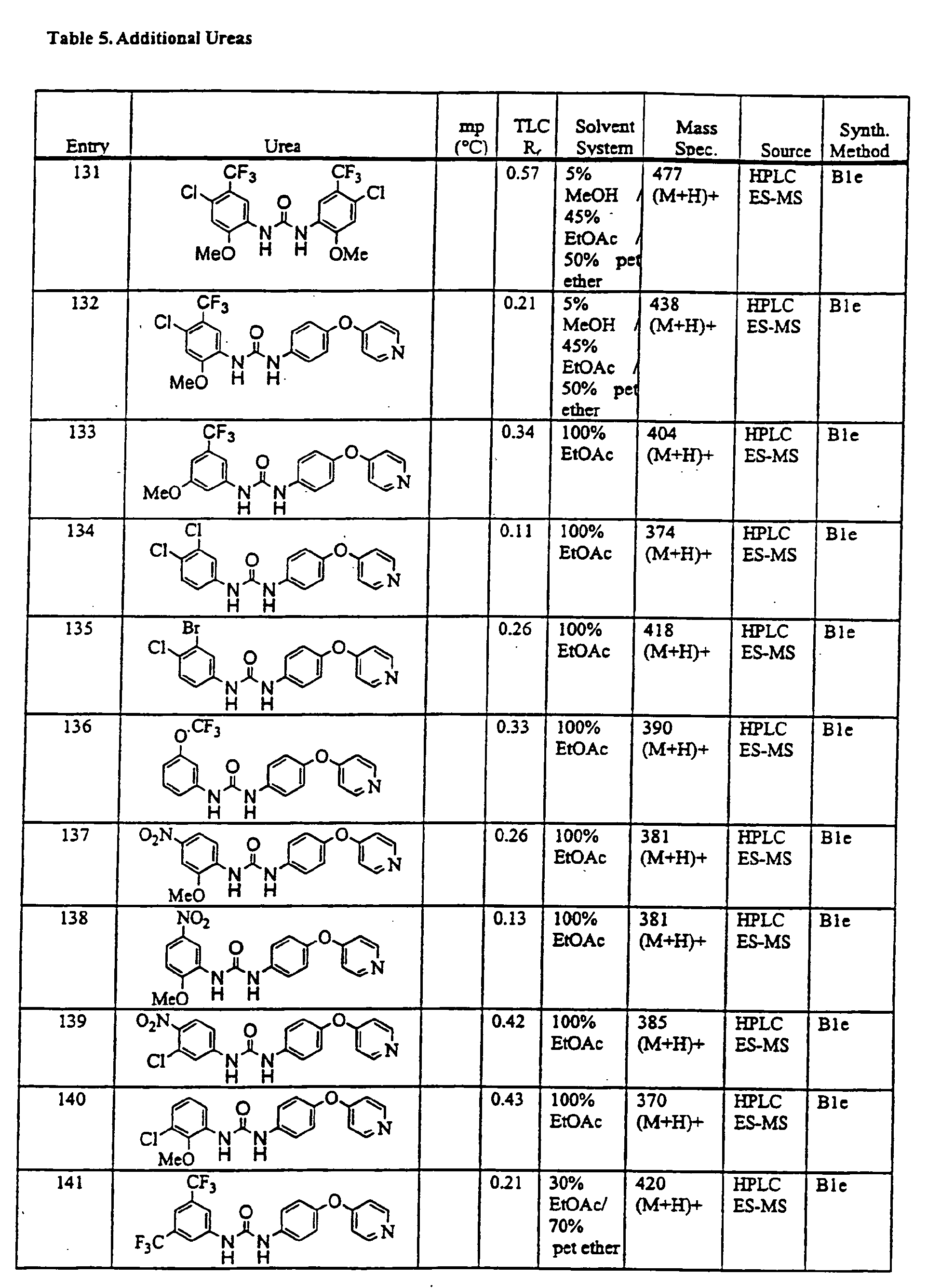

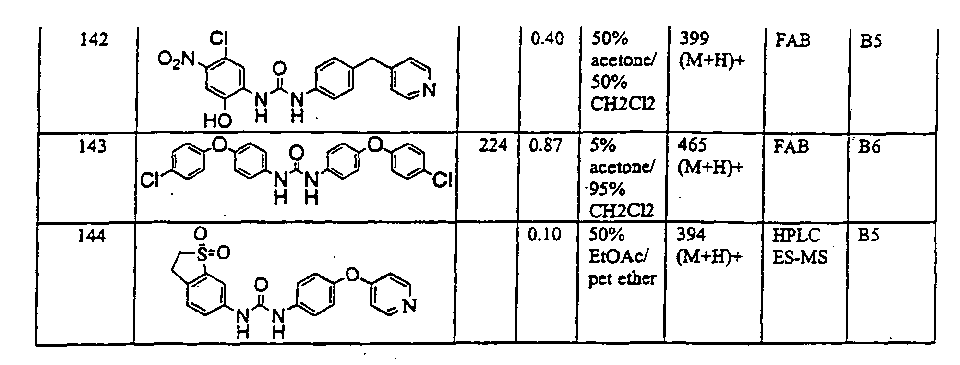

### BIOLOGICAL EXAMPLES

### In Vitro raf Kinase Assay:

In an in vitro kinase assay, raf was incubated with MEK in 20 mM Tris-HCl, pH 8.2 containing 2 mM 2-mercaptoethanol and 100 mM NaCl. This protein solution (20 µL) was mixed with water (5 µL) or with compounds diluted with distilled water from 10 mM stock solutions of compounds dissolved in DMSO. The kinase reaction was initiated by adding 25 µL [γ-³³P]ATP (1000-3000 dpm/pmol) in 80 mM Tris-HCl, pH 7.5, 120 mM NaCl, 1.6 mM DTT, 16 mM MgCl₂. The reaction mixtures were incubated at 32 °C, usually for 22 min. Incorporation of ³³P into protein was assayed by harvesting the reaction onto phosphocellulose mats, washing away free counts with a 1% phosphoric acid solution and quantitating phosphorylation by liquid scintillation counting. For high throughput screening, 10 µM ATP and 0.4 µM MEK was used. In some experiments, the kinase reaction was stopped by adding an equal amount of Laemmli sample buffer. Samples were boiled 3 min and the proteins resolved by electrophoresis on 7.5% Laemmli gels. Gels were fixed, dried and exposed to an imaging plate (Fuji). Phosphorylation was analyzed using a Fujix Bio-Imaging Analyzer System.

All compounds exemplified displayed IC₅₀s of between 1 nM and 10 µM.

### Cellular Assay:

For in vitro growth assay, human tumor cell lines, including but not limited to HCT116 and DLD-1, containing mutated K-ras genes were used in standard proliferation assays for anchorage dependent growth on plastic or anchorage independent growth in soft agar. Human tumor cell lines were obtained from ATCC (Rockville MD) and maintained in RPMI with 10% heat inactivated fetal bovine serum and 200 mM glutamine. Cell culture media and additives were obtained from Gibco/BRL (Gaithersburg, MD) except for fetal bovine serum (JRH Biosciences, Lenexa, KS). In a standard proliferation assay for anchorage dependent growth, 3 X 10³ cells were seeded into 96-well tissue culture plates and allowed to attach overnight at 37 °C in a 5% CO₂ incubator. Compounds were titrated in media in dilution series and added to 96-well cell cultures. Cells were allowed to grow 5 days typically with a feeding of fresh compound containing media on day three. Proliferation was monitored by measuring metabolic activity with standard XTT colorimetric assay (Boehringer Mannheim) measured by standard ELISA plate reader at OD 490/560, or by measuring ³H-thymidine incorporation into DNA following an 8 h culture with 1 µCu ³H-thymidine, harvesting the cells onto glass fiber mats using a cell harvester and measuring ³H-thymidine incorporation by liquid scintillant counting.

For anchorage independent cell growth, cells were plated at 1 x 10³ to 3 x 10³ in 0.4% Seaplaque agarose in RPMI complete media, overlaying a bottom layer containing only 0.64% agar in RPMI complete media in 24-well tissue culture plates. Complete media plus dilution series of compounds were added to wells and incubated at 37 °C in a 5% CO₂ incubator for 10-14 days with repeated feedings of fresh media containing compound at 3-4 day intervals. Colony formation was monitored and total cell mass, average colony size and number of colonies were quantitated using image capture technology and image analysis software (Image Pro Plus, media Cybernetics).

### In Vivo Assay:

An in vivo assay of the inhibitory effect of the compounds on tumors (e.g., solid cancers) mediated by raf kinase can be performed as follows:

CDI nu/nu mice (6-8 weeks old) are injected subcutaneously into the flank at 1 x 10⁶ cells with human colon adenocarcinoma cell line. The mice are dosed i.p., i.v. or p.o. at 10, 30, 100, or 300 mg/Kg beginning on approximately day 10, when tumor size is between 50-100 mg. Animals are dosed for 14 consecutive days once a day; tumor size was monitored with calipers twice a week.

The inhibitory effect of the compounds on raf kinase and therefore on tumors (e.g., solid cancers) mediated by raf kinase can further be demonstrated in vivo according to the technique of Monia et al. (*Nat. Med.* **1996**, *2*, 668-75).

The preceding examples can be repeated with similar success by substituting the generically or specifically described reactants and/or operating conditions of this invention for those used in the preceding examples.

From the foregoing description, one skilled in the art can easily ascertain the essential characteristics of this invention and, without departing from the spirit and scope thereof, can make various changes and modifications of the invention to adapt it to various usages and conditions.

Specifically preferred embodiments are disclosed as part of the specification under
Item 1: A compound of formula I: 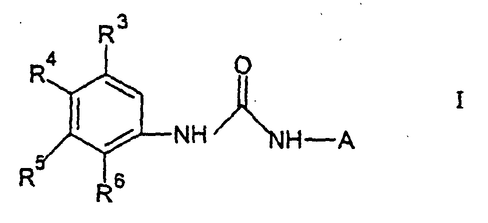 wherein
   - A: is 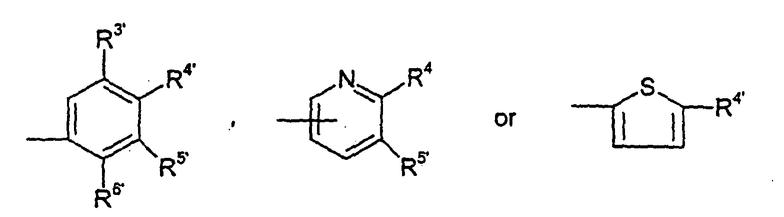
   - R³, R⁴, R⁵ and R⁶: are each, independently, H, halogen, NO₂, C₁₋₁₀- alkyl, optionally substituted by halogen up to perhaloalkyl, C₁₋₁₀-alkoxy, optionally substituted by halogen up to perhaloalkoxy, C₆₋₁₂ aryl, optionally substituted by C₁₋₁₀ alkyl or C₁₋₁₀ alkoxy, or C₅₋₁₂ hetaryl, optionally substituted by C₁₋₁₀ alkyl or C₁₋₁₀ alkoxy,
   and one of R³-R⁶ can be -X-Y;
   or two adjacent R³-R⁶ can together be an aryl or hetaryl ring with 5-12 atoms, optionally substituted by C₁₋₁₀-alkyl, C₁₋₁₀-alkoxy, C₃₋₁₀-cycloalkyl, C₂₋₁₀-alkenyl, C₁₋₁₀-alkanoyl, C₆₋₁₂-aryl, C₅₋₁₂-hetaryl; C₆₋₁₂-aralkyl, C₆₋₁₂-alkaryl, halogen; NR¹R¹; -NO₂; -CF₃; -COOR¹; -NHCOR¹; -CN; -CONR¹R¹; -SO₂R²; -SOR²; -SR²; in which R¹ is H or C₁₋₁₀-alkyl and R² is C₁₋₁₀-alkyl, optionally substituted by halogen, up to perhalo with -S(O₂)- optionally incorporated in the aryl or hetaryl ring;
   R^{4'}, R^{5'} and R^{6'} are independently H, halogen, C₁ - C₁₀ alkyl, optionally substituted by halogen up to perhaloalkyl, 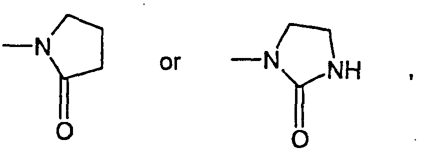 C₁ -C₁₀ alkoxy optionally substituted by halogen up to perhaloalkoxy or -X-Y, and either one of R^{4'}, R^{5'} or R^{6'} is -X-Y or two adjacent of R^{4'}, R^{5'} and R^{6'} together are a hetaryl ring with 5-12 atoms optionally substituted by C₁₋₁₀ alkyl, C₁₋₁₀ alkoxy, C₃₋₁₀ cycloalkyl, C₂₋₁₀ alkenyl, C₁₋₁₀ alkanoyl, C₆₋₁₂ aryl, C₅₋₁₂ hetaryl or C₆₋₁₂ aralkyl;
   - R^{6'}: is additionally -NHCOR¹, - NR¹COR¹ or NO₂;
   - R¹: is C₁₋₁₀ alkyl optionally substituted by halogen up to perhalo;
   - R^{3'}: is H, halogen, C₁-C₁₀ alkyl optionally substituted by halogen up to perhaloalkyl, C₁-C₁₀ alkoxy, optionally substituted by halogen up to perhaloalkoxy;
   - X: is -CH₂-, -S-, -N(CH₃)-, -NHC(O)- -CH₂-S-, -S-CH₂-, -C(O)-, or -O-; and
   - X: is additionally a single bond where Y is pyridyl; and
   - Y: is phenyl, pyridyl, naphthyl, pyridone, pyrazine, pyrimidine, benzodiaxane, benzopyridine or benzothiazole, each optionally substituted by C₁₋₁₀-alkyl, C₁₋₁₀-alkoxy, halogen, OH, -SCH₃, NO₂ or, where Y is phenyl, by 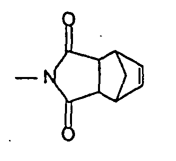 or a pharmaceutically acceptable salt thereof,
   with the proviso that if X is -O- or -S-, R^{3'} and R^{6'} are H, and Y is phenyl unsubstituted by OH, then R⁶ is alkoxy.
Item 2: A compound according to item 1, having a pKa greater than 10.
Item 3: A compound according to item 1, wherein
   R³ is halogen or C₁₋₁₀- alkyl, optionally substituted by halogen, up to perhaloalkyl;
   R⁴ is H, halogen or NO₂;
   R⁵ is H, halogen or C₁₋₁₀- alkyl; R⁶ is H, C₁₋₁₀- alkoxy, thiophene, pyrole or methyl substituted pyrole,
   R^{3'} is H, halogen, CH₃, or CF₃ and R^{6'} is H, halogen CH₃, CF₃ or -OCH_{3.}
Item 4: A compound according to item 1, wherein
   - R³: is C₄₋₁₀-alkyl, Cl, F or CF₃;
   - R⁴: is H, Cl, F or NO₂;
   - R⁵: is H, Cl, F or C₄₋₁₀-alkyl; and
   - R⁶: is H or OCH₃.
Item 5: A compound according to item 4, wherein R³ or R⁵ is t-butyl.
Item 6: A compound according to item 1, wherein X is -CH₂-, -N(CH₃)- or -NHC(O)-.
Item 7: A compound according to item 6, wherein Y is phenyl or pyridyl.
Item 8: A compound according to item 1, wherein X is -O-.
Item 9: A compound according to item 8, wherein Y is phenyl, pyridyl pyridone or benzothiazole.
Item 10: A compound according to item 1, wherein X is -S-.
Item 11: A compound according to item 10, wherein Y is phenyl or pyridyl.
Item 12: A compound of the formula 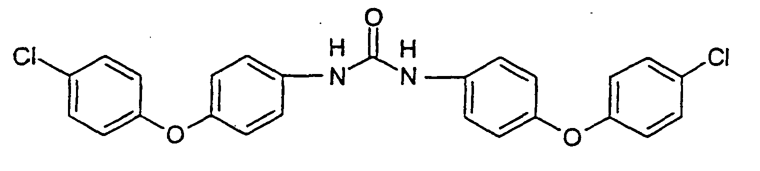
Item 13: A pharmaceutical composition comprising a compound of item 1, and a physiologically acceptable carrier.
Item 14: A pharmaceutical composition comprising a compound of item 12, and a physiologically acceptable carrier.

## Claims

1. Use of a compound of formula II 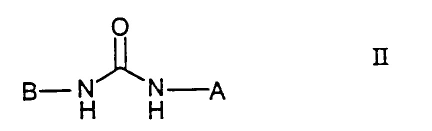 for the manufacture of a pharmaceutical composition for the treatment of a cancerous cell growth mediated by raf kinase,
wherein
A is 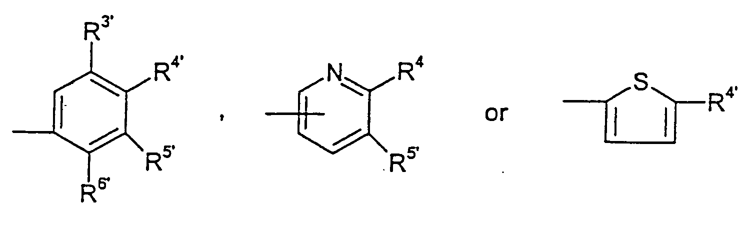
B is a substituted or unsubstituted, up to tricyclic aryl or heteroaryl moiety of up to 30 carbon atoms with at least one 6-member aromatic structure containing 0-4 members of the group consisting of nitrogen, oxygen and sulfur, wherein if B is substituted it is substituted by one or more substituents selected from the group consisting of halogen, up to per-halo, and Wₙ, wherein n is 0-3 and each W is independently selected from the group consisting of -CN, -CO₂R⁷, -C(O)NR⁷R⁷, -C(O)-R⁷, -NO₂, -OR⁷, - SR⁷, - NR⁷R⁷, -NR⁷C(O)OR⁷, -NR⁷C(O)R⁷, C₁-C₁₀ alkyl, C₂-C₁₀ alkenyl, C₁-C₁₀ alkoxy, C₃-C₁₀ cycloalkyl, C₆-C₁₄ aryl, C₇-C₂₄ alkaryl, C₃-C₁₃ heteroaryl, C₄-C₂₃ alkheteroaryl, substituted C₁-C₁₀ alkyl, substituted C₂-C₁₀ alkenyl, substituted C₁-C₁₀ alkoxy, substituted C₃-C₁₀ cycloalkyl, substituted C₄-C₂₃ alkheteroaryl and Q-Ar;
wherein if W is a substituted group, it is substituted by one or more substituents independently selected from the group consisting of -CN, -CO₂R⁷, -C(O)R⁷, -C(O)NR⁷R⁷, -OR⁷, -SR⁷, -NR⁷R⁷, NO₂, -NR⁷C(O)R⁷, -NR⁷C(O)OR⁷ and halogen up to per-halo; wherein each R¹ is independently selected from H, C₁-C₁₀ alkyl, C₂-C₁₀ alkenyl, C₃-C₁₀ cycloalkyl, C₆-C₁₄ aryl, C₃-C₁₃ hetaryl, C₇-C₂₄ alkaryl, C₄-C₂₃ alkheteroaryl, up to per-halosubstituted C₁-C₁₀ alkyl, up to per-halo substituted C₂-C₁₀ alkenyl, up to per-halosubstituted C₃-C₁₀ cycloalkyl, up to per-halosubstituted C₆-C₁₄ aryl and up to per-halosubstituted C₃-C₁₃ hetaryl,
wherein Q is - O-, -S-, -N(R⁷)-, -(CH₂)-ₘ, -C(O)-, -CH(OH)-, -(CH₂)ₘO-, -NR⁷C(O) NR⁷R⁷-, -NR⁷C(O)-, -C(O)NR⁷-, -(CH₂)ₘS-, -(CH₂)ₘN(R⁷)-, -O(CH₂)ₘ-, -CHX^{a}, -CX^{a}₂-, -S-(CH₂)ₘ- and -N(R⁷)(CH₂)ₘ-,
m = 1-3, and X^{a} is halogen; and
Ar is a 5-10 member aromatic structure containing 0-2 members of the group consisting of nitrogen, oxygen and sulfur, which is unsubstituted or substituted by halogen up to per-halo and optionally substituted by Zₙ₁, wherein ₙ₁ is 0 to 3 and each Z is independently selected from the group consisting of of -CN, -CO₂R⁷, -C(O)NR⁷R⁷, -C(O)- NR⁷, -NO₂, -OR⁷, - SR⁷, - NR⁷R⁷, -NR⁷C(O)OR⁷, -C(O)R⁷, -NR⁷C(O)R⁷, C₁-C₁₀ alkyl, C₃-C₁₀ cycloalkyl, C₆-C₁₄ aryl, C₃-C₁₃ hetaryl, C₇-C₂₄ alkaryl, C₄-C₂₃ alkheteroaryl, substituted C₁-C₁₀ alkyl, substituted C₃-C₁₀ cycloalkyl, substituted C₇-C₂₄ alkaryl and substituted C₄-C₂₃ alkheteroaryl; wherein the one or more substituents of Z is selected from the group consisting of -CN, -CO₂R⁷, -C(O)NR⁷R⁷, -OR⁷, -SR⁷, -NO₂, -NR⁷R⁷, -NR⁷C(O)R⁷ and -NR⁷C(O)OR⁷,
R^{4'}, R^{5'} and R^{6'} are each independently H, halogen, C₁₋₁₀-alkyl, optionally substituted by halogen up to perhaloalkyl, 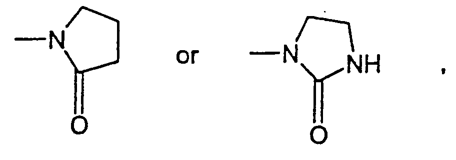 C₁ -C₁₀ alkoxy, optionally substituted by halogen up to perhaloalkoxy or -X-Y, and
either one of R^{4'}, R^{5'} or R^{6'} is -X-Y or two adjacent of R^{4'}, R^{5'} and R^{6'} together are a hetaryl ring with 5-12 atoms optionally substituted by C₁₋₁₀ alkyl, C₁₋₁₀ alkoxy, C₃₋₁₀ cycloalkyl, C₂₋₁₀ alkenyl, C₁₋₁₀ alkanoyl, C₆₋₁₂ aryl, C₅₋₁₂ hetaryl or C₆₋₁₂. aralkyl;
R^{6'} is additionally -NHCOR¹, - NR¹COR¹ or NO₂;
R¹ is C₁₋₁₀ alkyl optionally substituted by halogen up to perhalo;
R^{3'} is independently H, halogen, C₁₋₁₀ alkyl, optionally substituted by halogen up to perhaloalkyl, C₁₋₁₀ alkoxy, optionally substituted by halogen up to perhaloalkoxy;
X is -CH₂-, -S- -N(CH₃)-, -NHC(O)-, -CH₂-S-, -C(O)-, or -O-;
X is additionally a single bond where Y is pyridyl; and
Y is phenyl, pyridyl, naphthyl, pyridone, pyrazine, pyrimidine, benzodioxane, benzopyridine or benzothiazole, each optionally substituted by C₁₋₁₀-alkyl, C₁₋₁₀-alkoxy, halogen, OH, -SCH₃, or NO₂ or, where Y is phenyl, by 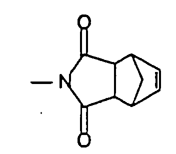 or a pharmaceutically acceptable salt thereof.

2. Use according to claim 1 , wherein the compound of formula II is the compound of formula IIa: 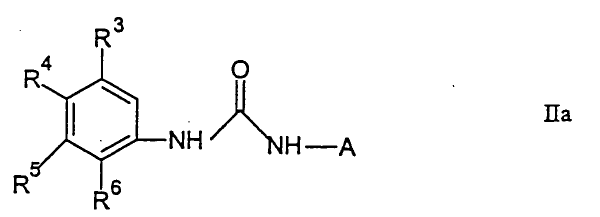 wherein
A is 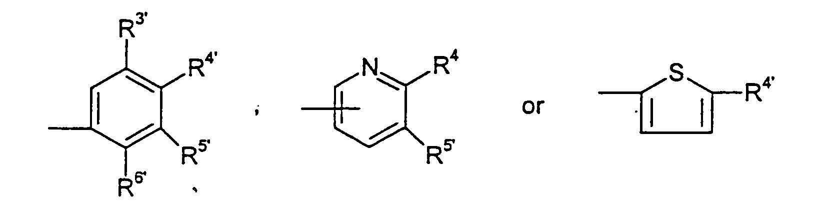
R³, R⁴ ,R⁵ and R⁶ are each independently H, halogen, NO₂, C₁₋₁₀- alkyl, optionally substituted by halogen up to perhaloalkyl, or C₁₋₁₀-alkoxy, optionally substituted by halogen up to perhaloalkoxy, C₆₋₁₂ aryl, optionally substituted by C₁₋₁₀ alkyl or C₁₋₁₀ alkoxy, or C₅₋₁₂ hetaryl, optionally substituted by C₁₋₁₀ alkyl or C₁₋₁₀ alkoxy,
and one of R³-R⁶ can be -X-Y;
or two adjacent R³-R⁶ can together be an aryl or hetaryl ring with 5-12 atoms, optionally substituted by C₁₋₁₀-alkyl, C₁₋₁₀-alkoxy, C₃₋₁₀-cycloalkyl, C₂₋₁₀-alkenyl, C₁₋₁₀-alkanoyl; C₆₋₁₂-aryl, C₅₋₁₂-hetaryl, C₆₋₁₂-alkaryl, halogen; -NR¹R¹; -NO₂; -CF₃; -COOR¹; -NHCOR¹; -CN; -CONR¹R¹; -SO₂R²; -SOR²; -SR²; in which R¹ is H or C₁₋₁₀-alkyl, optionally substituted by halogen, up to perhalo and R² is C₁₋₁₀-alkyl, optionally substituted by halogen, up to perhalo, with - SO₂- optionally incorporated in the aryl or hetaryl ring, and R^{3'}- R^{6'} are as defined in claim 2

3. Use according to claim 2, wherein
R³ is halogen or C₁₋₁₀- alkyl, optionally substituted by halogen, up to perhaloalkyl;
R⁴ is H, halogen or NO₂;
R⁵ is H, halogen or C₁₋₁₀- alkyl;
R⁶ is H [or] C₁₋₁₀- alkoxy, thiophene, pyrole or methylsubstituted pyrole
R^{3'} is H, halogen, CH₃, or CF₃ and
R^{6'} is H, halogen, CH₃, CF₃ or OCH₃.

4. Use according to claim 2 , wherein X is -CH₂-, [or] -S-, -N(CH₃)- or -NHC(O)- and Y is phenyl or pyndyl.

5. Use according to claim 2, wherein X is -O- and Y is phenyl, pyridone, pyrimidine, pyridyl or benzothiazole.
